# EUROPEAN PATENT SPECIFICATION

(11) **EP 0 853 679 B1**
(45) Date of publication and mention of the grant of the patent: **22.01.2003**
(21) Application number: 96931598.5
(22) Date of filing: 13.09.1996
(51) Int. Cl.: C12Q 1/68, C07H 21/04

(54) **EXPRESSION MONITORING BY HYBRIDIZATION TO HIGH DENSITY OLIGONUCLEOTIDE ARRAYS**
EXPRESSIONSNACHWEIS DURCH HYBRIDISIERUNG AN OLIGONUKLEOTIDARRAYS HOHER BELEGUNGSDICHTE
MESURE DE L'EXPRESSION PAR L'HYBRIDATION AVEC DES SYSTEMES TRES DENSES D'OLIGONUCLEOTIDES

(30) Priority: 15.09.1995 US 529115
(43) Date of publication of application: 22.07.1998
(73) Proprietor: Affymetrix, Inc. (a Delaware Corporation), Santa Clara, CA 95051 (US)
(72) Inventor: LOCKHART, David, J., Mountain View, CA 94041 (US); BROWN, Eugene, L., Newton Highlands, MA 02161 (US); WONG, Gordon, Brookline, MA 02146 (US); CHEE, Mark, Palo Alto, CA 94306 (US); GINGERAS, Thomas, R., Encinitas, CA 92021 (US); MITTMANN, Michael, P., Palo Alto, CA 94303 (US); LIPSHUTZ, Robert, J., Palo Alto, CA 94301 (US); FODOR, Stephen, P., A., Palo Alto, CA 94303 (US); WANG, Chunwei, Salt Lake City, UT 84102 (US)
(74) Representative: Bizley, Richard Edward
(86) International application number: PCT/US96/14839
(87) International publication number: WO 97/010365

(56) References cited:
- EP-A- 0 717 113
- EP-A- 0 721 016
- WO-A-89/10977
- WO-A-90/04652
- WO-A-90/15070
- WO-A-92/10588
- WO-A-95/00530
- WO-A-95/11995
- US-A- 5 202 231
- BIOTECHNIQUES (1995), 19(3), 442-7 CODEN: BTNQDO;ISSN: 0736-6205, 1995, XP000541924 LIPSHUTZ, R. J. ET AL: "Using oligonucleotide probe arrays to access genetic diversity"
- NATURE BIOTECHNOLOGY, vol. 14, no. 13, December 1996, NATURE PUBL. CO.,NEW YORK, US, pages 1675-1680, XP002022521 D.J. LOCKHART ET AL.: "Expression monitoring by hybridization to high-density oligonucleotide arrays"
- PROC. NATL. ACAD. SCI. U. S. A. (1996), 93(20), 10614-10619 CODEN: PNASA6;ISSN: 0027-8424, 1 October 1996, XP002022507 SCHENA, MARK ET AL: "Parallel human genome analysis: microarray-based expression monitoring of 1000 genes"
- SCIENCE, vol. 274, 25 October 1996, AAAS,WASHINGTON,DC,US, pages 610-614, XP002022508 M. CHEE ET AL.: "Accessing genetic information with high-density DNA arrays"

## Description

### BACKGROUND OF THE INVENTION

A portion of the disclosure of this patent document contains material which subject to copyright protection. The copyright owner has no objection to the xerographic reproduction by anyone of the patent document or the patent disclosure in exactly the form it appears in the Patent and Trademark Office patent file or records, but otherwise reserves all copyright rights whatsoever.

Many disease states are characterized by differences in the expression levels of various genes either through changes in the copy number of the genetic DNA or through changes in levels of transcription (*e*.*g*. through control of initiation, provision of RNA precursors, RNA processing, *etc*.) of particular genes. For example, losses and gains of genetic material play an important role in malignant transformation and progression. These gains and losses are thought to be "driven" by at least two kinds of genes. Oncogenes are positive regulators of tumorgenesis, while tumor suppressor genes are negative regulators of tumorgenesis (Marshall, *Cell,* 64: 313-326 (1991); Weinberg, Science, 254: 1138-1146 (1991)). Therefore, one mechanism of activating unregulated growth is to increase the number of genes coding for oncogene proteins or to increase the level of expression of these oncogenes (*e.g*. in response to cellular or environmental changes), and another is to lose genetic material or to decrease the level of expression of genes that code for tumor suppressors. This model is supported by the losses and gains of genetic material associated with glioma progression (Mikkelson *et al.* J. Cellular Biochm. 46: 3-8 (1991)). Thus, changes in the expression (transcription) levels of particular genes (*e*.*g*. oncogenes or tumor suppressors). serve as signposts for the presence and progression of various cancers.

Similarly, control of the cell cycle and cell development, as well as diseases, are characterized by the variations in the transcription levels of particular genes. Thus, for example, a viral infection is often characterized by the elevated expression of genes of the particular virus. For example, outbreaks of *Herpes simplex,* Epstein-Barr virus infections (*e*.*g*. infectious mononucleosis), cytomegalovirus, Varicella-zoster virus infections, parvovirus infections, human papillomavirus infections. *etc.* are all characterized by elevated expression of various genes present in the respective virus. Detection of elevated expression levels of characteristic viral genes provides an effective diagnostic of the disease state. In particular, viruses such as herpes simplex, enter quiescent states for periods of time only to erupt in brief periods of rapid replication. Detection of expression levels of characteristic viral genes allows detection of such active proliferative (and presumably infective) states.

Oligonucleotide probes have long been used to detect complementary nucleic acid sequences in a nucleic acid of interest (the "target" nucleic acid) and have been used to detect expression of particular genes (*e.g*., a Northern Blot). In some assay formats, the oligonucleotide probe is tethered, *i.e*., by covalent attachment, to a solid support, and arrays of oligonucleotide probes immobilized on solid supports have been used to detect specific nucleic acid sequences in a target nucleic acid. *See, e.g*., PCT patent publication Nos. WO 89/10977 and 89/11548. Others have proposed the use of large numbers of oligonucleotide probes to provide the complete nucleic acid sequence of a target nucleic acid but failed to provide an enabling method for using arrays of immobilized probes for this purpose. *See* U.S. Patent Nos. 5,202,231 and 5,002,867 and PCT patent publication No. WO 93/17126.

The use of "traditional" hybridization protocols for monitoring or quantifying gene expression is problematic. For example two or more gene products of approximately the same molecular weight will prove difficult or impossible to distinguish in a Northern blot because they are not readily separated by electrophoretic methods.
Similarly, as hybridization efficiency and cross-reactivity varies with the particular subsequence (region) of a gene being probed it is difficult to obtain an accurate and reliable measure of gene expression with one, or even a few, probes to the target gene.

The development of VLSIPS™ technology provided methods for synthesizing arrays of many different oligonucleotide probes that occupy a very small surface area. *See* U.S. Patent No. 5,143,854 and PCT patent publication No. WO 90/15070. U.S. Patent application Serial No. 082,937, filed June 25, 1993, describes methods for making arrays of oligonucleotide probes that can be used to provide the complete sequence of a target nucleic acid and to detect the presence of a nucleic acid containing a specific nucleotide sequence.

Prior to the present invention, however, it was unknown that high density oligonucleotide arrays could be used to reliably monitor message levels of a multiplicity of preselected genes in the presence of a large abundance of other (non-target) nucleic acids (*e.g*., in a cDNA library, DNA reverse transcribed from an mRNA, mRNA used directly or amplified, or polymerized from a DNA template). In addition, the prior art provided no rapid and effective method for identifying a set of oligonucleotide probes that maximize specific hybridization efficacy while minimizing cross-reactivity nor of using hybridization patterns (in particular hybridization patterns of a multiplicity of oligonucleotide probes in which multiple oligonucleotide probes are directed to each target nucleic acid) for quantification of target nucleic acid concentrations.

### Summary of the Invention

The present invention is premised, in part, on the discovery that microfabricated arrays of large numbers of different oligonucleotide probes (DNA chips) may effectively be used to not only detect the presence or absence of target nucleic acid sequences, but to quantify the relative abundance of the target sequences in a complex nucleic acid pool. In addition, it was also a surprising discovery that relatively short oligonucleotide probes (*e.g*., 20 mer) are sufficiently specific to allow quantitation of gene expression in complex mixtures of nucleic acids particularly when provided as in high density oligonucleotide probe arrays.

Prior to this invention it was unknown that hybridization to high density probe arrays would permit small variations in expression levels of a particular gene to be identified and quantified in a complex population of nucleic acids that out number the target nucleic acids by 1,000 fold to 1,000,000 fold or more. It was also unknown that the transcription levels of specific genes can be quantitated in a complex nucleic acid mixture with only a few (*e*.*g*., less than 20 or even less than 10) relatively short oligonucleotide probes.

Thus, this invention provides for a method of simultaneously monitoring the expression (*e*.*g*. detecting and or quantifying the expression) of a multiplicity of genes. The levels of transcription for virtually any number of genes may be determined simultaneously. Typically, at least about 10 genes, preferably at least about 100, more preferably at least about 1000 and most preferably at least about 10,000 different genes are assayed at one time.

The method of the invention involves simultaneously monitoring the expression of a multiplicity of genes, and comprises (a) providing a pool of target nucleic acids comprising RNA transcripts of some of said genes, or nucleic acids derived from said RNA transcripts; (b) providing a plurality of different probes for analysis of each of the RNA transcripts that are to be monitored; said probes being immobilized as an array on a surface of a substrate in known locations at a density greater than 60 different probes per cm²; said array probes including match and control probes; the array comprising more than 100 different probes; (c) hybridizing said pool of nucleic acids to the array of nucleic acid probes; and (d) quantifying hybridization of said target nucleic acids to said array by comparing hybridisation of match and control probes wherein said quantifying provides a measure of the levels of transcription of said genes.

The quantification preferably provides a measure of the levels of transcription of the genes. In a preferred embodiment, the pool of target nucleic acids is one in which the concentration of the target nucleic acids (mRNA transcripts or nucleic acids derived from the mRNA transcripts) is proportional to the expression levels of genes encoding those target nucleic acids.

In a preferred embodiment, the array of oligonucleotide probes is a high density array comprising greater than 100, preferably greater than about 1,000 more preferably greater than about 16,000 and most preferably greater than about 65,000 or 250,000 or even 1,000,000 different oligonucleotide probes. Such high density arrays comprise a probe density of generally greater than about 60, more generally greater than about 100, most generally greater than about 600, often greater than about 1000, more often greater than about 5,000, most often greater than about 10,000, preferably greater than about 40,000 more preferably greater than about 100,000, and most preferably greater than about 400,000 different oligonucleotide probes per cm² (where different oligonucleotides refers to oligonucleotides having different sequences). The oligonucleotide probes range from about 5 to about 50 nucleotides, preferably from about 5 to about 45 nucleotides, still more preferably from about 10 to about 40 nucleotides and most preferably from about 15 to about 40 nucleotides in length. Particularly preferred arrays contain probes ranging from about 20 to about 25 oligonucleotides in length. The array may comprise more than 10, preferably more than 50, more preferably more than 100, and most preferably more than 1000 oligonucleotide probes specific for each target gene. In a preferred embodiment, the array comprises at least 10 different oligonucleotide probes for each gene. In another preferred embodiment, the array 20 or fewer oligonucleotides complementary each gene. Although a planar array surface is preferred, the array may be fabricated on a surface of virtually any shape or even a multiplicity of surfaces.

The array may further comprise mismatch control probes. Where such mismatch controls are present, the quantifying step may comprise calculating the difference in hybridization signal intensity between each of the oligonucleotide probes and its corresponding mismatch control probe. The quantifying may further comprise calculating the average difference in hybridization signal intensity between each of the oligonucleotide probes and its corresponding mismatch control probe for each gene.

The probes present in the high density array can be oligonucleotide probes selected according to selection and optimization methods described below. Alternatively, non-optimal probes may be included in the array, but the probes used for quantification (analysis) can be selected according to the optimization methods described below.

Oligonucleotide arrays for the practice of this invention are preferably chemically synthesized by parallel immobilized polymer synthesis methods, more preferably by light directed polymer synthesis methods. Chemically synthesized arrays are advantageous in that probe preparation does not require cloning, a nucleic acid amplification step, or enzymatic synthesis. Indeed, the preparation of the probes does not require handling of any biological materials.

The array includes test probes which are oligonucleotide probes each of which has a sequence that is complementary to a subsequence of one of the genes (or the mRNA or the corresponding antisense cRNA) whose expression is to be detected. In addition, the array can contain normalization controls, mismatch controls and expression level controls as described herein.

In a particularly preferred embodiment, the variation between different copies (within and/or between batches) of each array is less than 20%, more preferably less than about 10%, and most preferably less than about 5% where the variation is measured as the coefficient of variation in hybridization intensity averaged over at least 5 oligonucleotide probes for each gene whose expression the array is to detect.

The pool of nucleic acids may be labeled before, during, or after hybridization, although in a preferred embodiment, the nucleic acids are labeled before hybridization. Fluorescence labels are particularly preferred, more preferably labeling with a single fluorophore, and, where fluorescence labeling is used, quantification of the hybridized nucleic acids is by quantification of fluorescence from the hybridized fluorescently labeled nucleic acid. Such quantification is facilitated by the use of a fluorescence microscope which can be equipped with an automated stage to permit automatic scanning of the array, and which can be equipped with a data acquisition system for the automated measurement recording and subsequent processing of the fluorescence intensity information.

In a preferred embodiment, hybridization is at low stringency (*e.g.* about 20°C to about 50°C, more preferably about 30°C to about 40°C, and most preferably about 37°C and 6X SSPE-T or lower) with at least one wash at higher stringency. Hybridization may include subsequent washes at progressively increasing stringency until a desired level of hybridization specificity is reached.

Quantification of the hybridization signal can be by any means known to one of skill in the art. However, in a particularly preferred embodiment, quantification is achieved by use of a confocal fluorescence microscope. Data is preferably evaluated by calculating the difference in hybridization signal intensity between each oligonucleotide probe and its corresponding mismatch control probe. It is particularly preferred that this difference be calculated and evaluated for each gene. Particularly preferred analytical methods are provided herein.

The pool of target nucleic acids can be the total polyA⁺ mRNA isolated from a biological sample, or cDNA made by reverse transcription of the RNA or second strand cDNA or RNA transcribed from the double stranded cDNA intermediate. Alternatively, the pool of target nucleic acids can be treated to reduce the complexity of the sample and thereby reduce the background signal obtained in hybridization. In one approach, a pool of mRNAs, derived from a biological sample, is hybridized with a pool of oligonucleotides comprising the oligonucleotide probes present in the high density array. The pool of hybridized nucleic acids is then treated with RNase A which digests the single stranded regions. The remaining double stranded hybridization complexes are then denatured and the oligonucleotide probes are removed, leaving a pool of mRNAs enhanced for those mRNAs complementary to the oligonucleotide probes in the high density array.

In another approach to background reduction, a pool of mRNAs derived from a biological sample is hybridized with paired target specific oligonucleotides where the paired target specific oligonucleotides are complementary to regions flanking subsequences of the mRNAs complementary to the oligonucleotide probes in the high density array. The pool of hybridized nucleic acids is treated with RNase H which digests the hybridized (double stranded) nucleic acid sequences. The remaining single stranded nucleic acid sequences which have a length about equivalent to the region flanked by the paired target specific oligonucleotides are then isolated (*e*.*g*. by electrophoresis) and used as the pool of nucleic acids for monitoring gene expression.

Finally, a third approach to background reduction involves eliminating or reducing the representation in the pool of particular preselected target mRNA messages (*e*.*g*., messages that are characteristically overexpressed in the sample). This method involves hybridizing an oligonucleotide probe that is complementary to the preselected target mRNA message to the pool of polyA⁺ mRNAs derived from a biological sample. The oligonucleotide probe hybridizes with the particular preselected polyA⁺ mRNA (message) to which it is complementary. The pool of hybridized nucleic acids is treated with RNase H which digests the double stranded (hybridized) region thereby separating the message from its polyA⁺ tail. Isolating or amplifying (*e*.*g*., using an oligo dT column) the polyA⁺ mRNA in the pool then provides a pool having a reduced or no representation of the preselected target mRNA message.

It will be appreciated that the methods of this invention can be used to monitor (detect and/or quantify) the expression of any desired gene of known sequence or subsequence. Moreover, these methods permit monitoring expression of a large number of genes simultaneously and effect significant advantages in reduced labor, cost and time. The simultaneous monitoring of the expression levels of a multiplicity of genes permits effective comparison of relative expression levels and identification of biological conditions characterized by alterations of relative expression levels of various genes. Genes of particular interest for expression monitoring include genes involved in the pathways associated with various pathological conditions (*e*.*g*., cancer) and whose expression is thus indicative of the pathological condition. Such genes include, but are not limited to the HER2 (c-erbB-2/neu) proto-oncogene in the case of breast cancer, receptor tyrosine kinases (RTKs) associated with the etiology of a number of tumors including carcinomas of the breast, liver. bladder, pancreas, as well as glioblastomas, sarcomas and squamous carcinomas, and tumor suppressor genes such as the P53 gene and other "marker" genes such as RAS, MSH2, MLH1 and BRCA1. Other genes of particular interest for expression monitoring are genes involved in the immune response (*e.g*., interleukin genes), as well as genes involved in cell adhesion (*e.g*., the integrins or selectins) and signal transduction (*e.g*., tyrosine kinases), *etc.*

In another embodiment, this invention provides a method of identifying genes that are effected by one or more drugs, or conversely, screening a number of drugs to identify those that have an effect on particular gene(s). This involves providing a pool of target nucleic acids from one or more cells contacted with the drug or drugs and hybridizing that pool to any of the high density oligonucleotide arrays described herein. The expression levels of the genes targeted by the probes in the array are determined and compared to expression levels of genes from "control" cells not exposed to the drug or drugs. The genes that are overexpressed or underexpressed in response to the drug or drugs are identified or conversely the drug or drugs that alter expression of one or more genes are identified.

In still yet another embodiment, this invention provide for a composition comprising any of the high density oligonucleotide arrays disclosed herein where the oligonucleotide probes are specifically hybridized to one or more fluorescently labeled nucleic acids (which are the transcription products of genes or derived from those transcription products) thereby forming a fluorescent array in which the fluorescence of the array is indicative of the transcription levels of the multiplicity of genes. One of skill will appreciate that such a hybridized array may be used as a reference, control, or standard (*e.g.*, provided in a kit) or may itself be a diagnostic array indicating the expression levels of a multiplicity of genes in a sample.

This invention also provides kits for simultaneously monitoring expression levels of a multiplicity of genes, comprising a selected plurality of different match and control probes for each RNA transcript that is to be monitored. The selected match and control probes are immobilized as an array on a surface of a substrate in known locations and the array comprises at least 100 different probes at a density greater than 60 different probes per cm². Optionally, instructions describing the use of said array for the quantification of expression levels of said multiplicity of genes are included. Optionally, the control probes are mismatch probes, there being a corresponding mismatch probe for each match probe. The kit may additionally include one or more of the following: buffers, hybridisation mix, wash and read solutions, labels, labelling reagents (enzymes *etc.*), "control" nucleic acids, software for probe selection, array reading or data analysis and any of the other materials or reagents described herein for the practice of the claimed methods.

In another embodiment, this invention provides for a method of selecting a set of oligonucleotide probes and immobilizing the probes to a surface of a substrate as an array for monitoring the expression of RNA transcripts or nucleic acids derived therefrom from a plurality of target genes. The method comprises: (a) providing an array of nucleic acid probes said array comprising a multiplicity of nucleic acid probes, wherein each probe is complementary to a subsequence of said target nucleic acids and for each probe there is a corresponding mismatch control probe, e.g. wherein said mismatch control probes have a 1 base mismatch; (b) hybridizing said target nucleic acids to said array of nucleic acid probes; (c) selecting those probes where the difference in hybridization signal intensity between each probe and its mismatch control is detectable, preferably, wherein said difference in hybridization intensity is at least 10% of the background signal; and (d) immobilizing a plurality of the selected probes for each of the target nucleic acids to be analysed together with control probes to the surface of a substrate to allow quantification of the target nucleic acids.

Preferably the difference in hybridisation signal intensity between each probe and its mismatch control is greater than about 10% of the background signal intensity, more preferably greater than about 20% of the background signal intensity and most preferably greater than about 50% of the background signal intensity). The method can further comprise hybridizing the array to a second pool of nucleic acids comprising nucleic acids other than the target nucleic acids; and identifying and selecting probes having the lowest hybridization signal and where both the probe and its mismatch control have a hybridization intensity equal to or less than about 5 times the background signal intensity, preferably equal to or less than about 2 times the background signal intensity, more preferably equal to or less than about 1 times the background signal intensity, and most preferably equal or less than about half the background signal intensity.

In a preferred embodiment, the multiplicity of probes can include every different probe of length n that is complementary to a subsequence of the target nucleic acid. The probes can range from about 10 to about 50 nucleotides in length. The array is preferably a high density array as described above. Similarly, the hybridization methods, conditions, times, fluid volumes, detection methods are as herein .

In another embodiment, the invention provides a computer-implemented method of monitoring expression of genes comprising the steps of: receiving input of hybridization intensities for a plurality of nucleic acid probes including pairs of perfect match probes and mismatch probes, the hybridization intensities indicating hybridization affinity between the plurality of nucleic acid probes and nucleic acids corresponding to a gene, and each pair including a perfect match probe that is perfectly complementary to a portion of the nucleic acids and a mismatch probe that differs from the perfect match probe by at least one nucleotide; comparing the hybridization intensities of the perfect match and mismatch probes of each pair; and indicating expression of the gene according to results of the comparing step. Preferably, the differences between the hybridization intensities of the perfect match and mismatch probes of each pair are calculated.

Additionally, the invention provides a computer-implemented method for monitoring expression of genes comprising the steps of: receiving input of a nucleic acid sequence constituting a gene; generating a set of probes that are perfectly complementary to the gene; and identifying a subset of probes, including less than all of the probes in the set, for monitoring the expression of the gene, Each probe of the set may be analyzed by criteria that specify characteristics indicative of low hybridization or high cross hybridization. The criteria may include if occurrences of a specific nucleotide in a probe crosses a threshold value, if the number of a specific nucleotide that repeats sequentially in a probe crosses a threshold value, if the length of a palindrome in a probe crosses a threshold value, and the like.

### Definitions.

The phrase "massively parallel screening" refers to the simultaneous screening of at least about 100, preferably about 1000, more preferably about 10,000 and most preferably about 1,000,000 different nucleic acid hybridizations.

The terms "nucleic acid" or "nucleic acid molecule" refer to a deoxyribonucleotide or ribonucleotide polymer in either single-or double-stranded form. and unless otherwise limited, would encompass known analogs of natural nucleotides that can function in a similar manner as naturally occumng nucleotides.

An oligonucleotide is a single-stranded nucleic acid ranging in length from 2 to about 500 bases.

As used herein a "probe" is defined as an oligonucleotide capable of binding to a target nucleic acid of complementary sequence through one or more types of chemical bonds, usually through complementary base pairing, usually through hydrogen bond formation. As used herein, an oligonucleotide probe may include natural (*i.e*. A, G, C, or T) or modified bases (7-deazaguanosine, inosine, *etc*.). In addition, the bases in oligonucleotide probe may be joined by a linkage other than a phosphodiester bond, so long as it does not interfere with hybridization. Thus, oligonucleotide probes may be peptide nucleic acids in which the constituent bases are joined by peptide bonds rather than phosphodiester linkages.

The term "target nucleic acid" refers to a nucleic acid (often derived from a biological sample), to which the oligonucleotide probe is designed to specifically hybridize. It is either the presence or absence of the target nucleic acid that is to be detected, or the amount of the target nucleic acid that is to be quantified. The target nucleic acid has a sequence that is complementary to the nucleic acid sequence of the corresponding probe directed to the target. The term target nucleic acid may refer to the specific subsequence of a larger nucleic acid to which the probe is directed or to the overall sequence (*e.g.*, gene or mRNA) whose expression level it is desired to detect. The difference in usage will be apparent from context.

"Subsequence" refers to a sequence of nucleic acids that comprise a part of a longer sequence of nucleic acids.

The term "complexity"is used here according to standard meaning of this term as established by Britten *et al. Methods of Enzymol*. 29:363 (1974). See, also *Cantor and Schimmel Biophysical Chemistry: Part III* at 1228-1230 for further explanation of nucleic acid complexity.

"Bind(s) substantially" refers to complementary hybridization between a probe nucleic acid and a target nucleic acid and embraces minor mismatches that can be accommodated by reducing the stringency of the hybridization media to achieve the desired detection of the target polynucleotide sequence.

The phrase "hybridizing specifically to", refers to the binding, duplexing, or hybridizing of a molecule only to a particular nucleotide sequence under stringent conditions when that sequence is present in a complex mixture (*e*.*g*., total cellular) DNA or RNA. The term "stringent conditions" refers to conditions under which a probe will hybridize to its target subsequence, but to no other sequences. Stringent conditions are sequence-dependent and will be different in different circumstances. Longer sequences hybridize specifically at higher temperatures. Generally, stringent conditions are selected to be about 5°C lower than the thermal melting point (Tm) for the specific sequence at a defined ionic strength and pH. The Tm is the temperature (under defined ionic strength, pH, and nucleic acid concentration) at which 50% of the probes complementary to the target sequence hybridize to the target sequence at equilibrium. (As the target sequences are generally present in excess, at Tm, 50% of the probes are occupied at equilibrium). Typically, stringent conditions will be those in which the salt concentration is at least about 0.01 to 1.0 M Na ion concentration (or other salts) at pH 7.0 to 8.3 and the temperature is at least about 30°C for short probes (*e.g.*, 10 to 50 nucleotides). Stringent conditions may also be achieved with the addition of destabilizing agents such as formamide.

The term "perfect match probe" refers to a probe that has a sequence that is perfectly complementary to a particular target sequence. The test probe is typically perfectly complementary to a portion (subsequence) of the target sequence. The perfect match (PM) probe can be a "test probe", a "normalization control" probe, an expression level control probe and the like. A perfect match control or perfect match probe is, however, distinguished from a "mismatch control" or "mismatch probe."

The term "mismatch control" or "mismatch probe" refer to probes whose sequence is deliberately selected not to be perfectly complementary to a particular target sequence. For each mismatch (MM) control in a high-density array there typically exists a corresponding perfect match (PM) probe that is perfectly complementary to the same particular target sequence. The mismatch may comprise one or more bases. While the mismatch(s) may be locates anywhere in the mismatch probe, terminal mismatches are less desirable as a terminal mismatch is less likely to prevent hybridization of the target sequence. In a particularly preferred embodiment, the mismatch is located at or near the center of the probe such that the mismatch is most likely to destabilize the duplex with the target sequence under the test hybridization conditions.

The terms "background" or "background signal intensity" refer to hybridization signals resulting from non-specific binding, or other interactions, between the labeled target nucleic acids and components of the oligonucleotide array (*e*.*g*., the oligonucleotide probes, control probes, the array substrate, *etc*.). Background signals may also be produced by intrinsic fluorescence of the array components themselves. A single background signal can be calculated for the entire array, or a different background signal may be calculated for each target nucleic acid. In a preferred embodiment, background is calculated as the average hybridization signal intensity for the lowest 5% to 10% of the probes in the array, or. where a different background signal is calculated for each target gene, for the lowest 5% to 10% of the probes for each gene. Of course, one of skill in the art will appreciate that where the probes to a particular gene hybridize well and thus appear to be specifically binding to a target sequence, they should not be used in a background signal calculation. Alternatively, background may be calculated as the average hybridization signal intensity produced by hybridization to probes that are not complementary to any sequence found in the sample (*e*.*g*. probes directed to nucleic acids of the opposite sense or to genes not found in the sample such as bacterial genes where the sample is mammalian nucleic acids). Background can also be calculated as the average signal intensity produced by regions of the array that lack any probes at all.

The term "quantifying" when used in the context of quantifying transcription levels of a gene can refer to absolute or to relative quantification. Absolute quantification may be accomplished by inclusion of known concentration(s) of one or more target nucleic acids (*e.g.* control nucleic acids such as Bio B or with known amounts the target nucleic acids themselves) and referencing the hybridization intensity of unknowns with the known target nucleic acids (*e.g*. through generation of a standard curve). Alternatively, relative quantification can be accomplished by comparison of hybridization signals between two or more genes, or between two or more treatments to quantify the changes in hybridization intensity and, by implication, transcription level.

The"percentage of sequence identity" or sequence identity" is determined by comparing two optimally aligned sequences or subsequences over a comparison window or span, wherein the portion of the polynucleotide sequence in the comparison window may optionally comprise additions or deletions (*i.e.*. gaps) as compared to the reference sequence (which does not comprise additions or deletions) for optimal alignment of the two sequences. The percentage is calculated by determining the number of positions at which the identical subunit (*e*.*g*. nucleic acid base or amino acid residue) occurs in both sequences to yield the number of matched positions, dividing the number of matched positions by the total number of positions in the window of comparison and multiplying the result by 100 to yield the percentage of sequence identity. Percentage sequence identity when calculated using the programs GAP or BESTFIT (see below) is calculated using default gap weights.

Methods of alignment of sequences for comparison are well known in the art. Optimal alignment of sequences for comparison may be conducted by the local homology algorithm of Smith and Waterman, *Adv. Appl. Math.* 2: 482 (1981), by the homology alignment algorithm of Needleman and Wunsch *J. Mol. Biol.* 48: 443 (1970), by the search for similarity method of Pearson and Lipman, *Proc. Natl. Acad. Sci. USA* 85: 2444 (1988), by computerized implementations of these algorithms (including, but not limited to CLUSTAL in the PC/Gene program by Intelligenetics. Moutain View, California, GAP, BESTFIT, FASTA, and TFASTA in the Wisconsin Genetics Software Package, Genetics Computer Group (GCG), 575 Science Dr.. Madison, Wisconsin. USA), or by inspection. In particular, methods for aligning sequences using the CLUSTAL program are well described by Higgins and Sharp in *Gene.* 73: 237-244 (1988) and in *CABIOS* 5: 151-153 (1989)).

### BRIEF DESCRIPTION OF THE DRAWINGS

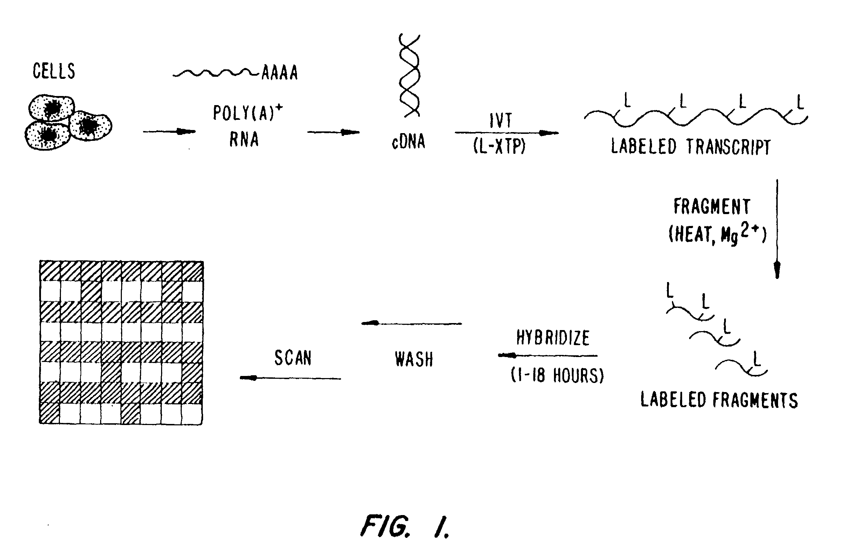

Fig. 1 shows a schematic of expression monitoring using oligonucleotide arrays. Extracted poly (A)⁺ RNA is converted to cDNA, which is then transcribed in the presence of labeled ribonucleotide triphosphates L is either biotin or a dye such as fluorescein. RNA is fragmented with heat in the presence of magnesium ions Hybridizations are carried out in a flow cell that contains the two-dimensional DNA probe arrays. Following a brief washing step to remove unhybridized RNA, the arrays are scanned using a scanning confocal microscope Alternatives in which cellular mRNA is directly labeled without a cDNA intermediate are described in the Examples Image analysis software converts the scanned array images into text files in which the observed intensities at specific physical locations are associated with particular probe sequences

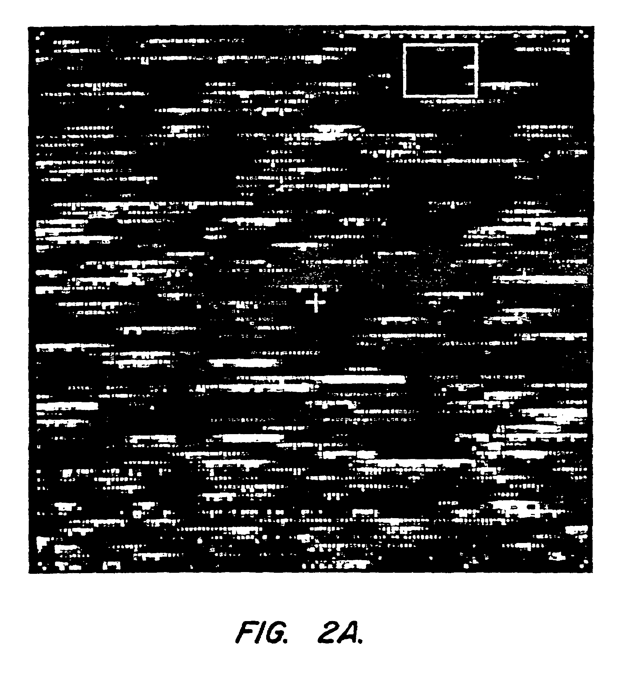

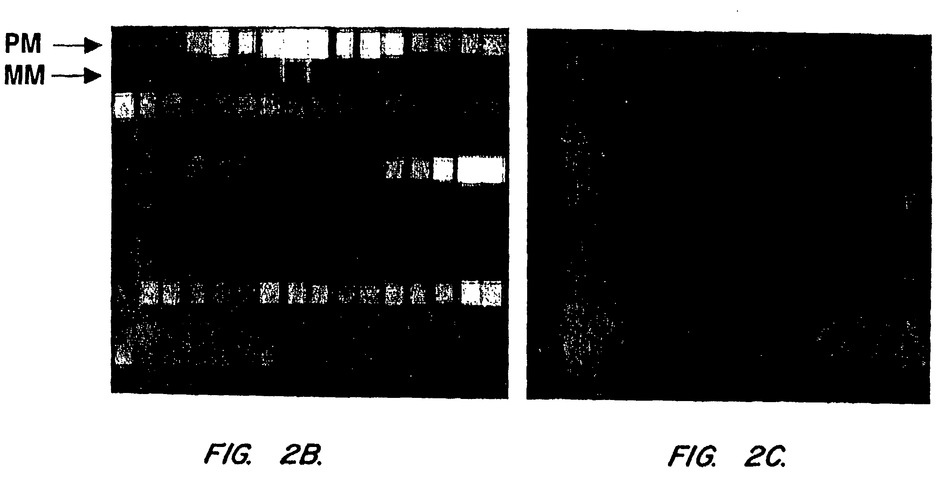

Fig. 2A shows a fluorescent image of a high density array containing over 16,000 different oligonucleotide probes The image was obtained following hybridization (15 hours at 40°C) of biotin-labeled randomly fragmented sense RNA transcribed from the murine B cell (T10) cDNA library, and spiked at the level of 1:3,000 (50 pM equivalent to about 100 copies per cell) with 13 specific RNA targets The brightness at any location is indicative of the amount of labeled RNA hybridized to the particular oligonucleotide probe. Fig 2B shows a small portion of the array (the boxed region of Fig. 2A) containing probes for IL-2 and IL-3 RNAS. For comparison, Fig 2C shows shown the same region of the array following hybridization with an unspiked T10 RNA samples (T10 cells do not express IL-2 and IL-3). The variation in the signal intensity was highly reproducible and reflected the sequence dependence of the hybridization efficiencies. The central cross and the four corners of the array contain a control sequence that is complementary to a biotin-labeled oligonucleotide that was added to the hybridization solution at a constant concentration (50 pM). The sharpness of the images near the boundaries of the features was limited by the resolution of the reading device (11.25 µm) and not by the spatial resolution of the array synthesis. The pixels in the border regions of each synthesis feature were systematically ignored in the quantitative analysis of the images.

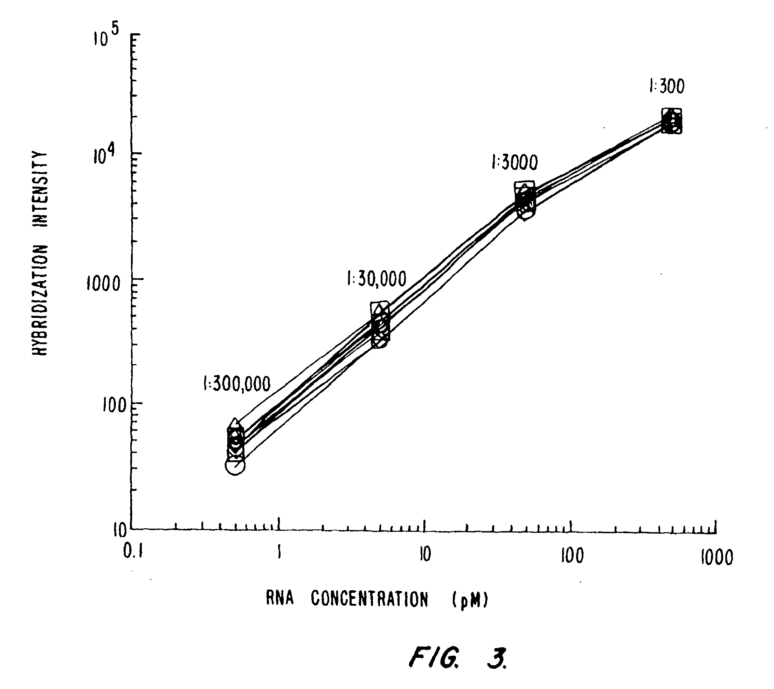

Fig. 3 provides a log/log plot of the hybridization intensity (average of the PM-MM intensity differences for each gene) versus concentration for 11 different RNA targets. The hybridization signals were quantitatively related to target concentration. The experiments were performed as described in the Examples herein and in Fig. 2. The ten 10 cytokine RNAs (plus *bioB*) were spiked into labeled T10 RNA at levels ranging from 1:300,000 to 1:3,000. The signals continued to increase with increased concentration up to frequencies of 1·300, but the response became sublinear at the high levels due to saturation of the probe sites. The linear range can be extended to higher concentrations by using shorter hybridization times. RNAs from genes expressed in T10 cells (IL-10, β-actin and GAPDH) were also detected at levels consistent with results obtained by probing cDNA libraries.

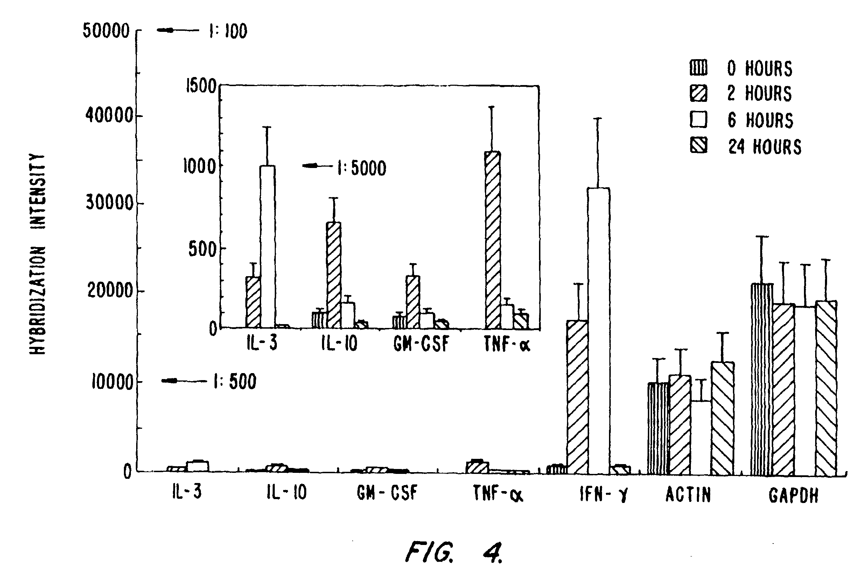

Fig. 4 shows cytokine mRNA levels in the munne 2D6 T helper cell line at different times following stimulation with PMA and a calcium ionophore. Poly (A)⁺ RNA was extracted at 0, 2, 6, and 24 hours following stimulation and converted to double stranded cDNA containing an RNA polymerase promoter. The cDNA pool was then transcribed in the presence of biotin labeled ribonucieotide triphosphates, fragmented, and hybridized to the oligonucleotide probe arrays for 2 and 22 hours. The fluorescence intensities were converted to RNA frequencies by comparison with the signals obtained for a bacterial RNA (biotin synthetase) spiked into the samples at known amounts prior to hybridization. A signal of 50,000 corresponds to a frequency of approximately 1:100,000 to a frequency of 1:5,000, and a signal of 100 to a frequency of 1:50,000. RNAs for IL-2, IL-4, IL-6, and IL-12p40 were not detected above the level of approximately 1:200,000 in these experiments. The error bars reflect the estimated uncertainty (25 percent) in the level for a given RNA relative to the level for the same RNA at a different time point The relative uncertainty estimate was based on the results of repeated spiking experiments, and on repeated measurements of IL-10, β-actin and GAPDH RNAs in preparations from both T10 and 2D6 cells (unstimulated). The uncertainty in the absolute frequencies includes message-to-message differences in the hybridization efficiency as well as differences in the mRNA isolation, cDNA synthesis, and RNA synthesis and labeling steps. The uncertainty in the absolute frequencies is estimated to be a factor of three.

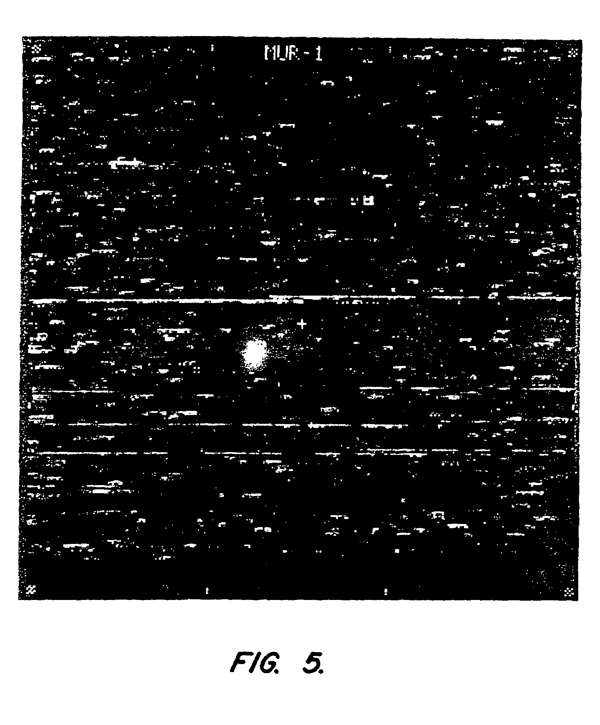

Fig 5 shows a fluorescence image of an array containing over 63,000 different oligonucleotide probes for 118 genes. The image was obtained following overnight hybridization of a labeled murine B cell RNA sample. Each square synthesis region is 50 x 50 µm and contains 107 to 108 copies of a specific oligonucleotide. The array was scanned at a resolution of 7.5 µm in approximately 15 minutes. The bright rows indicate RNAs present at high levels. Lower level RNAs were unambiguously detected based on quantitative evaluation of the hybridization patterns. A total of 21 murine RNAs were detected at levels ranging from approximately 1:300,000 to 1:100. The cross in the center, the checkerboard in the corners, and the MUR-1 region at the top contain probes complementary to a labeled control oligonucleotide that was added to all samples.

Fig 6 shows an example of a computer system used to execute the software of an embodiment of the present invention.

Fig 7 shows a system block diagram of a typical computer system used to execute the software of an embodiment of the present invention.

Fig 8 shows the high level flow of a process of monitoring the expression of a gene by comparing hybridization intensities of pairs of perfect match and mismatch probes.

Fig 9 shows the flow of a process of determining if a gene is expressed utilizing a decision matrix.

Figs. 10A and 10B show the flow of a process of determining the expression of a gene by comparing baseline scan data and experimental scan data.

Fig. 11 shows the flow of a process of increasing the number of probes for monitoring the expression of genes after the number of probes has been reduced or pruned.

### DETAILED DESCRIPTION

### I. High Density Arrays For Monitoring Gene Expression

This invention provides methods of monitoring (detecting and/or quantifying) the expression levels of a multiplicity of genes. The methods involve hybridization of a nucleic acid target sample to a high density array of nucleic acid probes and then quantifying the amount of target nucleic acids hybridized to each probe in the array.

While nucleic acid hybridization has been used for some time to determine the expression levels of various genes (*e.g*., Northern Blot), it was a surprising discovery of this invention that high density arrays are suitable for the quantification of the small variations in expression (transcription) levels of a gene in the presence of a large population of heterogenous nucleic acids. The signal may be present at a concentration of less than about 1 in 1,000, and is often present at a concentration less than 1 in 10,000 more preferably less than about 1 in 50,000 and most preferably less than about 1 in 100,000, 1 in 300,000, or even 1 in 1,000,000.

Prior to this invention, it was expected that hybridization of such a complex mixture to a high density array might overwhelm the available probes and make it impossible to detect the presence of low-level target nucleic acids. It was thus unclear that a low level signal could be isolated and detected in the presence of misleading signals due to cross-hybridization and non-specific binding both to substrate and probe. It was therefore a surprising discovery that, to the contrary, high density arrays are particularly well suited for monitoring expression of a multiplicity of genes and provide a level of sensitivity and discrimination hitherto unexpected.

It was also a surprising discovery of this invention that when used in a high-density array, even relatively short oligonucleotides can be used to accurately detect and quantify expression (transcription) levels of genes. Thus oligonucleotide arrays having oligonucleotides as short as 10 nucleotides, more preferably 15 oligonucleotides and most preferably 20 or 25 oligonucleotides are used to specifically detect and quantify gene expression levels. Of course arrays containing longer oligonucleotides. as described herein, are also suitable.

### A) Advantages of Oligonucleotide Arrays

In one preferred embodiment, the high density arrays used in the methods of this invention comprise chemically synthesized oligonucleotides. The use of chemically synthesized oligonucleotide arrays, as opposed to. for example, blotted arrays of genomic clones, restriction fragments, oligonucleotides, and the like, offers numerous advantages. These advantages generally fall into four categories:
1) Efficiency of production;
2) Reduced intra- and inter-array variability;
3) Increased information content; and
4) Higher signal to noise ratio (improved sensitivity).

### 1) Efficiency of production.

In a preferred embodiment, the arrays are synthesized using methods of spatially addressed parallel synthesis (*see, e.g*., Section V, below). The oligonucleotides are synthesized chemically in a highly parallel fashion covalently attached to the array surface. This allows extremely efficient array production. For example, arrays containing tens (or even hundreds) of thousands of specifically selected 20 mer oligonucleotides are synthesized in fewer than 80 synthesis cycles. The arrays are designed and synthesized based on sequence information alone. Thus, unlike blotting methods, the array preparation requires no handling of biological materials. There is no need for cloning steps, nucleic acid amplifications, cataloging of clones or amplification products, and the like. The preferred chemical synthesis of expression monitoring arrays in this invention is thus more efficient blotting methods and permits the production of highly reproducible high-density arrays with relatively little labor and expense.

### 2) Reduced intra- and inter-array variability.

The use of chemically synthesized high-density oligonucleotide arrays in the methods of this invention improves intra- and inter-array variability. The oligonucleotide arrays preferred for this invention are made in large batches (presently 49 arrays per wafer with multiple wafers synthesized in parallel) in a highly controlled reproducible manner. This makes them suitable as general diagnostic and research tools permitting direct comparisons of assays performed anywhere in the world.

Because of the precise control obtainable during the chemical synthesis the arrays of this invention show less than about 25%, preferably less than about 20%, more preferably less than about 15%, still more preferably less than about 10%, even more preferably less than about 5%, and most preferably less than about 2% variation between high density arrays (within or between production batches) having the same probe composition. Array variation is assayed as the variation in hybridization intensity (against a labeled control target nucleic acid mixture) in one or more oligonucleotide probes between two or more arrays. More preferably, array variation is assayed as the variation in hybridization intensity (against a labeled control target nucleic acid mixture) measured for one or more target genes between two or more arrays.

In addition to reducing inter- and intra-array variability, chemically synthesized arrays also reduce variations in relative probe frequency inherent in spotting methods, particularly spotting methods that use cell-derived nucleic acids (*e.g.*, cDNAs). Many genes are expressed at the level of thousands of copies per cell, while others are expressed at only a single copy per cell. A cDNA library will reflect this very large bias as will a cDNA library made from theis material. While normalization (adjustment of the amount of each different probe *e*.*g*., by comparison to a reference cDNA) of the library will reduce the representation of over-expressed sequences, normalization has been shown to lessen the odds of selecting highly expressed cDNAs by only about a factor of 2 or 3. In contrast, chemical synthesis methods can insure that all oligonucleotide probes are represented in approximately equal concentrations. This decreases the inter-gene (intra-array) variability and permits direct comparison between characteristically overexpressed and underexpressed nucleic acids.

### 3) Increased information content.

As indicated above, it was a discovery of this invention that the use of high density oligonucleotide arrays for expression monitoring provides a number of advantages not found with other methods. For example, the use of large numbers of different probes that specifically bind to the transcription product of a particular target gene provides a high degree of redundancy and internal control that permits optimization of probe sets for effective detection of particular target genes and minimizes the possibility of errors due to cross-reactivity with other nucleic acid species.

Apparently suitable probes often prove ineffective for expression monitoring by hybridization. For example, certain subsequences of a particular target gene may be found in other regions of the genome and probes directed to these subsequences will cross-hybridize with the other regions and not provide a signal that is a meaningful measure of the expression level of the target gene. Even probes that show little cross reactivity may be unsuitable because they generally show poor hybridization due to the formation of structures that prevent effective hybridization. Finally, in sets with large numbers of probes, it is difficult to identify hybridization conditions that are optimal for all the probes in a set. Because of the high degree of redundancy provided by the large number of probes for each target gene, it is possible to eliminate those probes that function poorly under a given set of hybridization conditions and still retain enough probes to a particular target gene to provide an extremely sensitive and reliable measure of the expression level (transcription level) of that gene.

In addition, the use of large numbers of different probes to each target gene makes it possible to monitor expression of families of closely-related nucleic acids. The probes may be selected to hybridize both with subsequences that are conserved across the family and with subsequences that differ in the different nucleic acids in the family. Thus, hybridization with such arrays permits simultaneous monitoring of the various members of a gene family even where the various genes are approximately the same size and have high levels of homology. Such measurements are difficult or impossible with traditional hybridization methods.

Because the high density arrays contain such a large number of probes it is possible to provide numerous controls including, for example, controls for variations or mutations in a particular gene, controls for overall hybridization conditions, controls for sample preparation conditions, controls for metabolic activity of the cell from which the nucleic acids are derived and mismatch controls for non-specific binding or cross hybridization.

Moreover, as explained above, it was a surprising discovery of this invention that effective detection and quantitation of gene transcription in complex mammalian cell message populations can be determined with relatively short oligonucleotides and with relative few (*e*.*g*., fewer than 40, preferably fewer than 30, more preferably fewer than 25, and most preferably fewer than 20, 15, or even 10) oligonucleotide probes per gene. In general, it was a discovery of this invention that there are a large number of probes which hybridize both strongly and specifically for each gene. This does not mean that a large number of probes is required for detection, but rather that there are many from which to choose and that choices can be based on other considerations such as sequence uniqueness (gene families), checking for splice variants, or genotyping hot spots (things not easily done with cDNA spotting methods).

Based on these discoveries, sets of four arrays are made that contain approximately 400,000 probes each. Sets of about 40 probes (20 probe pairs) are chosen that are complementary to each of about 40,000 genes for which there are ESTs in the public database. This set of ESTs covers roughly one-third to one-half of all human genes and these arrays will allow the levels of all of them to be monitored in a parallel set of overnight hybridizations.

### 4) Improved signal to noise ratio.

Blotted nucleic acids typically rely on ionic, electrostatic, and hydrophobic interactions to attach the blotted nucleic acids to the substrate. Bonds are formed at multiple points along the nucleic acid restricting degrees of freedom and interferign with the ability of the nucleic acid to hybridize to its complementary target. In contrast, the preferred arrays of this invention are chemically synthesized. The oligonucleotide probes are attached to the substrate by a single terminal covalent bond. The probes have more degrees of freedom and are capable of participating in complex interactions with their complementary targets. Consequently, such probe arrays show significantly higher hybridization efficiencies (10 times, 100 times, and even 1000 times more effecient) than blotted arrays. Less target oligonucleotide is used to produce a given signal thereby dramatically improving the signal to noise ratio. Consequently the methods of this invention permit detection of only a few copies of a nucleic acid in extremely complex nucleic acid mixtures.

### B) Preferred High Density Arrays

Preferred high density arrays of this invention comprise greater than about 100, preferably greater than about 1000, more preferably greater than about 16,000 and most preferably greater than about 65,000 or 250,000 or even greater than about 1,000,000 different oligonucleotide probes. The oligonucleotide probes range from about 5 to about 50 or about 5 to about 45 nucleotides, more preferably from about 10 to about 40 nucleotides and most preferably from about 15 to about 40 nucleotides in length. In particular preferred embodiments, the oligonucleotide probes are 20 or 25 nucleotides in length. It was a discovery of this invention that relatively short oligonucleotide probes sufficient to specifically hybridize to and distinguish target sequences. Thus in one preferred embodiment, the oligonucleotide probes are less than 50 nucleotides in length, generally less than 46 nucleotides, more generally less than 41 nucleotides, most generally less than 36 nucleotides, preferably less than 31 nucleotides. more preferably less than 26 nucleotides and most preferably less than 21 nucleotides in length. The probes can also be less than 16 nucleotides or less than even 11 nucleotides in length.

The location and sequence of each different oligonucleotide probe sequence in the array is known. Moreover, the large number of different probes occupies a relatively small area providing a high density array having a probe density of generally greater than about 60, more generally greater than about 100, most generally greater than about 600, often greater than about 1000, more often greater than about 5,000, most often greater than about 10,000, preferably greater than about 40,000 more preferably greater than about 100,000, and most preferably greater than about 400,000 different oligonucleotide probes per cm². The small surface area of the array (often less than about 10 cm², preferably less than about 5 cm² more preferably less than about 2 cm², and most preferably less than about 1.6 cm²) permits extremely uniform hybridization conditions (temperature regulation, salt content, *etc.*) while the extremely large number of probes allows massively parallel processing of hybridizations.

Finally, because of the small area occupied by the high density arrays, hybridization may be carried out in extremely small fluid volumes (*e.g*., 250 µl or less, more preferably 100 µl or less, and most preferably 10 µl or less). In small volumes, hybridization may proceed very rapidly. In addition, hybridization conditions are extremely uniform throughout the sample, and the hybridization format is amenable to automated processing.

### II. Uses of Expression monitoring.

This invention demonstrates that hybridization with high density oligonucleotide probe arrays provides an effective means of monitoring expression of a multiplicity of genes. In addition this invention provides for methods of sample treatment and array designs and methods of probe selection that optimize signal detection at extremely low concentrations in complex nucleic acid mixtures.

The expression monitoring methods of this invention may be used in a wide variety of circumstances including detection of disease, identification of differential gene expression between two samples (*e*.*g*., a pathological as compared to a healthy sample), screening for compositions that upregulate or downregulate the expression of particular genes, and so forth.

In one preferred embodiment, the methods of this invention are used to monitor the expression (transcription) levels of nucleic acids whose expression is altered in a disease state. For example, a cancer may be characterized by the overexpression of a particular marker such as the HER2 (c-erbB-2/neu) proto-oncogene in the case of breast cancer. Similarly, overexpression of receptor tyrosine kinases (RTKs) is associated with the etiology of a number of tumors including carcinomas of the breast, liver, bladder, pancreas, as well as glioblastomas, sarcomas and squamous carcinomas *(see* Carpenter, *Ann. Rev. Biochem.,* 56: 881-914 (1987)). Conversely, a cancer (*e.g.*, colerectal, lung and breast) may be characterized by the mutation of or underexpression of a tumor suppressor gene such as P53 (*see, e.g*., Tominaga *et al. Critical Rev. in Oncogenesis,* 3: 257-282 (1992)).

In another preferred embodiment, the methods of this invention are used to monitor expression of various genes in response to defined stimuli, such as a drug. The methods are particularly advantageous because they permit simultaneous monitoring of the expression of thousands of genes. This is especially useful in drug research if the end point description is a complex one, not simply asking if one particular gene is overexpressed or underexpressed. Thus, where a disease state or the mode of action of a drug is not well characterized, the methods of this invention allow rapid determination of the particularly relevant genes.

As indicated above, the materials and methods of this invention are typically used to monitor the expression of a multiplicity of different genes simultaneously. Thus, in one embodiment, the invention provide for simultaneous monitoring of at least about 10, preferably at least about 100, more preferably at least about 1000, still more preferably at least about 10,000, and most preferably at least about 100,000 different genes.

The expression monitoring methods of this invention can also be used for gene discovery. Many genes that have been discovered to date have been classified into families based on commonality of the sequences. Because of the extremely large number of probes it is possible to place in the high density array, it is possible to include oligonucleotide probes representing known or parts of known members from every gene class. In utilizing such a "chip" (high density array) genes that are already known would give a positive signal at loci containing both variable and common regions. For unknown genes, only the common regions of the gene family would give a positive signal. The result would indicate the possibility of a newly discovered gene.

The expression monitoring methods of this invention also allow the development of "dynamic" gene databases. The Human Genome Project and commercial sequencing projects have generated large static databases which list thousands of sequences without regard to function or genetic interaction. Expression analysis using the methods of this invention produces "dynamic" databases that define a gene's function and its interactions with other genes. Without the ability to monitor the expression of large numbers of genes simultaneously, however, the work of creating such a database is enormous. The tedious nature of using DNA sequence analysis for determining an expression pattern involves preparing a cDNA library from the RNA isolated from the cells of interest and then sequencing the library. As the DNA is sequenced, the operator lists the sequences that are obtained and counts them. Thousands of sequences would have to be determined and then the frequency of those gene sequences would define the expression pattern of genes for the cells being studied.

By contrast, using an expression monitoring array to obtain the data according to the methods of this invention is relatively fast and easy. The process involves stimulating the cells to induce expression, obtaining the RNA from the cells and then either labeling the RNA directly or creating a cDNA copy of the RNA. If cDNA is to be hybridized to the chip, fluorescent molecules are incorporated during the DNA polymerization. Either the labeled RNA or the labeled cDNA is then hybridized to a high density array in one overnight experiment. The hybridization provides a quantitative assessment of the levels of every single one of the genes with no additional sequencing. In addition the methods of this invention are much more sensitive allowing a few copies of expressed genes per cell to be detected. This procedure is demonstrated in the examples provided herein.

### III. Methods of monitoring gene expression.

Generally the methods of monitoring gene expression of this invention involve (1) providing a pool of target nucleic acids comprising RNA transcript(s) of one or more target gene(s), or nucleic acids derived from the RNA transcript(s); (2) hybridizing the nucleic acid sample to a high density array of probes (including control probes); and (3) detecting the hybridized nucleic acids and calculating a relative expression (transcription) level.

### A) Providing a nucleic acid sample.

One of skill in the art will appreciate that in order to measure the transcription level (and thereby the expression level) of a gene or genes, it is desirable to provide a nucleic acid sample comprising mRNA transcript(s) of the gene or genes, or nucleic acids derived from the mRNA transcript(s). As used herein, a nucleic acid derived from an mRNA transcript refers to a nucleic acid for whose synthesis the mRNA transcript or a subsequence thereof has ultimately served as a template. Thus, a cDNA reverse transcribed from an mRNA, an RNA transcribed from that cDNA, a DNA amplified from the cDNA, an RNA transcribed from the amplified DNA, *etc.,* are all derived from the mRNA transcript and detection of such derived products is indicative of the presence and/or abundance of the original transcript in a sample. Thus, suitable samples include, but are not limited to, mRNA transcripts of the gene or genes, cDNA reverse transcribed from the mRNA, cRNA transcribed from the cDNA, DNA amplified from the genes, RNA transcribed from amplified DNA, and the like.

In a particularly preferred embodiment, where it is desired to quantify the transcription level (and thereby expression) of a one or more genes in a sample, the nucleic acid sample is one in which the concentration of the mRNA transcript(s) of the gene or genes, or the concentration of the nucleic acids derived from the mRNA transcript(s), is proportional to the transcription level (and therefore expression level) of that gene. Similarly, it is preferred that the hybridization signal intensity be proportional to the amount of hybridized nucleic acid. While it is preferred that the proportionality be relatively strict (*e.g*., a doubling in transcription rate results in a doubling in mRNA transcript in the sample nucleic acid pool and a doubling in hybridization signal), one of skill will appreciate that the proportionality can be more relaxed and even non-linear. Thus, for example, an assay where a 5 fold difference in concentration of the target mRNA results in a 3 to 6 fold difference in hybridization intensity is sufficient for most purposes. Where more precise quantification is required appropriate controls can be run to correct for variations introduced in sample preparation and hybridization as described herein. In addition, serial dilutions of "standard" target mRNAs can be used to prepare calibration curves according to methods well known to those of skill in the art. Of course, where simple detection of the presence or absence of a transcript is desired, no elaborate control or calibration is required.

In the simplest embodiment, such a nucleic acid sample is the total mRNA isolated from a biological sample. The term "biological sample", as used herein, refers to a sample obtained from an organism or from components (*e*.*g*., cells) of an organism. The sample may be of any biological tissue or fluid. Frequently the sample will be a "clinical sample" which is a sample derived from a patient. Such samples include, but are not limited to, sputum, blood, blood cells (e.g., white cells), tissue or fine needle biopsy samples, urine, peritoneal fluid, and pleural fluid, or cells therefrom. Biological samples may also include sections of tissues such as frozen sections taken for histological purposes.

The nucleic acid (either genomic DNA or mRNA) may be isolated from the sample according to any of a number of methods well known to those of skill in the art. One of skill will appreciate that where alterations in the copy number of a gene are to be detected genomic DNA is preferably isolated. Conversely, where expression levels of a gene or genes are to be detected, preferably RNA (mRNA) is isolated.

Methods of isolating total mRNA are well known to those of skill in the art. For example, methods of isolation and purification of nucleic acids are described in detail in Chapter 3 of *Laboratory Techniques in Biochemistry and Molecular Biology: Hybridization With Nucleic Acid Probes. Part 1. Theory and Nucleic Acid Preparation,* P. Tijssen, ed. Elsevier, N.Y. (1993) and Chapter 3 of *Laboratory Techniques in Biochemistry and Molecular Biology: Hybridization With Nucleic Acid Probes, Part 1. Theory and Nucleic Acid Preparation*, P. Tijssen, ed. Elsevier, N.Y. (1993)).

In a preferred embodiment, the total nucleic acid is isolated from a given sample using, for example, an acid guanidinium-phenol-chloroform extraction method and polyA⁺ mRNA is isolated by oligo dT column chromatography or by using (dT)n magnetic beads (*see*, *e*.*g*., Sambrook *et al*., *Molecular Cloning: A Laboratory Manual* (2nd ed.), Vols. 1-3, Cold Spring Harbor Laboratory, (1989), or *Current Protocols in Molecular Biology,* F. Ausubel *et al*., ed. Greene Publishing and Wiley-Interscience. New York (1987)).

Frequently, it is desirable to amplify the nucleic acid sample prior to hybridization. One of skill in the art will appreciate that whatever amplification method is used, if a quantitative result is desired, care must be taken to use a method that maintains or controls for the relative frequencies of the amplified nucleic acids.

Methods of "quantitative" amplification are well known to those of skill in the art. For example, quantitative PCR involves simultaneously co-amplifying a known quantity of a control sequence using the same primers. This provides an internal standard that may be used to calibrate the PCR reaction. The high density array may then include probes specific to the internal standard for quantification of the amplified nucleic acid.

One preferred internal standard is a synthetic AW106 cRNA. The AW106 cRNA is combined with RNA isolated from the sample according to standard techniques known to those of skill in the art. The RNA is then reverse transcribed using a reverse transcriptase to provide copy DNA. The cDNA sequences are then amplified (*e*.*g*., by PCR) using labeled primers. The amplification products are separated, typically by electrophoresis, and the amount of radioactivity (proportional to the amount of amplified product) is determined. The amount of mRNA in the sample is then calculated by comparison with the signal produced by the known AW106 RNA standard. Detailed protocols for quantitative PCR are provided in *PCR Protocols, A Guide to Methods and Applications*, Innis *et al*., Academic Press, Inc. N.Y., (1990).

Other suitable amplification methods include, but are not limited to polymerase chain reaction (PCR) (Innis, *et al*., *PCR Protocols. A guide to Methods and Application.* Academic Press, Inc. San Diego, (1990)), ligase chain reaction (LCR) (see Wu and Wallace, *Genomics*, 4: 560 (1989), Landegren, *et al*., *Science*, 241: 1077 (1988) and Barringer, *et al*., *Gene,* 89: 117 (1990). transcription amplification (Kwoh, et al., *Proc. Natl. Acad. Sci. USA,* 86: 1173 (1989)), and self-sustained sequence replication (Guatelli, *et al*., *Proc. Nat. Acad. Sci. USA*, 87: 1874 (1990)).

In a particularly preferred embodiment, the eample mRNA is reverse transcribed with a reverse transcriptase and a primer consisting of oligo dT and a sequence encoding the phage T7 promoter to provide single stranded DNA template. The second DNA strand is polymerized using a DNA polymerase. After synthesis of double-stranded cDNA, T7 RNA polymerase is added and RNA is transcribed from the cDNA template. Successive rounds of transcription from each single cDNA template results in amplified RNA. Methods of *in vitro* polymerization are well known to those of skill in the art (*see, e.g.*, Sambrook, *supra.*) and this particular method is described in detail by Van Gelder, *et al., Proc. Natl. Acad. Sci. USA*, 87: 1663-1667 (1990) who demonstrate that *in vitro* amplification according to this method preserves the relative frequencies of the various RNA transcripts. Moreover, Eberwine *et al. Proc. Natl. Acad. Sci. USA.* 89: 3010-3014 provide a protocol that uses two rounds of amplification via *in vitro* transcription to achieve greater than 10° fold amplification of the original starting material thereby permitting expression monitoring even where biological samples are limited.

It will be appreciated by one of skill in the art that the direct transcription method described above provides an antisense (aRNA) pool. Where antisense RNA is used as the target nucleic acid, the oligonucleotide probes provided in the array are chosen to be complementary to subsequences of the antisense nucleic acids. Conversely. where the target nucleic acid pool is a pool of sense nucleic acids, the oligonucleotide probes are selected to be complementary to subsequences of the sense nucleic acids. Finally, where the nucleic acid pool is double stranded, the probes may be of either sense as the target nucleic acids include both sense and antisense strands.

The protocols cited above include methods of generating pools of either sense or antisense nucleic acids. Indeed, one approach can be used to generate either sense or antisense nucleic acids as desired. For example, the cDNA can be directionally cloned into a vector (*e.g*., Stratagene's p Bluscript II KS (+) phagemid) such that it is flanked by the T3 and T7 promoters. *In vitro* transcription with the T3 polymerase will produce RNA of one sense (the sense depending on the orientation of the insert), while *in vitro* transcription with the T7 polymerase will produce RNA having the opposite sense. Other suitable cloning systems include phage lambda vectors designed for Cre-*loxP* plasmid subcloning (*see e*.*g*., Palazzolo *et al., Gene*, 88: 25-36 (1990)).

In a particularly preferred embodiment, a high activity RNA polymerase (*e*.*g*. about 2500 units/µL for T7, available from Epicentre Technologies) is used.

### B) Labeling nucleic acids.

In a preferred embodiment, the hybridized nucleic acids are detected by detecting one or more labels attached to the sample nucleic acids. The labels may be incorporated by any of a number of means well known to those of skill in the art. However, in a preferred embodiment, the label is simultaneously incorporated during the amplification step in the preparation of the sample nucleic acids. Thus, for example, polymerase chain reaction (PCR) with labeled primers or labeled nucleotides will provide a labeled amplification product. In a preferred embodiment, transcription amplification, as described above, using a labeled nucleotide (*e*.*g*. fluorescein-labeled UTP and/or CTP) incorporates a label into the transcribed nucleic acids.

Alternatively, a label may be added directly to the original nucleic acid sample (*e*.*g*., mRNA, polyA mRNA, cDNA, *etc*.) or to the amplification product after the amplification is completed. Means of attaching labels to nucleic acids are well known to those of skill in the art and include, for example nick translation or end-labeling (*e*.*g*. with a labeled RNA) by kinasing of the nucleic acid and subsequent attachment (ligation) of a nucleic acid linker joining the sample nucleic acid to a label (*e*.*g*., a fluorophore).

Detectable labels suitable for use in the present invention include any composition detectable by spectroscopic, photochemical, biochemical, immunochemical, electrical, optical or chemical means. Useful labels in the present invention include biotin for staining with labeled streptavidin conjugate, magnetic beads (*e*.*g*., Dynabeads™), fluorescent dyes (*e.g.*, fluorescein, texas red, rhodamine, green fluorescent protein, and the like), radiolabels (*e*.*g*., ³H, ¹²⁵I, ³⁵S, ¹⁴C, or ³²P), enzymes (*e.g.,* horse radish peroxidase, alkaline phosphatase and others commonly used in an ELISA), and colorimetric labels such as colloidal gold or colored glass or plastic (*e*.*g*., polystyrene, polypropylene, latex, etc.) beads. Patents teaching the use of such labels include U.S. Patent Nos. 3,817,837; 3,850,752; 3,939,350; 3,996,345; 4,277,437; 4,275,149; and 4,366,241.

Means of detecting such labels are well known to those of skill in the art. Thus, for example, radiolabels may be detected using photographic film or scintillation counters, fluorescent markers may be detected using a photodetector to detect emitted light. Enzymatic labels are typically detected by providing the enzyme with a substrate and detecting the reaction product produced by the action of the enzyme on the substrate, and colorimetric labels are detected by simply visualizing the colored label.

The label may be added to the target (sample) nucleic acid(s) prior to, or after the hybridization. So called "direct labels" are detectable labels that are directly attached to or incorporated into the target (sample) nucleic acid prior to hybridization. In contrast, so called "indirect labels" are joined to the hybrid duplex after hybridization. Often, the indirect label is attached to a binding moiety that has been attached to the target nucleic acid prior to the hybridization. Thus, for example, the target nucleic acid may be biotinylated before the hybridization. After hybridization, an aviden-conjugated fluorophore will bind the biotin bearing hybrid duplexes providing a label that is easily detected. For a detailed review of methods of labeling nucleic acids and detecting labeled hybridized nucleic acids see *Laboratory Techniques in Biochemistry and Molecular Biology, Vol. 24: Hybridization With Nucleic Acid Probes*, P. Tijssen, ed. Elsevier, N.Y., (1993)).

Fluorescent labels are preferred and easily added during an *in vitro* transcription reaction. In a preferred embodiment, fluorescein labeled UTP and CTP are incorporated into the RNA produced in an *in vitro* transcription reaction as described above.

### C) Modifying sample to improve signal/noise ratio.

The nucleic acid sample may be modified prior to hybridization to the high density probe array in order to reduce sample complexity thereby decreasing background signal and improving sensitivity of the measurement. In one embodiment, complexity reduction is achieved by selective degradation of background mRNA. This is accomplished by hybridizing the sample mRNA (*e.g*., polyA⁺ RNA) with a pool of DNA oligonucleotides that hybridize specifically with the regions to which the probes in the array specifically hybridize. In a preferred embodiment, the pool of oligonucleotides consists of the same probe oligonucleotides as found on the high density array.

The pool of oligonucleotides hybridizes to the sample mRNA forming a number of double stranded (hybrid duplex) nucleic acids. The hybridized sample is then treated with RNase A, a nuclease that specifically digests single stranded RNA. The RNase A is then inhibited, using a protease and/or commercially available RNase inhibitors, and the double stranded nucleic acids are then separated from the digested single stranded RNA. This separation may be accomplished in a number of ways well known to those of skill in the art including, but not limited to, electrophoresis, and gradient centrifugation. However, in a preferred embodiment, the pool of DNA oligonucleotides is provided attached to beads forming thereby a nucleic acid affinity column. After digestion with the RNase A, the hybridized DNA is removed simply by denaturing (*e.g*., by adding heat or increasing salt) the hybrid duplexes and washing the previously hybridized mRNA off in an elution buffer.

The undigested mRNA fragments which will be hybridized to the probes in the high density array are then preferably end-labeled with a fluorophore attached to an RNA linker using an RNA ligase. This procedure produces a labeled sample RNA pool in which the nucleic acids that do not correspond to probes in the array are eliminated and thus unavailable to contribute to a background signal.

Another method of reducing sample complexity involves hybridizing the mRNA with deoxyoligonucleotides that hybridize to regions that border on either size the regions to which the high density array probes are directed. Treatment with RNAse H selectively digests the double stranded (hybrid duplexes) leaving a pool of single-stranded mRNA corresponding to the short regions (*e.g*., 20 mer) that were formerly bounded by the deoxyoligonucleotide probes and which correspond to the targets of the high density array probes and longer mRNA sequences that correspond to regions between the targets of the probes of the high density array. The short RNA fragments are then separated from the long fragments (*e.g*., by electrophoresis), labeled if necessary as described above, and then are ready for hybridization with the high density probe array.

In a third approach, sample complexity reduction involves the selective removal of particular (preselected) mRNA messages. In particular, highly expressed mRNA messages that are not specifically probed by the probes in the high density array are preferably removed. This approach involves hybridizing the polyA⁺ mRNA with an oligonucleotide probe that specifically hybridizes to the preselected message close to the 3' (poly A) end. The probe may be selected to provide high specificity and low cross reactivity. Treatment of the hybridized message/probe complex with RNase H digests the double stranded region effectively removing the polyA⁺ tail from the rest of the message. The sample is then treated with methods that specifically retain or amplify polyA⁺ RNA (*e*.*g*., an oligo dT column or (dT)n magnetic beads). Such methods will not retain or amplify the selected message(s) as they are no longer associated with a polyA⁺ tail. These highly expressed messages are effectively removed from the sample providing a sample that has reduced background mRNA.

### IV. Hybridization Array Design.

### A) Probe composition.

One of skill in the art will appreciate that an enormous number of array designs are suitable for the practice of this invention. The high density array will typically include a number of probes that specifically hybridize to the nucleic acid(s) expression of which is to be detected. In addition, in a preferred embodiment, the array will include one or more control probes.

### 1) Test probes.

In its simplest embodiment, the high density array includes "test probes". These are oligonucleotides that range from about 5 to about 45 or 5 to about 50 nucleotides, more preferably from about 10 to about 40 nucleotides and most preferably from about 15 to about 40 nucleotides in length. In other particularly preferred embodiments the probes are 20 or 25 nucleotides in length. These oligonucleotide probes have sequences complementary to particular subsequences of the genes whose expression they are designed to detect. Thus, the test probes are capable of specifically hybridizing to the target nucleic acid they are to detect.

In addition to test probes that bind the target nucleic acid(s) of interest. the high density array can contain a number of control probes. The control probes fall into three categories referred to herein as 1) Normalization controls; 2) Expression level controls; and 3) Mismatch controls.

### 2) Normalization controls.

Normalization controls are oligonucleotide probes that are perfectly complementary to labeled reference oligonucleotides that are added to the nucleic acid sample. The signals obtained from the normalization controls after hybridization provide a control for variations in hybridization conditions, label intensity, "reading" efficiency and other factors that may cause the signal of a perfect hybridization to vary between arrays. In a preferred embodiment, signals (*e*.*g*., fluorescence intensity) read from all other probes in the array are divided by the signal (*e.g*., fluorescence intensity) from the control probes thereby normalizing the measurements.

Virtually any probe may serve as a normalization control. However, it is recognized that hybridization efficiency varies with base composition and probe length. Preferred normalization probes are selected to reflect the average length of the other probes present in the array, however, they can be selected to cover a range of lengths. The normalization control(s) can also be selected to reflect the (average) base composition of the other probes in the array, however in a preferred embodiment, only one or a few normalization probes are used and they are selected such that they hybridize well (*i.e*. no secondary structure) and do not match any target-specific probes.

Normalization probes can be localized at any position in the array or at multiple positions throughout the array to control for spatial variation in hybridization efficiently. In a preferred embodiment, the normalization controls are located at the corners or edges of the array as well as in the middle.

### 3) Expression level controls,

Expression level controls are probes that hybridize specifically with constitutively expressed genes in the biological sample. Expression level controls are designed to control for the overall health and metabolic activity of a cell. Examination of the covariance of an expression level control with the expression level of the target nucleic acid indicates whether measured changes or variations in expression level of a gene is due to changes in transcription rate of that gene or to general variations in health of the cell. Thus, for example, when a cell is in poor health or lacking a critical metabolite the expression levels of both an active target gene and a constitutively expressed gene are expected to decrease. The converse is also true. Thus where the expression levels of both an expression level control and the target gene appear to both decrease or to both increase, the change may be attributed to changes in the metabolic activity of the cell as a whole, not to differential expression of the target gene in question. Conversely, where the expression levels of the target gene and the expression level control do not covary, the variation in the expression level of the target gene is attributed to differences in regulation of that gene and not to overall variations in the metabolic activity of the cell.

Virtually any constitutively expressed gene provides a suitable target for expression level controls. Typically expression level control probes have sequences complementary to subsequences of constitutively expressed "housekeeping genes" including, but not limited to the β-actin gene, the transferrin receptor gene, the GAPDH gene, and the like.

### 4) Mismatch controls.

Mismatch controls may also be provided for the probes to the target genes, for expression level controls or for normalization controls. Mismatch controls are oligonucleotide probes identical to their corresponding test or control probes except for the presence of one or more mismatched bases. A mismatched base is a base selected so that it is not complementary to the corresponding base in the target sequence to which the probe would otherwise specifically hybridize. One or more mismatches are selected such that under appropriate hybridization conditions (e.g. stringent conditions) the test or control probe would be expected to hybridize with its target sequence, but the mismatch probe would not hybridize (or would hybridize to a significantly lesser extent). Preferred mismatch probes contain a central mismatch. Thus, for example, where a probe is a 20 mer, a corresponding mismatch probe will have the identical sequence except for a single base mismatch (*e.g*., substituting a G, a C or a T for an A) at any of positions 6 through 14 (the central mismatch).

Mismatch probes thus provide a control for non-specific binding or cross-hybridization to a nucleic acid in the sample other than the target to which the probe is directed. Mismatch probes thus indicate whether a hybridization is specific or not. For example, if the target is present the perfect match probes should be consistently brighter than the mismatch probes. In addition, if all central mismatches are present, the mismatch probes can be used to detect a mutation. Finally, it was also a discovery of the present invention that the difference in intensity between the perfect match and the mismatch probe (I(PM)-I(MM)) provides a good measure of the concentration of the hybridized material.

### 5) Sample preparation/amplification controls.

The high density array may also include sample preparation/amplification control probes. These are probes that are complementary to subsequences of control genes selected because they do not normally occur in the nucleic acids of the particular biological sample being assayed. Suitable sample preparation/amplification control probes include, for example, probes to bacterial genes (*e.g*., Bio B) where the sample in question is a biological from a eukaryote.

The RNA sample is then spiked with a known amount of the nucleic acid to which the sample preparation/amplification control probe is directed before processing. Quantification of the hybridization of the sample preparation/amplification control probe then provides a measure of alteration in the abundance of the nucleic acids caused by processing steps (*e.g.* PCR, reverse transcription, *in vitro* transcription, *etc*.).

### B) Probe Selection and Optimization.

In a preferred embodiment, oligonucleotide probes in the high density array are selected to bind specifically to the nucleic acid target to which they are directed with minimal non-specific binding or cross-hybridization under the particular hybridization conditions utilized. Because the high density arrays of this invention can contain in excess of 1,000,000 different probes, it is possible to provide every probe of a characteristic length that binds to a particular nucleic acid sequence. Thus, for example, the high density array can contain every possible 20 mer sequence complementary to an IL-2 mRNA.

There, however, may exist 20 mer subsequences that are not unique to the IL-2 mRNA. Probes directed to these subsequences are expected to cross hybridize with occurrences of their complementary sequence in other regions of the sample genome. Similarly, other probes simply may not hybridize effectively under the hybridization conditions (*e.g.*, due to secondary structure, or interactions with the substrate or other probes). Thus, in a preferred embodiment, the probes that show such poor specificity or hybridization efficiency are identified and may not be included either in the high density array itself (*e.g.,* during fabrication of the array) or in the post-hybridization data analysis.

In addition, in a preferred embodiment, expression monitoring arrays are used to identify the presence and expression (transcription) level or genes which are several hundred base pairs long. For most applications it would be useful to identify the presence, absence, or expression level of several thousand to one hundred thousand genes. Because the number of oligonucleotides per array is limited in a preferred embodiment, it is desired to include only a limited set of probes specific to each gene whose expression is to be detected.

It is a discovery of this invention that probes as short as 15, 20, or 25 nucleotides are sufficient to hybridize to a subsequence of a gene and that, for most genes, there is a set of probes that performs well across a wide range of target nucleic acid concentrations. In a preferred embodiment, it is desirable to choose a preferred or "optimum" subset of probes for each gene before synthesizing the high density array.

### 1) Hybridization and Cross-Hybridization Data.

Thus, in one embodiment, this invention provides for a method of optimizing a probe set for detection of a particular gene. Generally. this method involves providing a high density array containing a multiplicity of probes of one or more particular length(s) that are complementary to subsequences of the mRNA transcribed by the target gene. In one embodiment the high density array may contain every probe of a particular length that is complementary to a particular mRNA. The probes of the high density array are then hybridized with their target nucleic acid alone and then hybridized with a high complexity, high concentration nucleic acid sample that does not contain the targets complementary to the probes. Thus, for example, where the target nucleic acid is an RNA, the probes are first hybridized with their target nucleic acid alone and then hybridized with RNA made from a cDNA library (*e*.*g*., reverse transcribed polyA⁺ mRNA) where the sense of the hybridized RNA is opposite that of the target nucleic acid (to insure that the high complexity sample does not contain targets for the probes). Those probes that show a strong hybridization signal with their target and little or no cross-hybridization with the high complexity sample are preferred probes for use in the high density arrays of this invention.

The high density array may additionally contain mismatch controls for each of the probes to be tested. In a preferred embodiment, the mismatch controls contain a central mismatch. Where both the mismatch control and the target probe show high levels of hybridization (*e*.*g*., the hybridization to the mismatch is nearly equal to or greater than the hybridization to the corresponding test probe), the test probe is preferably not used in the high density array.

In a particularly preferred embodiment, optimal probes are selected according to the following method: First, as indicated above, an array is provided containing a multiplicity of oligonucleotide probes complementary to subsequences of the target nucleic acid. The oligonucleotide probes may be of a single length or may span a variety of lengths ranging from 5 to 50 nucleotides. The high density array may contain every probe of a particular length that is complementary to a particular mRNA or may contain probes selected from various regions of particular mRNAs. For each target-specific probe the array also contains a mismatch control probe; preferably a central mismatch control probe.

The oligonucleotide array is hybridized to a sample containing target nucleic acids having subsequences complementary to the oligonucleotide probes and the difference in hybridization intensity between each probe and its mismatch control is determined. Only those probes where the difference between the probe and its mismatch control exceeds a threshold hybridization intensity (*e*.*g*. preferably greater than 10% of the background signal intensity, more preferably greater than 20% of the background signal intensity and most preferably greater than 50% of the background signal intensity) are selected. Thus, only probes that show a strong signal compared to their mismatch control are selected.

The probe optimization procedure can optionally include a second round of selection. In this selection, the oligonucleotide probe array is hybridized with a nucleic acid sample that is not expected to contain sequences complementary to the probes. Thus, for example, where the probes are complementary to the RNA sense strand a sample of antisense RNA is provided. Of course, other samples could be provided such as samples from organisms or cell lines known to be lacking a particular gene, or known for not expressing a particular gene.

Only those probes where both the probe and its mismatch control show hybridization intensities below a threshold value (*e*.*g*. less than about 5 times the background signal intensity, preferably equal to or less than about 2 times the background signal intensity, more preferably equal to or less than about 1 times the background signal intensity, and most preferably equal or less than about half background signal intensity) are selected. In this way probes that show minimal non-specific binding are selected. Finally, in a preferred embodiment, the n probes (where n is the number of probes desired for each target gene) that pass both selection criteria and have the highest hybridization intensity for each target gene are selected for incorporation into the array, or where already present in the array, for subsequent data analysis. Of course, one of skill in the art, will appreciate that either selection criterion could be used alone for selection of probes.

### 2) Heuristic rules.

Using the hybridization and cross-hybridization data obtained as described above, graphs can be made of hybridization and cross-hybridization intensities versus various probe properties *e.g*., number of As, number of Cs in a window of 8 bases, palindomic strength, *etc.* The graphs can then be examined for correlations between those properties and the hybridization or cross-hybridization intensities. Thresholds can be set beyond which it looks like hybridization is always poor or cross hybridization is always very strong. If any probe fails one of the criteria, it is rejected from the set of probes and therefore, not placed on the chip. This will be called the heuristic rules method.

One set of rules developed for 20 mer probes in this manner is the following:
Hybridization rules:
   1) Number of As is less than 9.
   2) Number of Ts is less than 10 and greater than 0.
   3) Maximum run of As, Gs, or Ts is less than 4 bases in a row.
   4) Maximum run of any 2 bases is less than 11 bases.
   5) Palindrome score is less than 6.
   6) Clumping score is less than 6.
   7) Number of As + Number of Ts is less than 14
   8) Number of As + number of Gs is less than 15
With respect to rule number 4, requiring the maximum run of any two bases to be less than 11 bases guarantees that at least three different bases occur within any 12 consecutive nucleotides. A palindrome score is the maximum number of complementary bases if the oligonucleotide is folded over at a point that maximizes self complementarity. Thus, for example a 20 mer that is perfectly self-complementary would have a palindrome score of 10. A clumping score is the maximum number of three-mers of identical bases in a given sequence. Thus, for example, a run of 5 identical bases will produce a clumping score of 3 (bases 1-3. bases 2-4, and bases 3-5).

If any probe failed one of these criteria (1-8). the probe was not a member of the subset of probes placed on the chip. For example, if a hypothetical probe was 5'-AGCTTTTTTCATGCATCTAT-3' the probe would not be synthesized on the chip because it has a run of four or more bases (*i*.*e*., run of six).

The cross hybridization rules developed for 20 mers were as follows:
1) Number of Cs is less than 8;
2) Number of Cs in any window of 8 bases is less than 4.

Thus, if any probe failed any of either the hybridization ruses (1-8) or the cross-hybridization rules (1-2), the probe was not a member of the subset of probes placed on the chip. These rules eliminated many of the probes that cross hybridized strongly or exhibited low hybridization, and performed moderate job of eliminating weakly hybridizing probes.

These heuristic rules may be implemented by hand calculations, or alternatively, they may be implemented in software as is discussed below in Section IV.B.7.

### 3) Neural net.

In another embodiment, a neural net can be trained to predict the hybridization and cross-hybridization intensities based on the sequence of the probe or on other probe properties. The neural net can then be used to pick an arbitrary number of the "best" probes. One such neural net was developed for selecting 20-mer probes. This neural net was produced a moderate (0 7) correlation between predicted intensity and measured intensity, with a better model for cross hybridization than hybridization. Details of this neural net are provided in Example 6.

### 4) ANOVA Model

An analysis of variance (ANOVA) model may be built to model the intensities based on positions of consecutive base pairs. This is based on the theory that the melting energy is based on stacking energies of consecutive bases. The annova model was used to find correlation between the a probe sequence and the hybridization and cross-hybridization intensities. The inputs were probe sequences broken down into consecutive base pairs. One model was made to predict hybridization, another was made to predict cross hybridization. The output was the hybridization or crosshybridization intensity.

There were 304 (19 * 16) possible inputs, consisting of the 14 possible two base combinations, and the 19 positions that those combinations could be found in. For example, the sequence aggctga... has "ag" in the first position, "gg" in the second position, "gc" in the third, "ct" in the fourth and so on.

The resulting model assigned a component of the output intensity to each of the possible inputs, so to estimate the intensity for a given sequence one simply adds the intensities for each of it's 19 components.

### 5) Pruning (removal) of similar probes.

One of the causes of poor signals in expression chips is that genes other than the ones being monitored have sequences which are very similar to parts of the sequences which are being monitored. The easiest way to solve this is to remove probes which are similar to more than one gene. Thus, in a preferred embodiment, it is desirable to remove (prune) probes that hybridize to transcription products of more than one gene.

The simplest pruning method is to line up a proposed probe with all known genes for the organism being monitored, then count the number of matching bases. For example, given a probe to gene 1 of an organism and gene 2 of an organism as follows 
probe from gene 1 aagcgcgatcgattatgctc
| |||||||
gene 2: atctcggatcgatcggataagcgcgatcgattatgctcggcga
 has 8 matching bases in this alignment, but 20 matching bases in the following alignment: 
probe from gene 1 aagcgcgatcgattatgctc
||||||||||||||||||||
gene 2 atctcggatcgatcggataagcgcgatcgattatgctcggcga
 More complicated algorithms also exist, which allow the detection of insertion or deletion mismatches. Such sequence alignment algorithms are well known to those of skill in the art and include, but are not limited to BLAST, or FASTA, or other gene matching programs such as those described above in the definitions section.

In another variant, where an organism has many different genes which are very similar, it is difficult to make a probe set that measures the concentration only one of those very similar genes. One can then prune out any probes which are dissimilar, and make the probe set a probe set for that family of genes.

### 6) Synthesis cycle pruning.

The cost of producing masks for a chip is approximately linearly related to the number of synthesis cycles. In a normal set of genes the distribution of the number of cycles any probe takes to build approximates a Gausian distribution. Because of this the mask cost can normally be reduced by 15% by throwing out about 3 percent of the probes In a preferred embodiment, synthesis cycle pruning simply involves eliminating (not including) those probes those probes that require a greater number of synthesis cycles than the maximum number of synthesis cycles selected for preparation of the particular subject high density oligonucleotide array. Since the typical synthesis of probes follows a regular pattern of bases put down (acgtacgtacgt ) counting the number of synthesis steps needed to build a probe is easy. The listing shown in Table 1 povides typical code for counting the number of synthesis cycles a probe will need.

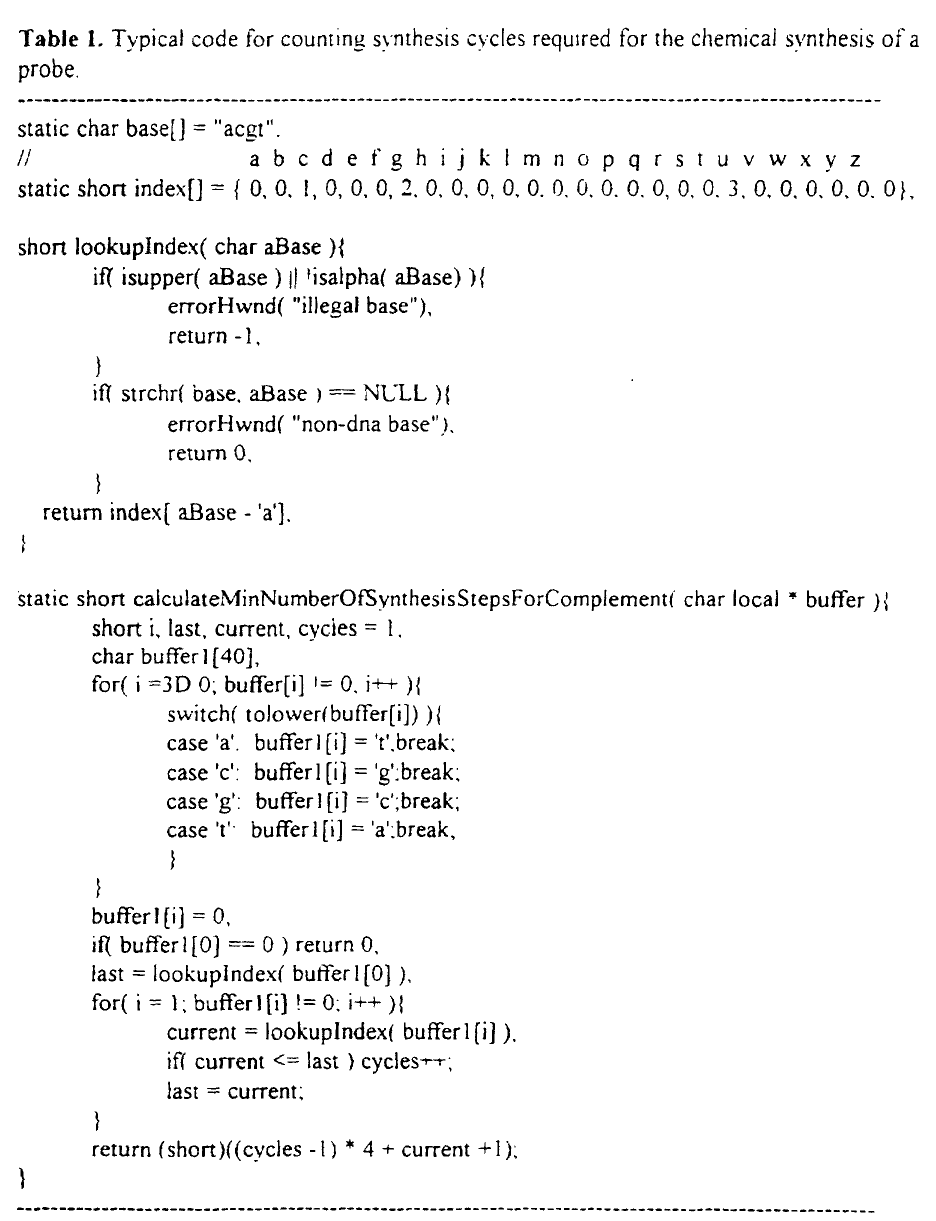

### 7) Combination of Selection methods.

The heuristic rules, neural net and annova model provide ways of pruning or reducing the number of probes for monitoring the expression of genes As these methods do not necessarily produce the same results, or produce entirely independent results. it may be advantageous to combine the methods For example, probes may be pruned or reduced if more than one method (*e.g*., two out of three) indicate the probe will not likely produce good results. Then. synthesis cycle pruning may be performed to reduce costs.

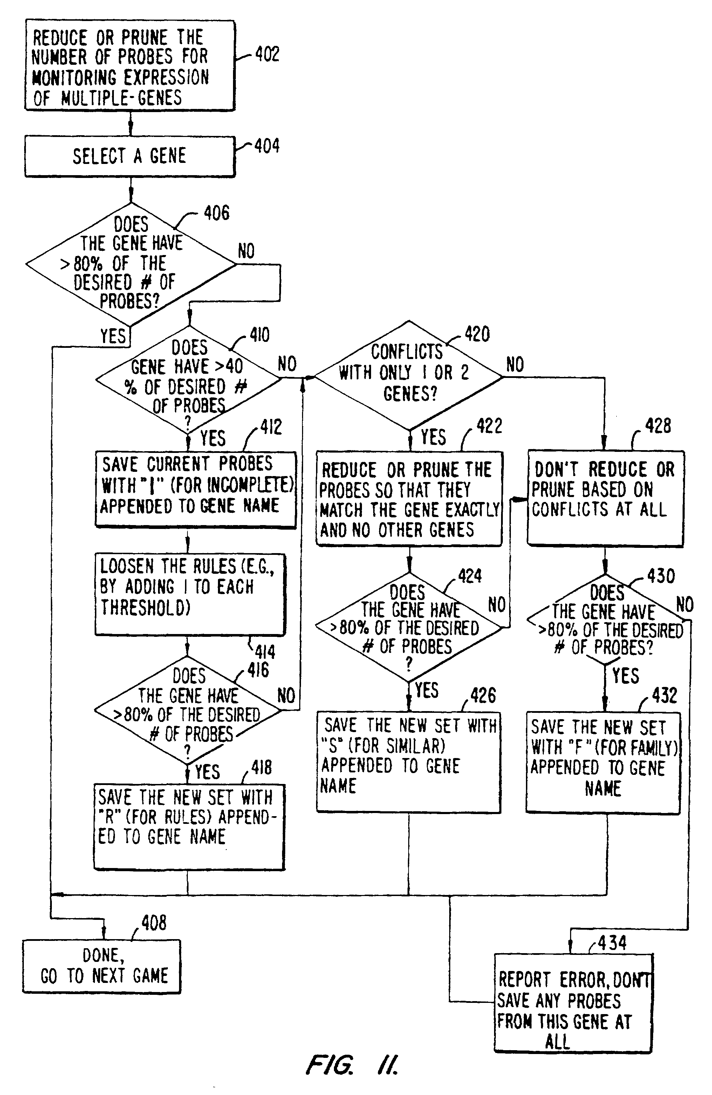

Fig. 11 shows the flow of a process of increasing the number of probes for monitoring the expression of genes after the number of probes has been reduced or pruned In one embodiment, a user is able to specify the number of nucleic acid probes that should be placed on the chip to monitor the expression of each gene. As discussed above, it is advantageous to reduce probes that will not likely produce good results; however, the number of probes may be reduced to substantially less than the desired number of probes.

At step 402, the number of probes for monitoring multiple genes is reduced by the heuristic rules method, neural net, annova model, synthesis cycle pruning, or any other method, or combination of methods. A gene is selected at step 404.

A determination is made whether the remaining probes for monitoring the selected gene number greater than 80% (which may be varied or user defined) of the desired number of probes. If yes, the computer system proceeds to the next gene at step 408 which will generally return to step 404.

If the remaining probes for monitoring the selected gene do not number greater than 80% of the desired number of probes, a determination is made whether the remaining probes for monitoring the selected gene number greater than 40% (which may be varied or user defined) of the desired number of probes. If yes, an "i" is appended to the end of the gene name to indicate that after pruning, the probes were incomplete at step 412.

At step 414, the number of probes is increased by loosening the constraints that rejected probes. For example, the thresholds in the heuristic rules may be increased by 1. Therefore, if previously probes were rejected if they had four As in a row, the rule may be loosened to five As in a row.

A determination is then made whether the remaining probes for monitoring the selected gene number greater than 80% of the desired number of probes at step 416. If yes, an "r" is appended to the end of the gene name at step 412 to indicate that the rules were loosened to generate the number of synthesized probes for that gene.

At step 420, a check is made to see if the probes for monitonng the selected gene only conflict with one or two other genes. If yes, the full set of probes complementary to the gene (or target sequence) are taken and pruned so that the probes remaining are exactly complementary to the selected gene exclusively at step 422.

A determination is then made whether the remaining probes for monitoring the selected gene number greater than 80% of the desired number of probes at step 424. If yes, an "s" is appended to the end of the gene name at step 426 to indicate that the only a few genes were similar to the selected gene.

At step 428, the probes for monitoring the selected gene are not reduced by conflicts at all. A determination is then made whether the remaining probes for monitoring the selected gene number greater than 80% of the desired number of probes at step 430. If yes, an "f" is appended to the end of the gene name at step 432 to indicate that the probes include the whole family of probes perfectly complementary to the gene.

If there are still not 80% of the desired number of probes, an error is reported at step 434. Any number of error handling procedures may be undertaken. For example, an error message may be generated for the user and the probes for the gene may not be stored. Alternatively, the user may be prompted to enter a new desired number of probes.

### V. Synthesis of High Density Arrays

Methods of forming high density arrays of oligonucleotides, peptides and other polymer sequences with a minimal number of synthetic steps are known. The oligonucleotide analogue array can be synthesized on a solid substrate by a variety of methods, including, but not limited to, light-directed chemical coupling, and mechanically directed coupling. See Pirrung *et al*., U.S. Patent No. 5,143,854 (see also PCT Application No. WO 90/15070) and Fodor *et al*., PCT Publication Nos. WO 92/10092 and WO 93/09668 which disclose methods of forming vast arrays of peptides, oligonucleotides and other molecules using, for example, light-directed synthesis techniques. See also, Fodor *et al*., *Science*, 251, 767-77 (1991). These procedures for synthesis of polymer arrays are now referred to as VLSIPS™ procedures. Using the VLSIPS™ approach, one heterogenous array of polymers is converted, through simultaneous coupling at a number of reaction sites, into a different heterogenous array. See, U.S. Application Serial Nos. 07/796,243 and 07/980,523.

The development of VLSIPS™ technology as described in the above-noted U.S. Patent No. 5,143,854 and PCT patent publication Nos. WO 90/15070 and 92/10092, is considered pioneering technology in the fields of combinatorial synthesis and screening of combinatorial libraries. More recently, patent application Serial No. 08/082,937, filed June 25, 1993 describes methods for making arrays of oligonucleotide probes that can be used to check or determine a partial or complete sequence of a target nucleic acid and to detect the presence of a nucleic acid containing a specific oligonucleotide sequence.

In brief, the light-directed combinatorial synthesis of oligonucleotide arrays on a glass surface proceeds using automated phosphoramidite chemistry and chip masking techniques. In one specific implementation, a glass surface is derivatized with a silane reagent containing a functional group, *e.g.*, a hydroxyl or amine group blocked by a photolabile protecting group. Photolysis through a photolithogaphic mask is used selectively to expose functional groups which are then ready to react with incoming 5'-photoprotected nucleoside phosphoramidites. The phosphoramidites react only with those sites which are illuminated (and thus exposed by removal of the photolabile blocking group). Thus, the phosphoramidites only add to those areas selectively exposed from the preceding step. These steps are repeated until the desired array of sequences have been synthesized on the solid surface. Combinatorial synthesis of different oligonucleotide analogues at different locations on the array is determined by the pattern of illumination during synthesis and the order of addition of coupling reagents.

In the event that an oligonucleotide analogue with a polyamide backbone is used in the VLSIPS™ procedure, it is generally inappropriate to use phosphoramidite chemistry to perform the synthetic steps, since the monomers do not attach to one another via a phosphate linkage. Instead, peptide synthetic methods are substituted. See, *e*.*g*., Pirrung *et al.* U.S. Pat. No. 5,143,854.

Peptide nucleic acids are commercially available from, *e*.*g*., Biosearch, Inc. (Bedford, MA) which comprise a polyamide backbone and the bases found in naturally occurring nucleosides. Peptide nucleic acids are capable of binding to nucleic acids with high specificity, and are considered "oligonucleotide analogues" for purposes of this disclosure.

In addition to the foregoing, additional methods which can be used to generate an array of oligonucleotides on a single substrate are described in co-pending Applications Ser. No. 07/980,523, filed November 20. 1992, and 07/796,243, filed November 22, 1991 and in PCT Publication No. WO 93/09668. In the methods disclosed in these applications, reagents are delivered to the substrate by either (1) flowing within a channel defined on predefined regions or (2) "spotting" on predefined regions. However, other approaches, as well as combinations of spotting and flowing, may be employed. In each instance, certain activated regions of the substrate are mechanically separated from other regions when the monomer solutions are delivered to the various reaction sites.

A typical "flow channel" method applied to the compounds and libraries of the present invention can generally be described as follows. Diverse polymer sequences are synthesized at selected regions of a substrate or solid support by forming flow channels on a surface of the substrate through which appropriate reagents flow or in which appropriate reagents are placed. For example, assume a monomer "A" is to be bound to the substrate in a first group of selected regions. If necessary, all or part of the surface of the substrate in all or a part of the selected regions is activated for binding by, for example, flowing appropriate reagents through all or some of the channels, or by washing the entire substrate with appropriate reagents. After placement of a channel block on the surface of the substrate, a reagent having the monomer A flows through or is placed in all or some of the channel(s). The channels provide fluid contact to the first selected regions, thereby binding the monomer A on the substrate directly or indirectly (via a spacer) in the first selected regions.

Thereafter, a monomer B is coupled to second selected regions, some of which may be included among the first selected regions. The second selected regions will be in fluid contact with a second flow channel(s) through translation, rotation, or replacement of the channel block on the surface of the substrate: through opening or closing a selected valve; or through deposition of a layer of chemical or photoresist. If necessary, a step is performed for activating at least the second regions. Thereafter, the monomer B is flowed through or placed in the second flow channel(s), binding monomer B at the second selected locations. In this particular example, the resulting sequences bound to the substrate at this stage of processing will be, for example, A, B, and AB. The process is repeated to form a vast array of sequences of desired length at known locations on the substrate.

After the substrate is activated, monomer A can be flowed through some of the channels, monomer B can be flowed through other channels, a monomer C can be flowed through still other channels, *etc.* In this manner, many or all of the reaction regions are reacted with a monomer before the channel block must be moved or the substrate must be washed and/or reactivated. By making use of many or all of the available reaction regions simultaneously, the number of washing and activation steps can be minimized.

One of skill in the art will recognize that there are alternative methods of forming channels or otherwise protecting a portion of the surface of the substrate. For example, according to some embodiments, a protective coating such as a hydrophilic or hydrophobic coating (depending upon the nature of the solvent) is utilized over portions of the substrate to be protected, sometimes in combination with materials that facilitate wetting by the reactant solution in other regions. In this manner, the flowing solutions are further prevented from passing outside of their designated flow paths.

The "spotting" methods of preparing compounds and libraries of the present invention can be implemented in much the same manner as the flow channel methods. For example, a monomer A can be delivered to and coupled with a first group of reaction regions which have been appropriately activated. Thereafter, a monomer B can be delivered to and reacted with a second group of activated reaction regions. Unlike the flow channel embodiments described above, reactants are delivered by directly depositing (rather than flowing) relatively small quantities of them in selected regions. In some steps, of course, the entire substrate surface can be sprayed or otherwise coated with a solution. In preferred embodiments, a dispenser moves from region to region, depositing only as much monomer as necessary at each stop. Typical dispensers include a micropipette to deliver the monomer solution to the substrate and a robotic system to control the position of the micropipette with respect to the substrate. In other embodiments, the dispenser includes a series of tubes. a manifold, an array of pipettes, or the like so that various reagents can be delivered to the reaction regions simultaneously.

### VI. Hybridization.

Nucleic acid hybridization simply involves providing a denatured probe and target nucleic acid under conditions where the probe and its complementary target can form stable hybrid duplexes through complementary base pairing. The nucleic acids that do not form hybrid duplexes are then washed away leaving the hybridized nucleic acids to be detected, typically through detection of an attached detectable label. It is generally recognized that nucleic acids are denatured by increasing the temperature or decreasing the salt concentration of the buffer containing the nucleic acids. Under low stringency conditions (*e.g*., low temperature and/or high salt) hybrid duplexes (*e.g*., DNA:DNA, RNA:RNA, or RNA:DNA) will form even where the annealed sequences are not perfectly complementary. Thus specificity of hybridization is reduced at lower stringency. Conversely, at higher stringency (*e.g.*, higher temperature or lower salt) successful hybridization requires fewer mismatches.

One of skill in the art will appreciate that hybridization conditions may be selected to provide any degree of stringency. In a preferred embodiment, hybridization is performed at low stringency in this case in 6X SSPE-T at 37°C (0.005% Triton X-100) to ensure hybridization and then subsequent washes are performed at higher stringency (*e.g*., 1 X SSPE-T at 37°C) to eliminate mismatched hybrid duplexes. Successive washes may be performed at increasingly higher stringency (*e.g*., down to as low as 0.25 X SSPE-T at 37°C to 50°C) until a desired level of hybridization specificity is obtained. Stringency can also be increased by addition of agents such as formamide. Hybridization specificity may be evaluated by comparison of hybridization to the test probes with hybridization to the various controls that can be present (*e.g*., expression level control, normalization control, mismatch controls, *etc*.).

In general, there is a tradeoff between hybridization specificity (stringency) and signal intensity. Thus, in a preferred embodiment, the wash is performed at the highest stringency that produces consistent results and that provides a signal intensity greater than approximately 10% of the background intensity. Thus. in a preferred embodiment, the hybridized array may be washed at successively higher stringency solutions and read between each wash. Analysis of the data sets thus produced will reveal a wash stringency above which the hybridization pattern is not appreciably altered and which provides adequate signal for the particular oligonucleotide probes of interest.

In a preferred embodiment, background signal is reduced by the use of a detergent (*e.g.,* C-TAB) or a blocking reagent (*e*.*g*., sperm DNA, cot-1 DNA, etc.) during the hybridization to reduce non-specific binding. In a particularly preferred embodiment, the hybridization is performed in the presence of about 0.5 mg/ml DNA (*e.g.*, herring sperm DNA). The use of blocking agents in hybridization is well known to those of skill in the art (*see*, *e*.*g*., Chapter 8 in P. Tijssen, *supra*.)

The stability of duplexes formed between RNAs or DNAs are generally in the order of RNA:RNA > RNA:DNA > DNA:DNA, in solution. Long probes have better duplex stability with a target, but poorer mismatch discrimination than shorter probes (mismatch discrimination refers to the measured hybridization signal ratio between a perfect match probe and a single base mismatch probe). Shorter probes (*e.g.*, 8-mers) discriminate mismatches very well, but the overall duplex stability is low.

Altering the thermal stability (Tₘ) of the duplex formed between the target and the probe using, *e.g.*, known oligonucleotide analogues allows for optimization of duplex stability and mismatch discrimination. One useful aspect of altering the Tₘ arises from the fact that adenine-thymine (A-T) duplexes have a lower Tₘ than guanine-cytosine (G-C) duplexes, due in part to the fact that the A-T duplexes have 2 hydrogen bonds per base-pair, while the G-C duplexes have 3 hydrogen bonds per base pair. In heterogeneous oligonucleotide arrays in which there is a non-uniform distribution of bases, it is not generally possible to optimize hybridization for each oligonucleotide probe simultaneously. Thus, in some embodiments, it is desirable to selectively destabilize G-C duplexes and/or to increase the stability of A-T duplexes. This can be accomplished, *e.g.*, by substituting guanine residues in the probes of an array which form G-C duplexes with hypoxanthine, or by substituting adenine residues in probes which form A-T duplexes with 2,6 diaminopurine or by using the salt tetramethyl ammonium chloride (TMACl) in place of NaCl.

Altered duplex stability conferred by using oligonucleotide analogue probes can be ascertained by following, *e.g*., fluorescence signal intensity of oligonucleotide analogue arrays hybridized with a target oligonucleotide over time. The data allow optimization of specific hybridization conditions at, *e.g*., room temperature (for simplified diagnostic applications in the future).

Another way of verifying altered duplex stability is by following the signal intensity generated upon hybridization with time. Previous experiments using DNA targets and DNA chips have shown that signal intensity increases with time. and that the more stable duplexes generate higher signal intensities faster than less stable duplexes. The signals reach a plateau or "saturate" after a certain amount of time due to all of the binding sites becoming occupied. These data allow for optimization of hybridization, and determination of the best conditions at a specified temperature.

Methods of optimizing hybridization conditions are well known to those of skill in the art (*see, e.g., Laboratory Techniques in Biochemistry and Molecular Biology, Vol. 24: Hybridization With Nucleic Acid Probes.* P. Tijssen, ed. Elsevier, N.Y., (1993)).

### VII. Signal Detection.

Means of detecting labeled target (sample) nucleic acids hybridized to the probes of the high density array are known to those of skill in the art. Thus, for example, where a colorimetric label is used, simple visualization of the label is sufficient. Where a radioactive labeled probe is used, detection of the radiation (*e.g* with photographic film or a solid state detector) is sufficient.

In a preferred embodiment, however, the target nucleic acids are labeled with a fluorescent label and the localization of the label on the probe array is accomplished with fluorescent microscopy. The hybridized array is excited with a light source at the excitation wavelength of the particular fluorescent label and the resulting fluorescence at the emission wavelength is detected. In a particularly preferred embodiment, the excitation light source is a laser appropriate for the excitation of the fluorescent label.

The confocal microscope may be automated with a computer-controlled stage to automatically scan the entire high density array. Similarly, the microscope may be equipped with a phototransducer (*e.g*., a photomultiplier, a solid state array, a ccd camera, *etc.*) attached to an automated data acquisition system to automatically record the fluorescence signal produced by hybridization to each oligonucleotide probe on the array. Such automated systems are described at length in U.S. Patent No: 5,143,854, PCT Application 20 92/10092, and copending U.S.S.N. 08/195,889 filed on February 10, 1994. Use of laser illumination in conjunction with automated confocal microscopy for signal detection permits detection at a resolution of better than about 100 µm, more preferably better than about 50 µm. and most preferably better than about 25 µm.

### VIII. Signal Evaluation.

One of skill in the art will appreciate that methods for evaluating the hybridization results vary with the nature of the specific probe nucleic acids used as well as the controls provided. In the simplest embodiment, simple quantification of the fluorescence intensity for each probe is determined. This is accomplished simply by measuring probe signal strength at each location (representing a different probe) on the high density array (*e.g*., where the label is a fluorescent label. detection of the amount of florescence (intensity) produced by a fixed excitation illumination at each location on the array). Comparison of the absolute intensities of an array hybridized to nucleic acids from a "test" sample with intensities produced by a "control" sample provides a measure of the relative expression of the nucleic acids that hybridize to each of the probes.

One of skill in the art, however, will appreciate that hybridization signals will vary in strength with efficiency of hybridization, the amount of label on the sample nucleic acid and the amount of the particular nucleic acid in the sample. Typically nucleic acids present at very low levels (*e.g*., < 1pM) will show a very weak signal. At some low level of concentration, the signal becomes virtually indistinguishable from background. In evaluating the hybridization data, a threshold intensity value may be selected below which a signal is not counted as being essentially indistinguishable from background.

Where it is desirable to detect nucleic acids expressed at lower levels, a lower threshold is chosen. Conversely, where only high expression levels are to be evaluated a higher threshold level is selected. In a preferred embodiment, a suitable threshold is about 10% above that of the average background signal.

In addition, the provision of appropriate controls permits a more detailed analysis that controls for variations in hybridization conditions, cell health, non-specific binding and the like. Thus, for example, in a preferred embodiment, the hybridization array is provided with normalization controls as described above in Section IV.A.2. These normalization controls are probes complementary to control sequences added in a known concentration to the sample. Where the overall hybridization conditions are poor, the normalization controls will show a smaller signal reflecting reduced hybridization. Conversely, where hybridization conditions are good, the normalization controls will provide a higher signal reflecting the improved hybridization. Normalization of the signal derived from other probes in the array to the normalization controls thus provides a control for variations in hybridization conditions. Typically, normalization is accomplished by dividing the measured signal from the other probes in the array by the average signal produced by the normalization controls. Normalization may also include correction for variations due to sample preparation and amplification. Such normalization may be accomplished by dividing the measured signal by the average signal from the sample preparation/amplfication control probes (*e.g*., the Bio B probes). The resulting values may be multiplied by a constant value to scale the results.

As indicated above, the high density array can include mismatch controls. In a preferred embodiment, there is a mismatch control having a central mismatch for every probe (except the normalization controls) in the array. It is expected that after washing in stringent conditions, where a perfect match would be expected to hybridize to the probe, but not to the mismatch, the signal from the mismatch controls should only reflect non-specific binding or the presence in the sample of a nucleic acid that hybridizes with the mismatch. Where both the probe in question and its corresponding mismatch control both show high signals, or the mismatch shows a higher signal than its corresponding test probe, there is a problem with the hybridization and the signal from those probes is ignored. The difference in hybridization signal intensity between the target specific probe and its corresponding mismatch control is a measure of the discrimination of the target-specific probe. Thus, in a preferred embodiment, the signal of the mismatch probe is subtracted from the signal from its corresponding test probe to provide a measure of the signal due to specific binding of the test probe.

The concentration of a particular sequence can then be determined by measuring the signal intensity of each of the probes that bind specifically to that gene and normalizing to the normalization controls. Where the signal from the probes is greater than the mismatch, the mismatch is subtracted. Where the mismatch intensity is equal to or greater than its corresponding test probe, the signal is ignored. The expression level of a particular gene can then be scored by the number of positive signals (either absolute or above a threshold value), the intensity of the positive signals (either absolute or above a selected threshold value), or a combination of both metrics (*e.g*., a weighted average).

It is a surprising discovery of this invention, that normalization controls are often unnecessary for useful quantification of a hybridization signal. Thus, where optimal probes have been identified in the two step selection process as described above, in Section II.B., the average hybridization signal produced by the selected optimal probes provides a good quantified measure of the concentration of hybridized nucleic acid.

### IX. Computer-implemented Expression Monitoring

The methods of monitoring gene expression of this invention may be performed utilizing a computer. The computer typically runs a software program that includes computer code incorporating the invention for analyzing hybridization intensities measured from a substrate or chip and thus, monitoring the expression of one or more genes. Although the following will describe specific embodiments of the invention, the invention is not limited to any one embodiment so the following is for purposes of illustration and not limitation.

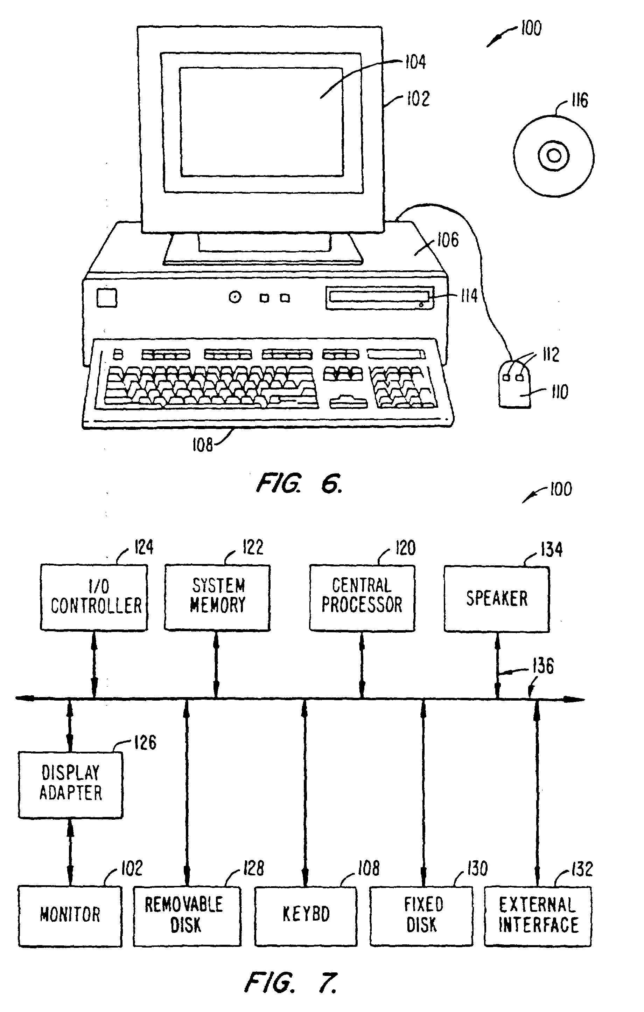

Fig. 6 illustrates an example of a computer system used to execute the software of an embodiment of the present invention. As shown, shows a computer system 100 includes a monitor 102, screen 104, cabinet 106, keyboard 108, and mouse 110. Mouse 110 may have one or more buttons such as mouse buttons 112. Cabinet 106 houses a CD-ROM drive 114, a system memory and a hard drive (both shown in Fig. 7) which may be utilized to store and retrieve software programs incorporating computer code that implements the invention, data for use with the invention, and the like. Although a CD-ROM 116 is shown as an exemplary computer readable storage medium, other computer readable storage media including floppy disks, tape, flash memory, system memory, and hard drives may be utilized. Cabinet 106 also houses familiar computer components (not shown) such as a central processor, system memory. hard disk, and the like.

Fig. 7 shows a system block diagram of computer system 100 used to execute the software of an embodiment of the present invention. As in Fig. 6, computer system 100 includes monitor 102 and keyboard 108. Computer system 100 further includes subsystems such as a central processor 120, system memory 122, I/O controller 124, display adapter 126, removable disk 128 (*e.g*., CD-ROM drive), fixed disk 130 (*e.g*., hard drive), network interface 132, and speaker 134. Other computer systems suitable for use with the present invention may include additional or fewer subsystems. For example, another computer system could include more than one processor 120 (*i.e.,* a multi-processor system) or a cache memory.

Arrows such as 136 represent the system bus architecture of computer system 100. However, these arrows are illustrative of any interconnection scheme serving to link the subsystems. For example, a local bus could be utilized to connect the central processor to the system memory and display adapter. Computer system 100 shown in Fig. 7 is but an example of a computer system suitable for use with the present invention. Other configurations of subsystems suitable for use with the present invention will be readily apparent to one of ordinary skill in the art.

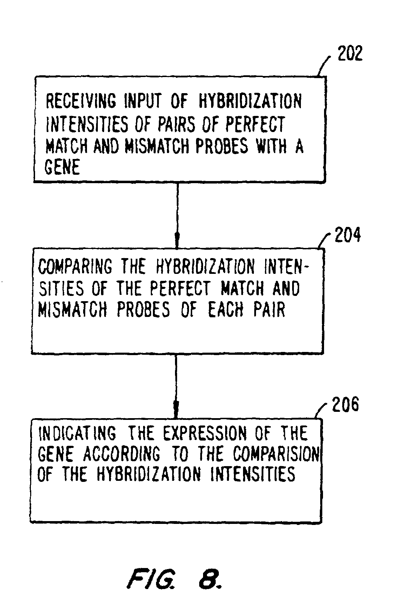

Fig. 8 shows a flowchart of a process of momtonng the expression of a gene. The process compares hybridization intensities of pairs of perfect match and mismatch probes that are preferably covalently attached to the surface of a substrate or chip. Most preferably, the nucleic acid probes have a density greater than about 60 different nucleic acid probes per 1 cm² of the substrate. Although the flowcharts show a sequence of steps for clarity, this is not an indication that the steps must be performed in this specific order. One of ordinary skill in the art would readily recognize that many of the steps may be reordered, combined, and deleted without departing from the invention.

Initially, nucleic acid probes are selected that are complementary to the target sequence (or gene). These probes are the perfect match probes. Another set of probes is specified that are intended to be not perfectly complementary to the target sequence. These probes are the mismatch probes and each mismatch probe includes at least one nucleotide mismatch from a perfect match probe. Accordingly, a mismatch probe and the perfect match probe from which it was derived make up a pair of probes. As mentioned earlier, the nucleotide mismatch is preferably near the center of the mismatch probe.

The probe lengths of the perfect match probes are typically chosen to exhibit high hybridization affinity with the target sequence. For example, the nucleic acid probes may be all 20-mers. However, probes of varying lengths may also be synthesized on the substrate for any number of reasons including resolving ambiguities.

The target sequence is typically fragmented, labeled and exposed to a substrate including the nucleic acid probes as described earlier. The hybridization intensities of the nucleic acid probes is then measured and input into a computer system. The computer system may be the same system that directs the substrate hybridization or it may be a different system altogether. Of course, any computer system for use with the invention should have available other details of the experiment including possibly the gene name, gene sequence, probe sequences, probe locations on the substrate, and the like.

Referring to Fig. 8, after hybridization, the computer system receives input of hybridization intensities of the multiple pairs of perfect match and mismatch probes at step 202. The hybridization intensities indicate hybridization affinity between the nucleic acid probes and the target nucleic acid (which corresponds to a gene). Each pair includes a perfect match probe that is perfectly complementary to a portion of the target nucleic acid and a mismatch probe that differs from the perfect match probe by at least one nucleotide.

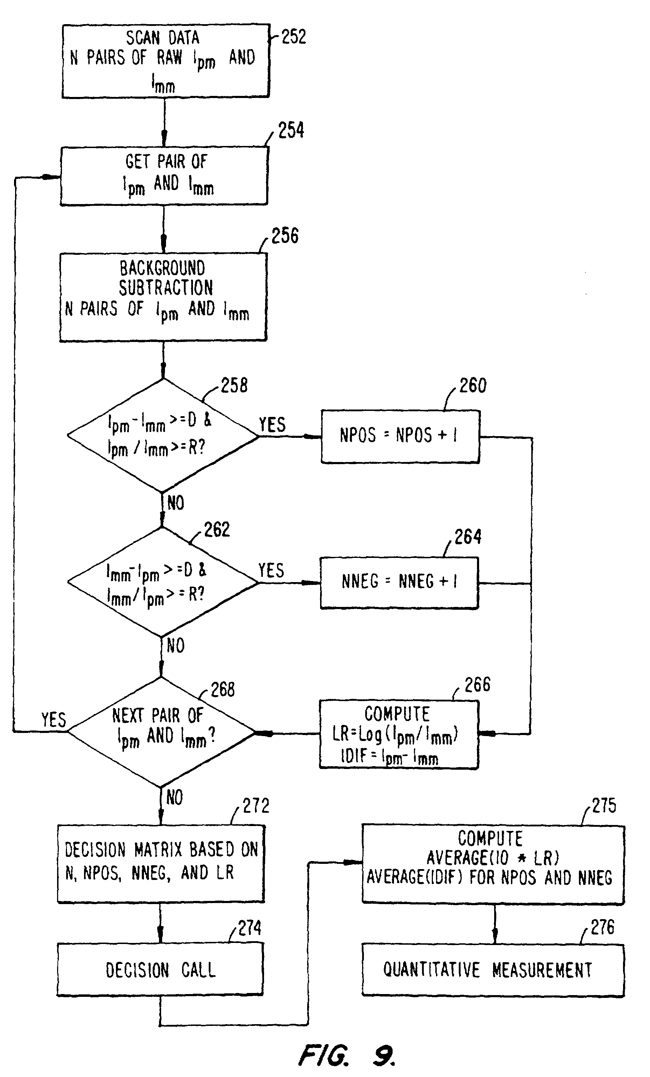

At step 204. the computer system compares the hybridization intensities of the perfect match and mismatch probes of each pair. If the gene is expressed, the hybridization intensity (or affinity) of a perfect match probe of a pair should be recognizably higher than the corresponding mismatch probe. Generally, if the hybridizations intensities of a pair of probes are substantially the same, it may indicate the gene is not expressed. However, the determination is not based on a single pair of probes, the determination of whether a gene is expressed is based on an analysis of many pairs of probes. An exemplary process of comparing the hybridization intensities of the pairs of probes will be described in more detail in reference to Fig. 9.

After the system compares the hybridization intensity of the perfect match and mismatch probes, the system indicates expression of the gene at step 206. As an example, the system may indicate to a user that the gene is either present (expressed), marginal or absent (unexpressed).

Fig. 9 shows a flowchart of a process of determining if a gene is expressed utilizing a decision matrix. At step 252, the computer system receives raw scan data of N pairs of perfect match and mismatch probes. In a preferred embodiment, the hybridization intensities are photon counts from a fluorescein labeled target that has hybridized to the probes on the substrate. For simplicity, the hybridization intensity of a perfect match probe will be designed "Iₚₘ" and the hybridization intensity of a mismatch probe will be designed "Iₘₘ."

Hybridization intensities for a pair of probes is retrieved at step 254. The background signal intensity is subtracted from each of the hybridization intensities of the pair at step 256. Background subtraction may also be performed on all the raw scan data at the same time.

At step 258, the hybridization intensities of the pair of probes are compared to a difference threshold (D) and a ratio threshold (R). It is determined if the difference between the hybridization intensities of the pair (Iₚₘ - Iₘₘ) is greater than or equal to the difference threshold AND the quotient of the hybridization intensities of the pair (Iₚₘ / Iₘₘ) is greater than or equal to the ratio threshold. The difference thresholds are typically user defined values that have been determined to produce accurate expression monitoring of a gene or genes. In one embodiment, the difference threshold is 20 and the ratio threshold is 1.2.

If Iₚₘ - Iₘₘ > = D and Iₚₘ / Iₘₘ > = R, the value NPOS is incremented at step 260. In general, NPOS is a value that indicates the number of pairs of probes which have hybridization intensities indicating that the gene is likely expressed. NPOS is utilized in a determination of the expression of the gene.

At step 262, it is determined if Iₘₘ - Iₚₘ > = D and Iₘₘ / Iₚₘ > = R. If this expression is true, the value NNEG is incremented at step 264. In general, NNEG is a value that indicates the number of pairs of probes which have hybridization intensities indicating that the gene is likely not expressed. NNEG, like NPOS, is utilized in a determination of the expression of the gene.

For each pair that exhibits hybridization intensities either indicating the gene is expressed or not expressed, a log ratio value (LR) and intensity difference value (IDIF) are calculated at step 266. LR is calculated by the log of the quotient of the hybridization intensities of the pair (Iₚₘ / Iₘₘ). The IDIF is calculated by the difference between the hybridization intensities of the pair (Iₚₘ - Iₘₘ). If there is a next pair of hybridization intensities at step 268, they are retrieved at step 254.

At step 272, a decision matrix is utilized to indicate if the gene is expressed. The decision matrix utilizes the values N, NPOS, NNEG, and LR (multiple LRs). The following four assignments are performed:
P1 = NPOS / NNEG
P2 = NPOS / N
P3 = (10 * SUM(LR)) / (NPOS + NNEG)
These P values are then utilized to determine if the gene is expressed.

For purposes of illustration, the P values are broken down into ranges. If P1 is greater than or equal to 2.1, then A is true. If P1 is less than 2.1 and greater than or equal to 1.8, then B is true. Otherwise, C is true. Thus, P1 is broken down into three ranges A, B and C. This is done to aid the readers understanding of the invention.

Thus, all of the P values are broken down into ranges according to the following:
A = (P1 > = 2.1)
B = (2.1 > P1 > = 1.8)
C = (P1 < 1.8)

X = (P2 > = 0.35)
Y = (0.35 > P2 > = 0.20)
Z = (P2 < 0.20)

Q = (P3 > = 1.5)
R = (1.5 > P3 > = 1.1)
S = (P3 < 1.1)
Once the P values are broken down into ranges according to the above boolean values. the gene expression is determined.

The gene expression is indicated as present (expressed). marginal or absent (not expressed). The gene is indicated as expressed if the following expression is true: A and (X or Y) and (Q or R). In other words, the gene is indicated as expressed if P1 > = 2.1, P2 > = 0.20 and P3 > = 1.1. Additionally, the gene is indicated as expressed if the following expression is true: B and X and Q.

With the forgoing explanation, the following is a summary of the gene expression indications:
- Present: A and (X or Y) and (Q or R)
B and X and I
- Marginal: A and X and S
B and X and R
B and Y and (Q or R)
- Absent: All others cases (*e.g*., any C combination)

In the output to the user, present may be indicated as "P," marginal as "M" and absent as "A" at step 274.

Once all the pairs of probes have been processed and the expression of the gene indicated, an average of ten times the LRs is computed at step 275. Additionally, an average of the IDIF values for the probes that incremented NPOS and NNEG is calculated. These values may be utilized for quantitative comparisons of this experiments with other experiments.

Quantitative measurements may be performed at step 276. For example, the current expenment may be compared to a previous experiment (*e.g*., utilizing values calculated at step 270). Additionally, the experiment may be compared to hybridization intensities of RNA (such as from bacteria) present in the biological sample in a known quantity. In this manner, one may verify the correctness of the gene expression indication or call, modify threshold values, or perform any number of modifications of the preceding.

For simplicity, Fig. 9 was described in reference to a single gene. However, the process may be utilized on multiple genes in a biological sample. Therefore, any discussion of the analysis of a single gene is not an indication that the process may not be extended to processing multiple genes.

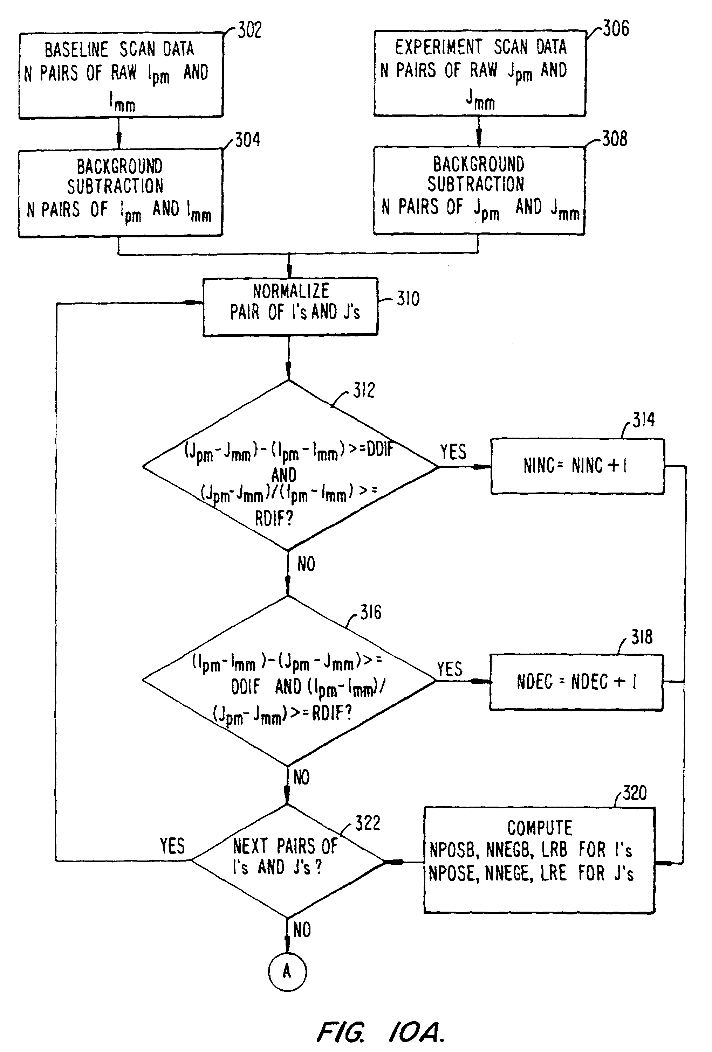

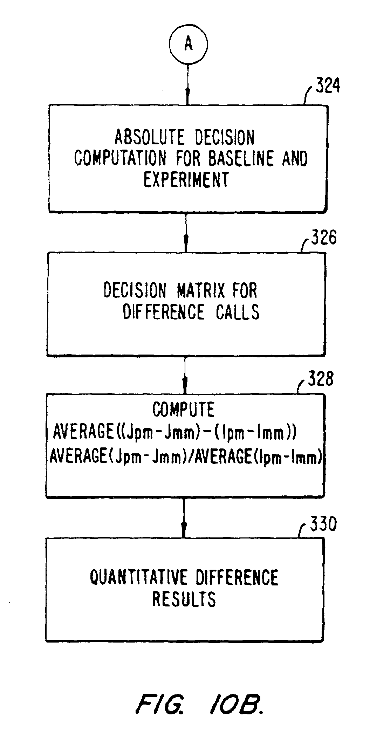

Figs. 10A and 10B show the flow of a process of determining the expression of a gene by comparing baseline scan data and experimental scan data. For example, the baseline scan data may be from a biological sample where it is known the gene is expressed. Thus, this scan data may be compared to a different biological sample to determine if the gene is expressed. Additionally, it may be determined how the expression of a gene or genes changes over time in a biological organism.

At step 302, the computer system receives raw scan data of N pairs of perfect match and mismatch probes from the baseline. The hybridization intensity of a perfect match probe from the baseline will be designed "Iₚₘ" and the hybridization intensity of a mismatch probe from the baseline will be designed "Iₘₘ." The background signal intensity is subtracted from each of the hybridization intensities of the pairs of baseline scan data at step 304.

At step 306, the computer system receives raw scan data of N pairs of perfect match and mismatch probes from the experimental biological sample. The hybridization intensity of a perfect match probes from the experiment will be designed "Jₚₘ" and the hybridization intensity of a mismatch probe from the experiment will be designed "Jₘₘ." The background signal intensity is subtracted from each of the hybridization intensities of the pairs of experimental scan data at step 308.

The hybridization intensities of an I and J pair may be normalized at step 310. For example, the hybridization intensities of the I and J pairs may be divided by the hybridization intensity of control probes as discussed in Section II.A.2.

At step 312, the hybridization intensities of the I and J pair of probes are compared to a difference threshold (DDIF) and a ratio threshold (RDIF). It is determined if the difference between the hybridization intensities of the one pair (Jₚₘ-Jₘₘ) and the other pair (Iₚₘ - Iₘₘ) are greater than or equal to the difference threshold AND the quotient of the hybridization intensities of one pair (Jₚₘ - Jₘₘ) and the other pair (Iₚₘ - Iₘₘ) are greater than or equal to the ratio threshold. The difference thresholds are typically user defined values that have been determined to produce accurate expression monitoring of a gene or genes.

If (Jₚₘ - Jₘₘ) - (Iₚₘ - Iₘₘ) > = DDIF and (Jₚₘ - Jₘₘ) / (Iₚₘ - Iₘₘ) > = RDIF, the value NINC is incremented at step 314. In general, NINC is a value that indicates the experimental pair of probes indicates that the gene expression is likely greater (or increased) than the baseline sample. NINC is utilized in a determination of whether the expression of the gene is greater (or increased), less (or decreased) or did not change in the experimental sample compared to the baseline sample.

At step 316, it is determined if (Jₚₘ - Jₘₘ) - (Iₚₘ - Iₘₘ) > = DDIF and (Jₚₘ - Jₘₘ) / (Iₚₘ / Iₘₘ) > = RDIF. If this expression is true, NDEC is incremented. In general, NDEC is a value that indicates the experimental pair of probes indicates that the gene expression is likely less (or decreased) than the baseline sample. NDEC is utilized in a determination of whether the expression of the gene is greater (or increased), less (or decreased) or did not change in the experimental sample compared to the baseline sample.

For each of the pairs that exhibits hybridization intensities either indicating the gene is expressed more or less in the experimental sample, the values NPOS, NNEG and LR are calculated for each pair of probes. These values are calculated as discussed above in reference to Fig. 9. A suffix of either "B" or "E" has been added to each value in order to indicate if the value denotes the baseline sample or the experimental sample, respectively. If there are next pairs of hybridization intensities at step 322, they are processed in a similar manner as shown.

Referring now to Fig. 10B, an absolute decision computation is performed for both the baseline and experimental samples at step 324. The absolute decision computation is an indication of whether the gene is expressed, marginal or absent in each of the baseline and experimental samples. Accordingly, in a preferred embodiment, this step entails performing steps 272 and 274 from Fig. 9 for each of the samples. This being done, there is an indication of gene expression for each of the samples taken alone.

At step 326, a decision matrix is utilized to determine the difference in gene expression between the two samples. This decision matrix utilizes the values, N, NPOSB, NPOSE, NNEGB, NNEGE, NINC, NDEC, LRB, and LRE as they were calculated above. The decision matrix performs different calculations depending on whether NINC is greater than or equal to NDEC. The calculations are as follows.

If NINC > = NDEC, the following four P values are determined:
P1 = NINC / NDEC
P2 = NINC / N
P3 = ((NPOSE - NPOSB) - (NNEGE - NNEGB)) / N
P4 = 10 * SUM(LRE - LRB) / N
These P values are then utilized to determine the difference in gene expression between the two samples.

For purposes of illustration, the P values are broken down into ranges as was done previously. Thus, all of the P values are broken down into ranges according to the following:
A = (P1 > = 2.7)
B = (2.7 > P1 > = 1.8)
C = (P1 < 1.8)

X = (P2 > = 0.24)
Y = (0.24 > P2 > = 0.16)
Z = (P2 < 0.160)

M = (P3 > = 0.17)
N = (0.17 > P3 > = 0.10)
O = (P3 < 0.10)

Q = (P4 > = 1.3)
R = (1.3 > P4 > = 0.9)
S = (P4 < 0.9)
Once the P values are broken down into ranges according to the above boolean values, the difference in gene expression between the two samples is determined.

In this case where NINC > = NDEC, the gene expression change is indicated as increased, marginal increase or no change. The following is a summary of the gene expression indications:
- Increased: A and (X or Y) and (Q or R) and (M or N or O)
A and (X or Y) and (Q or R or S) and (M or N)
B and (X or Y) and (Q or R) and (M or N)
A and X and (Q or R or S) and (M or N or O)
- Marginal: A or Y or S or O
- Increase: B and (X or Y) and (Q or R) and O
B and (X or Y) and S and (M or N)
C and (X or Y) and (Q or R) and (M or N)
- No Change: All others cases (*e.g*., any Z combination)
In the output to the user, increased may be indicated as "I," marginal increase as "MI" and no change as "NC."

If NINC < NDEC, the following four P values are determined:
P1 = NDEC / NINC
P2 = NDEC / N
P3 = ((NNEGE - NNEGB) - (NPOSE - NPOSB)) / N
P4 = 10 * SUM(LRE - LRB) / N
These P values are then utilized to determine the difference in gene expression between the two samples.

The P values are broken down into the same ranges as for the other case where NINC > = NDEC. Thus, P values in this case indicate the same ranges and will not be repeated for the sake of brevity. However, the ranges generally indicate different changes in the gene expression between the two samples as shown below.

In this case where NINC < NDEC. the gene expression change is indicated as decreased, marginal decrease or no change. The following is a summary of the gene expression indications:
- Decreased: A and (X or Y) and (Q or R) and (M or N or O)
A and (X or Y) and (Q or R or S) and (M or N)
B and (X or Y) and (Q or R) and (M or N)
A and X and (Q or R or S) and (M or N or O)
- Marginal: A or Y or S or O
- Decrease: B and (X or Y) and (Q or R) and O
B and (X or Y) and S and (M or N)
C and (X or Y) and (Q or R) and (M or N)
- No Change: All others cases (*e.g*., any Z combination)
In the output to the user, decreased may be indicated as "D," marginal decrease as "MD" and no change as "NC."

The above has shown that the relative difference between the gene expression between a baseline sample and an experimental sample may be determined. An additional test may be performed that would change an I, MI, D, or MD (*i*.*e*., not NC) call to NC if the gene is indicated as expressed in both samples (*e*.*g*., from step 324) and the following expressions are all true:
Average(IDIFB) > = 200
Average(IDIFE) > = 200
1.4 > = Average(IDIFE) / Average(IDIFB) > = 0.7
Thus, when a gene is expressed in both samples, a call of increased or decreased (whether marginal or not) will be changed to a no change call if the average intensity difference for each sample is relatively large or substantially the same for both samples. The IDIFB and IDIFE are calculated as the sum of all the IDIFs for each sample divided by N.

At step 328, values for quantitative difference evaluation are calculated. An average of ((Jₚₘ - Jₘₘ) - (Iₚₘ - Iₘₘ)) for each of the pairs is calculated. Additionally, a quotient of the average of Jₚₘ - Jₘₘ and the average of Iₚₘ - Iₘₘ is calculated. These values may be utilized to compare the results with other experiments in step 330.

### X. Monitoring Expression Levels

As indicated above, the methods of this invention may be used to monitor expression levels of a gene in a wide variety of contexts. For example, where the effects of a drug on gene expression is to be determined the drug will be administered to an organism, a tissue sample, or a cell. Nucleic acids from the tissue sample, cell, or a biological sample from the organism and from an untreated organism tissue sample or cell are isolated as described above, hybridized to a high density probe array containing probes directed to the gene of interest and the expression levels of that gene are determined as described above.

Similarly, where the expression levels of a disease marker (*e.g*., P53. RTK, or HER2) are to be detected (*e.g.*, for the diagnosis of a pathological condition in a patient), comparison of the expression levels of the disease marker in the sample to disease markers from a healthy organism will reveal any deviations in the expression levels of the marker in the test sample as compared to the healthy sample. Correlation of such deviations with a pathological condition provides a diagnostic assay for that condition.

### EXAMPLES

The following examples are offered to illustrate, but not to limit the present invention.

### Example 1

### First Generation Oligonucleotide Arrays Designed to Measure mRNA Levels for a Small Number of Murine Cytokines.

### A) Preparation of labeled RNA.

### 1) From each of the preselected genes.

Fourteen genes (IL-2, IL-3, Il-4, IL-6, Il-10, IL-12p40, GM-CSF, IFN-γ, TNF-α, CTLA8, β-actin, GAPDH, IL-11 receptor, and Bio B) were each cloned into the p Bluescript II KS (+) phagemid (Stratagene, La Jolla. California, USA). The orientation of the insert was such that T3 RNA polymerase gave sense transcripts and T7 polymerase gave antisense RNA.

Labeled ribonucleoudes in an in vitro transcription (IVT) reaction. Either biotin- or fluorescein-labeled UTP and CTP (1:3 labeled to unlabeled) plus unlabeled ATP and GTP were used for the reaction with 2500 units of T7 RNA polymerase (Epicentre Technologies, Madison, Wisconsin, USA). *In vitro* transcription was done with cut templates in a manner like that described by Melton *et al*., *Nucleic Acids Research,* 12: 7035-7056 (1984). A typical *in vitro* transcription reaction used 5 µg DNA template, a buffer such as that included in Ambion's Maxiscript *in vitro* Transcription Kit (Ambion Inc., Huston, Texas, USA) and GTP (3 mM), ATP (1.5 mM), and CTP and fluoresceinated UTP (3 mM total, UTP: Fl-UTP 3:1) or UTP and fluoresceinated CTP (2 mM total, CTP: Fl-CTP, 3:1). Reactions done in the Ambion buffer had 20 mM DTT and RNase inhibitor. The reaction was run from 1.5 to about 8 hours.

Following the reaction, unincorporated nucleotide triphosphates were removed using a size-selective membrane (microcon-100) or Pharmacia microspin S-200 column. The total molar concentration of RNA was based on a measurement of the absorbance at 260 nm. Following quantitation of RNA amounts. RNA was fragmented randomly to an average length of approximately 50 - 100 bases by heating at 94°C in 40 mM Tris-acetate pH 8.1, 100 mM potassium acetate, 30 mM magnesium acetate for 30 - 40 minutes. Fragmentation reduces possible interference from RNA secondary structure, and minimizes the effects of multiple interactions with closely spaced probe molecules.

### 2) From cDNA libraries.

Labeled RNA was produced from one of two murine cell lines: T10, a B cell plasmacytoma which was known not to express the genes (except IL-10, actin and GAPDH) used as target genes in this study, and 2D6, an IL-12 growth dependent T cell line (Th₁ subtype) that is known to express most of the genes used as target genes in this study. Thus, RNA derived from the T10 cell line provided a good total RNA baseline mixture suitable for spiking with known quantities of RNA from the particular target genes. In contrast, mRNA derived from the 2D6 cell line provided a good positive control providing typical endogenously transcribed amounts of the RNA from the target genes.

### i) The T10 murine B cell line.

The T10 cell line (B cells) was derived from the IL-6 dependent murine plasmacytoma line T1165 (Nordan *et al.* (1986) *Science* 233: 566-569) by selection in the presence of IL-11. To prepare the directional cDNA library, total cellular RNA was isolated from T10 cells using RNAStat60 (Tel-Test B), and poly (A)⁺ RNA was selected using the PolyAtract kit (Promega, Madison, Wisconsin, USA). First and second strand cDNA was synthesized according to Toole *et al*., (1984) *Nature*, 312: 342-347, except that 5-methyldeoxycytidine 5'triphosphate (Pharmacia LKB, Piscataway. New Jersey, USA) was substituted for DCTP in both reactions.

To determine cDNA frequencies T10 libraries were plated, and DNA was transfered to nitrocellulose filters and probed with ³²P-labeled β-actin, GAPDH and IL-10 probes. Actin was represented at a frequency of 1:3000, GAPDH at 1:1000, and IL-10 at 1:35,000. Labeled sense and antisense T10 RNA samples were synthesized from NotI and SfiI cut CDNA libraries in *in vitro* transcription reactions as described above.

### ii) The 2D6 murine helper T cells line.

The 2D6 cell line is a murine IL-12 dependent T cell line developed by Fujiwara *et al*. Cells were cultured in RPMI 1640 medium with 10% heat inactivated fetal calf serum (JRH Biosciences), 0.05 mM P-mercaptoethanoi and recombinant murine IL-12 (100 units/mL. Genetics Institute, Cambridge, Massachusetts, USA). For cytokine induction, cells were preincubated overnight in IL-12 free medium and then resuspended (10⁶ cells/ml). After incubation for 0, 2, 6 and 24 hours in media containing 5 nM calcium ionophore A23187 (Sigma Chemical Co., St. Louis Missouri, USA) and 100 nM 4-phorbol-12-myristate 13-acetate (Sigma), cells were collected by centrifugation and washed once with phosphate buffered saline prior to isolation of RNA.

Labeled 2D6 mRNA was produced by directionally cloning the 2D6 cDNA with αZipLox, NotI-Sall arms available from GibcoBRL in a manner similar to T10. The linearized pZ11 library was transcribed with T7 to generate sense RNA as described above.

### iii) RNA preparation.

For material made directly from cellular RNA, cyloplasmic RNA was extracted from cells by the method of Favaloro *et al*., (1980) *Meth. Enzym*., 65: 718-749, and poly (A)⁺ RNA was isolated with an oligo dT selection step (PolyAtract, Promega, ). RNA was amplified using a modification of the procedure described by Eberwine *et al.* (1992) *Proc. Natl. Acad. Sci. USA, 89: 3010-3014* (*see also* Van Gelder *et al.* (1990) *Science* 87: 1663-1667). One microgram of poly (A)+ RNA was converted into double-stranded cDNA using a cDNA synthesis kit (Life Technologies) with an oligo dT prime incorporating a T7 RNA polymerase promoter site. After second strand synthesis, the reaction mixture was extracted with phenol/chloroform and the double-stranded DNA isolated using a membrane filtration step (Mircocon-100. Amicon, Inc. Beverly, Massachusetts, USA). Labeled cRNA was made directly from the cDNA pool with an IVT step as described above. The total molar concentration of labeled CRNA was determined from the absorbance at 260 and assuming an average RNA size of 1000 ribonucleotides. RNA concentration was calculated using the conventional conversion that 1 OD is equivalent to 40 µg of RNA, and that 1 µg of cellular mRNA consists of 3 pmoles of RNA molecules.

Cellular mRNA was also labeled directly without any intermediate cDNA or RNA synthesis steps. Poly (A)⁺ RNA was fragmented as described above, and the 5' ends of the fragments were kinased and then incubated ovenight with a biotinylated oligoribonucleotide (5'-biotin-AAAAAA-3') in the presence of T4 RNA ligase (Epicentre Technologies). Alternatively, mRNA was labeled directly by UV-induced crosslinking to a psoralen derivative linked to biotin (Schleicher & Schuell).

### B) High Density Array Preparation

A high density array of 20 mer oligonucleotide probes was produced using VLSIPS technology. The high density array included the oligonucleotide probes as listed in Table 2. A central mismatch control probe was provided for each gene-specific probe resulting in a high density array containing over 16,000 different oligonucleotide probes.

**Table 2.**

| High density array design. For every probe there was also a mismatch control having a central 1 base mismatch. | | |
|---|---|---|
| Probe Type | Target Nucleic Acid | Number of Probes |
| Test Probes: | IL-2 | 691 |
| | IL-3 | 751 |
| | IL-4 | 361 |
| | IL-6 | 691 |
| | IL-10 | 481 |
| | IL-12p40 | 911 |
| | GM-CSF | 661 |
| | IFN-γ | 991 |
| | TNF-α | 641 |
| | mCTLA8 | 391 |
| | IL-11 receptor | 158 |
| House Keeping Genes: | GAPDH | 388 |
| | β-actin | 669 |
| Bacterial gene (sample preparation/amplification control) | Bio B | 286 |

The high density array was synthesized on a planar glass slide.

### C) Array hybridization and scanning.

The RNA transcribed from cDNA was hybridized to the high density oligonucleotide probe array(s) at low stringency and then washed under more stringent conditions. The hybridization solutions contained 0.9 M NaCl, 60 mM NaH₂PO₄, 6 mM EDTA and 0.005 % Triton X-100 , adjusted to pH 7.6 (referred to as 6x SSPE-T). In addition, the solutions contained 0.5 mg/ml unlabeled, degraded herring sperm DNA (Sigma Chemical Co., St. Louis, Missouri, USA). Prior to hybridization, RNA samples were heated in the hybridization solution to 9 "C for 10 minutes, placed on ice for 5 minutes, and allowed to equilibrate at room temperature before being placed in the hybridization flow cell. Following hybridization, the solution was removed, the arrays were washed with 6xSSPE-T at 22°C for 7 minutes, and then washed with 0.5x SSPE-T at 40°C for 15 minutes. When biotin-labeled RNA was used, the hybridized RNA was stained with a streptavidin-phycoerythrin conjugate (Molecular Probes, Inc., Eugene. Oregon, USA) prior to reading. Hybridized arrays were stained with 2 µg/ml streptavidinphycoerythrin in 6xSSPE-T at 40°C for 5 minutes.

The arrays were read using scanning confocal microscope (Molecular Dynamics, Sunnyvale, California, USA) modified for the purpose. The scanner uses an argon ion laser as the excitation source, and the emission was detected with a photomultiplier tube through either a 530 nm bandpass filter (fluorescein) or a 560 nm longpass filter (phycoerythrin).

Nucleic acids of either sense or antisense orientations were used in hybridization experiments. Arrays with for either orientation (reverse complements of each other) were made using the same set of photolithographic masks by reversing the order of the photochemical steps and incorporating the complementary nucleotide.

### D) Quantitative analysis of hybridization patterns and intensities.

The quantitative analysis of the hybridization results involved counting the instances in which the perfect match probe (PM) was brighter than the corresponding mismatch probe (MM), averaging the differences (PM minus MM) for each probe family (*i.e*., probe collection for each gene), and comparing the values to those obtained in a side-by-side experiment on an identically synthesized array with an unspiked sample (if applicable). The advantage of the difference method is that signals from random cross hybridization contribute equally, on average, to the PM and MM probes while specific hybridization contributes more to the PM probes. By averaging the pairwise differences, the real signals add constructively while the contributions from cross hybridization tend to cancel.

The magnitude of the changes in the average of the difference (PM-MM) values was interpreted by comparison with the results of spiking experiments as well as the signal observed for the internal standard bacterial RNA spiked into each sample at a known amount. Analysis was performed using algorithms and software described herein.

### D) Optimization of Probe Selection

In order to optimize probe selection for each of the target genes, the high density array of oligonucleotide probes was hybridized with the mixture of labeled RNAs transcribed from each of the target genes. Fluorescence intensity at each location on the high density array was determined by scanning the high density array with a laser illuminated scanning confocal fluorescence microscope connected to a data acquisition system.

Probes were then selected for further data analysis in a two-step procedure. First, in order to be counted, the difference in intensity between a probe and its corresponding mismatch probe had to exceed a threshold limit (50 counts, or about half background, in this case). This eliminated from consideration probes that did not hybridize well and probes for which the mismatch control hybridizes at an intensity comparable to the perfect match.

The high density array was hybridized to a labeled RNA sample which, in principle, contains none of the sequences on the high density array. In this case, the oligonucleotide probes were chosen to be complementary to the sense RNA. Thus, an anti-sense RNA population should have been incapable of hybridizing to any of the probes on the array. Where either a probe or its mismatch showed a signal above a threshold value (100 counts above background) it was not included in subsequent analysis.

Then, the signal for a particular gene was counted as the average difference (perfect match - mismatch control) for the selected probes for each gene.

### E) Results: The high density arrays provide specific and sensitive detection of target nucleic acids.

As explained above, the initial arrays contained more than 16,000 probes that were complementary to 12 murine mRNAs - 9 cytokines, 1 cytokine receptor, 2 constitutively expressed genes (5-actin and glyceraldehyde 3-phosphate dehydrogenase) - 1 rat cytokine and 1 bacterial gene (*E. coli* biotin synthetase, *bioB*) which serves as a quantitation reference. The initial experiments with these relatively simple arrays were designed to determine whether short *in situ* synthesized oligonucleotides can be made to hybridize with sufficient sensitivity and specificity to quantitatively detect RNAs in a complex cellular RNA population. These arrays were intentionally highly redundant, containing hundreds of oligonucleotide probes per RNA, many more than necessary for the determination of expression levels. This was done to investigate the hybridization behavior of a large number of probes and develop general sequence rules for *a priori* selection of minimal probe sets for arrays covering substantially larger numbers of genes.

The oligonucleotide arrays contained collections of pairs of probes for each of the RNAs being monitored. Each probe pair consisted of a 20-mer that was perfectly complementary (referred to as a perfect match, or PM probe) to a subsequence of a particular message, and a companion that was identical except for a single base difference in a central position. The mismatch (MM) probe of each pair served as an internal control for hybridization specificity. The analysis of PM/MM pairs allowed low intensity hybridization patterns from rare RNAs to be sensitively and accurately recognized in the presence of crosshybridization signals.

For array hybridization experiments, labeled RNA target samples were prepared from individual clones, cloned CDNA libraries, or directly from cellular mRNA as described above. Target RNA for array hybridization was prepared by incorporating fluorescently labeled ribonucleotides in an *in vitro* transcription (IVT) reaction and then randomly fragmenting the RNA to an average size of 30 - 100 bases. Samples were hybridized to arrays in a self-contained flow cell (volume ⁻200 µL) for times ranging from 30 minutes to 22 hours. Fluorescence imaging of the arrays was accomplished with a scanning confocal microscope (Molecular Dynamics). The entire array was read at a resolution of 11.25 µm (^{∼} 80-fold oversampling in each of the 100 x 100 µm synthesis regions) in less than 15 minutes, yielding a rapid and quantitative measure of each of the individual hybridization reactions.

### 1) Specificity of Hybridization

In order to evaluate the specificity of hybridization, the high density array described above was hybridized with 50 pM of the RNA sense strand of IL-2, IL-3, IL-4, IL-6, Actin, GAPDH and Bio B or IL-10, IL-12p40, GM-CSF, IFN-γ, TNF-α, mCTLA8 and Bio B. The hybridized array showed strong specific signals for each of the test target nucleic acids with minimal cross hybridization.

### 2) Detection of Gene Expression levels in a complex target sample.

To determine how well individual RNA targets could be detected in the presence of total mammalian cell message populations, spiking experiments were carried out. Known amounts of individual RNA targets were spiked into labeled RNA derived from a representative cDNA library made from the murine B cell line T10. The T10 cell line was chosen because of the cytokines being monitored, only IL-10 is expressed at a detectable level.

Because simply spiking the RNA mixture with the selected target genes and then immediately hybridizing might provide an artificially elevated reading relative to the rest of the mixture, the spiked sample was treated to a series of procedures to mitigate differences between the library RNA and the added RNA. Thus the "spike" was added to the sample which was then heated to 37°C and annealed. The sample was then frozen, thawed, boiled for 5 minutes, cooled on ice and allowed to return to room temperature before performing the hybridization.

Figure 2A shows the results of an experiment in which 13 target RNAS were spiked into the total RNA pool at a level of 1:3000 (equivalent to a few hundred copies per cell). RNA frequencies are given as the molar amount of an individual RNA per mole of total RNA. Figure 2B shows a small portion of the array (the boxed region of 2A) containing probes specific for interleukin-2 and interleukin-3 (IL-2 and IL-3,) RNA, and Figure 2C shows the same region in the absence of the spiked targets. The hybridization signals are specific as indicated by the comparison between the spiked and unspiked images, and perfect match (PM) hybridizations are well discriminated from missmatches (MM) as shown by the pattern of alternating brighter rows (corresponding to PM probes) and darker rows (corresponding to MM probes). The observed variation among the different perfect match hybridization signals was highly reproducible and reflects the sequence dependence of the hybridizations. In a few instances, the perfect match (PM) probe was not significantly brighter than its mismatch (MM) partner because of cross-hybridization with other members of the complex RNA population. Because the patterns are highly reproducible and because detection does not depend on only a single probe per RNA, infrequent cross hybridization of this type did not preclude sensitive and accurate detection of even low level RNAS.

Similarly, infrequent poor hybridization due to, for example, RNA or probe secondary structure, the presence of polymorphism or database sequence errors does not preclude detection. An analysis of the observed patterns of hybridization and cross hybridization led to the formulation of general rules for the selection of oligonucleotide probes with the best sensitivity and specificity described herein.

### 3) Relationship between Target Concentration and Hybridization Signal

A second set of spiking experiments was carried out to determine the range of concentrations over which hybridization signals could be used for direct quantitation of RNA levels. Figure 3 shows the results of experiments in which the ten cytokine RNAs were spiked together into 0.05 mg/ml of labeled RNA from the B cell (T10) cDNA library at levels ranging from 1:300 to 1:300.000. A frequency of 1:300,000 is that of an mRNA present at less than a few copies per cell. In 10 µg of total RNA and a volume of 200 µl, a frequency of 1:300,000 corresponds to a concentration of approximately 0.5 picomolar and 0.1 femptomole (⁻ 6 x 10⁷ molecules or about 30 picograms)of specific RNA.

Hybridizations were carried out in parallel at 40°C for 15 to 16 hours. The presence of each of the 10 cytokine RNAs was reproducibly detected above the background even at the lowest frequencies. Furthermore, the hybridization intensity was linearly related to RNA target concentration between 1:300,000 and 1:3000 (Figure 3). Between 1:3000 and 1:300, the signals increased by a factor of 4 - 5 rather than 10 because the probe sites were beginning to saturate at the higher concentrations in the course of a 15 hour hybridization. The linear response range can be extended to higher concentrations by reducing the hybridization time. Short and long hybridizations can be combined to quantitatively cover more than a 10⁴-fold range in RNA concentration.

Blind spiking experiments were performed to test the ability to simultaneously detect and quantitate multiple related RNAs present at a wide range of concentrations in a complex RNA population. A set of four samples was prepared that contained 0.05 mg/ml of sense RNA transcribed from the munne B cell CDNA library, plus combinations of the 10 cytokine RNAs each at a different concentration. Individual cytokine RNAs were spiked at one of the following levels: 0, 1:300,000, 1:30,000. 1:3000, or 1:300. The four samples plus an unspiked reference were hybridized to separate arrays for 15 hours at 40°C. The presence or absence of an RNA target was determined by the pattern of hybridization and how it differed from that of the unspiked reference, and the concentrations were detected by the intensities. The concentrations of each of the ten cytokines in the four blind samples were correctly determined, with no false positives or false negatives.

One case is especially noteworthy: IL-10 is expressed in the mouse B cells used to make the CDNA library, and was known to be present in the library at a frequency of 1:60,000 to 1:30,000. In one of the unknowns, an additional amount of IL-10 RNA (corresponding to a frequency of 1:300.000) was spiked into the sample. The amount of the spiked IL-10 RNA was correctly determined, even though it represented an increase of only 10 - 20% above the intrinsic level. These results indicate that subtle changes in expression are sensitively determined by performing side-by-side experiments with identically prepared samples on identically synthesized arrays.

### Example 2

### T Cell Induction Experiments Measuring Cytokine mRNAs as a Function of Time Following Stimulation.

The high density arrays of this invention were next used to monitor cytokine MRNA levels in murine T cells at different times following a biochemical stimulus. Cells from the murine T helper cell line (2D6) were treated with the phorbol ester 4-phorbol-12-myristate 13-acetate (PMA) and a calcium ionophore. Poly (A)⁺ MRNA was then isolated at 0, 2, 6 and 24 hours after stimulation. Isolated mRNA (approximately 1 µg) was converted to labeled antisense RNA using a procedure that combines a double-stranded cDNA synthesis step with a subsequent *in vitro* transcription reaction. This RNA synthesis and labeling procedure amplifies the entire mRNA population by 20 to 50-fold in an apparently unbiased and reproducible fashion (Table 2).

The labeled antisense T-cell RNA from the four time points was then hybridized to DNA probe arrays for 2 and 22 hours. A large increase in the γ-interferon mRNA level was observed, along with significant changes in four other cytokine mRNAs (IL-3, IL-10, GM-CSF and TNFα). As shown in Figure 4, the cytokine messages were not induced with identical kinetics. Changes in cytokine mRNA levels of less than 1:130,000 were unambiguously detected along with the very large changes observed for γ-interferon.

These results highlight the value of the large experimental dynamic range inherent in the method. The quantitative assessment of RNA levels from the hybridization results is direct, with no additional control hybridizations, sample manipulation, amplification, cloning or sequencing. The method is also efficient. Using current protocols, instrumentation and analysis software, a single user with a single scanner can read and analyze as many as 30 arrays in a day.

### Example 3

### Higher-Density Arrays Containing 65,000 probes for over 100 Murine Genes

Figure 5 shows an array that contains over 65,000 different oligonucleotide probes (50 µm feature size) following hybridization with an entire murine B cell RNA population. Arrays of this complexity were read at a resolution of 7.5 lim in less than fifteen minutes. The array contains probes for 118 genes including 12 murine genes represented on the simpler array described above, 35 U.S.C. §102() additional murine genes, three bacterial genes and one phage gene. There are approximately 300 probe pairs per gene, with the probes chosen using the selection rules described herein. The probes were chosen from the 600 bases of sequence at the 3' end of the translated region of each gene. A total of 21 murine RNAs were unambiguously detected in the B cell RNA population, at levels ranging from approximately 1:300.000 to 1:100.

Labeled RNA samples from the T cell induction experiments (Fig. 4) were hybridized to these more complex 118-gene arrays, and similar results were obtained for the set of genes in common to both chip types. Expression changes were unambiguously observed for more than 20 other genes in addition to those shown in Figure 4.

To determine whether much smaller sets of probes per gene are sufficient for reliable detection of RNAs, hybridization results from the 118 gene chip were analyzed using ten different subsets of 20 probe pairs per gene. That is to say, the data were analyzed as if the arrays contained only 20 probe pairs per gene. The ten subsets of 20 pairs were chosen from the approximately 300 probe pairs per gene on the arrays. The initial probe selection was made utilizing the probe selection and pruning algorithms described above. The ten subjects of 20 pairs were then randomly chosen from those probes that survived selection and pruning. Labeled RNAs were spiked into the murine B cell RNA population at levels of 1:25,000, 1:50,000 and 1:100,000. Changes in hybridization signals for the spiked RNAs were consistently detected at all three levels with the smaller probe sets. As expected, the hybridization intensities do not cluster as tightly as when averaging over larger numbers of probes. This analysis indicates that sets of 20 probe pairs per gene are sufficient for the measurement of expression changes at low levels, but that improvements in probe selection and experimental procedures will are preferred to routinely detect RNAs at the very lowest levels with such small probe sets. Such improvements include, but are not limited to higher stnngency hybridizations coupled with use of slightly longer oligonucleotide probes (*e.g*., 25 mer probes)) are in progress.

### Example 4

### Scale Up to Thousands of Genes

A set of four high density arrays each containing 25-mer oligonucleotide probes approximately 1650 different human genes provided probes to a total of 6620 genes There were about 20 probes for each gene. The feature size on arrays was 50 microns This high density array was successfully hybridized to a cDNA library using essentially the protocols described above. Similar sets of high density arrays containing oligonucleotide probes to every known expressed sequence tag (EST) are in preparation.

### Example 5

### Direct Scale up for the Simultaneous Monitoring of Tens of Thousands of RNAs.

In addition to being sensitive, specific and quantitative, the approach described here is intrinsically parallel and readily scalable to the monitoring of very large numbers of mRNAs. The number of RNAs monitored can be increased greatly by decreasing the number of probes per RNA and increasing the number of probes per array. For example, using the above-described technology, arrays containing as many as 400,000 probes in an area of 1.6 cm² (20 x 20 µm synthesis features) are currently synthesized and read. Using 20 probe pairs per gene allows 10,000 genes to be monitored on a single array while maintaining the important advantages of probe redundancy. A set of four such arrays could cover the more than 40,000 human genes for which there are expressed sequence tags (ESTS) in the public data bases, and new ESTs can be incorporated as they become available. Because of the combinatorial nature of the chemical synthesis, arrays of this complexity are made in the same amount of time with the same number of steps as the simpler ones used here. The use of even fewer probes per gene and arrays of higher density makes possible the simultaneous monitoring of all sequenced human genes on a single, or small number of small chips.

The quantitative monitoring of expression levels for large numbers of genes will prove valuable in elucidating gene function, exploring the causes and mechanisms of disease, and for the discovery of potential therapeutic and diagnostic targets. As the body of genomic information grows, highly parallel methods of the type described here provide an efficient and direct way to use sequence information to help elucidate the underlying physiology of the cell.

### Example 6

### Probe Selection Using a Neural Net

A neural net can be trained to predict the hybridization and cross hybridization intensities of a probe based on the sequence of bases in the probe, or on other probe properties The neural net can then be used to pick an arbitrary number of the "best" probes. When a neural net was trained to do this it produced a moderate (0.7) correlation between predicted intensity and measured intensity, with a better model for cross hybridization than hybridization

### A) Input/output mapping.

The neural net was trained to identify the hybridization properties of 20-mer probes. The 20-mer probes were mapped to an eighty bit long input vector, with the first four bits representing the base in the first position of the probe, the next four bits representing the base in the second position, *etc.* Thus, the four bases were encoded as follows:

| | |
|---|---|
| A | 1000 |
| C | 0100 |
| G | 0010 |
| T | 0001 |

The neural network produced two outputs; hybridization intensity, and crosshybridization intensity. The output was scaled linearly so that 95% of the outputs from the actual experiments fell in the range 0 to 1.

### B) Neural net architecture.

The neural net was a backpropagation network with 80 input neurons, one hidden layer of 20 neurons, and an output layer of two neurons A sigmoid transfer function was used. ( s(x) = 1/(1+ exp(-1 * x)) ) that scales the input values from 0 to 1 in a non-linear (sigmoid) manner.

### C) Neural net training.

The network was trained using the default parameters from Neural Works Professional 2.5 for a backprop network (Neural Works Professional is a product of NeuralWare, Pittsburgh Pennsylvania, USA). The training set consisted of approximately 8000 examples of probes, and the associated hybridization and crosshybridization intensities.

### D) Neural net weights.

Neural net weights are provided in two matrices, an 81 x 20 matrix (Table 3) (weights_1 ) and a 2 x 20 matrix Table 4 (weights_2)

**Table 3.**

| Neural net weights (81 x 20 matrix) (weights_1) | | | | | |
|---|---|---|---|---|---|
| -0.0316746 | -0.0263491 | 0.15907079 | -0.0353881 | -0.0529314 | 0.09014647 |
| 0.19370709 | -0.0515666 | 0.06444275 | -0.0480836 | 0.29237783 | -0.034054 |
| 0.02240546 | 0.08460676 | 0.14313674 | 0.06798329 | 0.06746746 | 0.033717 |
| 0.16692482 | -0.0913482 | 0.05571244 | 0.22345543 | 0.04707823 | -0.0035547 |
| 0.02129388 | 0.12105247 | 0.1405973 | -0.0066357 | -0.0760119 | 0.11165894 |
| 0.03684745 | -0.0714359 | 0.02903421 | 0.09420238 | 0.12839544 | 0.08542864 |
| 0.00603615 | 0.04986877 | 0.02134438 | 0.0852259 | 0.13453935 | 0.03089394 |
| 0.11111762 | 0.12571541 | 0.09278143 | 0.11373715 | 0.03250757 | -0.0460193 |
| 0.01354388 | 0.1131407 | 0.06123798 | 0.14818664 | 0.07090721 | 0.05089445 |
| -0.0635492 | -0.0227965 | 0.1081195 | 0.13419148 | 0.08916269 | -0.010634 |
| 0.18790121 | 0.09624594 | -0.0865264 | -0.0126238 | 0.11497019 | -0.0057307 |
| 0.02378313 | 0.10295142 | 0.05553147 | -0.0193289 | -0.0627925 | -0.024633 |
| -0.0403537 | 0.23566079 | 0.10335726 | 0.07325625 | 0.11329328 | 0.2555581 |
| -0.0694051 | -0.0637478 | 0.2687766= | | | |
| | | | | | |
| -0.0731941 | 0.08858298 | 0.39719725 | -0.0709359 | 0.14039235 | 0.23244983 |
| 0.06500423 | 0.11003297 | 0.0403917 | 0.02953459 | 0.26901209 | -0.0605089 |
| 0.03036973 | 0.06836637 | 0.02345118 | 0.0206452 | -0.0079707 | 0.20967795 |
| 0.17097448 | -0.007098 | -0.0348659 | 0.09989586 | 0.07417496 | -0.1236805 |
| 0.05442215 | 0.23686385 | 0.01979881 | -9.80E-06 | -0.0549301 | 0.08891765 |
| 0.08683836 | 0.14047802 | 0.00982503 | 0.11756061 | 0.09054346 | -0.028868 |
| 0.08829379 | 0.17881326 | 0.12465772 | 0.13134554 | 0.09500015 | 0.04572553 |
| 0.0749867 | 0.08564588 | 0.05334799 | 0.14341639 | 0.11468539 | 0.14277624 |
| 0.05022619 | 0.14544216 | 0.03519877 | 0.12799838 | 0.01427337 | 0.16172577 |
| 0.08078995 | -0.0022168 | 0.05439407 | -0.0789278 | 0.07312368 | 0.11417327 |
| 0.03405219 | 0.06140256 | 0.01802093 | 0.0954654 | 0.00130152 | -0.035995 |
| 0.11517255 | 0.17431773 | 0.09664405 | 0.01782892 | 0.03840308 | 0.05180788 |
| 0.14236264 | 0.17182963 | 0.02306779 | -0.0489743 | -0.0006051 | 0.19077648 |
| -0.0866363 | 0.11008894 | 0.40543473= | | | |
| | | | | | |
| -0.0163019 | 0.06256609 | 0.16058824 | 0.14149499 | 0.15698175 | -0.1197781 |
| 0.38030735 | 0.28241798 | 0.2882407 | -0.2227429 | 0.34799534 | 0.38490915 |
| 0.23144296 | -0.3207987 | 0.56366867 | 0.35976714 | 0.20325871 | -0.343972 |
| 0.46158856 | 0.20649959 | 0.35099933 | -0.5071837 | 0.56459975 | 0.21605791 |
| 0.45084599 | -0.5829023 | 0.51297456 | 0.33494622 | 0.43086055 | -0.5538613 |
| 0.55080342 | 0.30968052 | 0.54485208 | -0.7155912 | 0.30799151 | 0.29871368 |
| 0.36848074 | -0.5196409 | 0.33829662 | 0.21612473 | 0.41646513 | -0.5573701 |
| 0.47133151 | 0.30909833 | 0.37790757 | -0.464661 | 0.50172138 | 0.21158406 |
| 0.46017882 | -0.5331213 | 0.60684419 | 0.47586009 | 0.28597337 | -0.3345993 |
| 0.33042327 | 0.4072904 | 0.24270254 | -0.3750777 | 0.14083703 | 0.30998308 |
| 0.19591335 | -0.4028497 | 0.30585453 | 0.35896543 | 0.24851802 | -0.2937264 |
| 0.19672842 | 0.16133355 | 0.21780767 | -0.2419563 | 0.17847325 | 0.07593013 |
| 0.1710967 | -0.2728708 | 0.1234024 | 0.06987085 | 0.1741322 | 0.05922241 |
| 0.03326527 | 0.22045346 | 0.98782647= | | | |
| | | | | | |
| -0.0752053 | -0.0571054 | -0.1834571 | 0.14263187 | -0.0715346 | -0.0524248 |
| -0.0838031 | 0.01667063 | -0.0945634 | -0.1137057 | -0.1040308 | 0.04263301 |
| -0.2039919 | -0.0532526 | -0.0828366 | 0.1373803 | -0.0562212 | -0.2127942 |
| -0.0482095 | 0.04316666 | -0.1732933 | 0.0550463 | -0.0526818 | 0.06739104 |
| -0.0065265 | -0.2011867 | -0.0434558 | -0.0369132 | -0.0196296 | -0.1314755 |
| 0.09420983 | -0.0010159 | -0.1768979 | -0.2365085 | -0.0150508 | 0.14120786 |
| 0.00565713 | -0.1990354 | 0.11568499 | -0.0690084 | -0.1509431 | -0.0575663 |
| 0.11275655 | 0.01772332 | -0.0016695 | -0.249011 | 0.09066539 | 0.05357879 |
| -0.0850152 | -0.1931012 | 0.08498721 | 0.03673514 | -0.1446398 | -0.199778 |
| 0.1065109 | 0.07205399 | -0.1304159 | -0.1723315 | 0.09151162 | 0.05596334 |
| -0.0922655 | -0.1478272 | 0.08858409 | 0.14206541 | -0.0314846 | -0.1985286 |
| 0.19862956 | -0.0502828 | -0.11447 | -0.1440073 | 0.01366408 | 0.11101657 |
| -0.0721622 | -0.1506944 | 0.14910588 | 0.03297219 | -0.0266356 | -0.2501774 |
| 0.20344114 | -0.061502 | -0.1647823= | | | |
| | | | | | |
| 0.02848385 | 0.00254791 | -0.0646306 | 0.02634032 | -0.0654473 | 0.04731949 |
| -0.0742345 | -0.0545447 | -0.1119258 | 0.10765317 | -0.0606677 | 0.05693235 |
| -0.0747124 | 0.13325705 | -0.0508435 | -0.1761459 | -0.0883804 | -0.0777852 |
| -0.1090026 | -0.0988943 | -0 0445145 | 0.03802977 | -0.0484086 | -0.0337959 |
| 0.07326921 | 0.02654305 | -0.1239398 | 0.03043288 | 0.09781751 | 0.02590732 |
| -0.0586419 | -0.08015 | -0.0073617 | -0.1682889 | 0.00400978 | 0.01282504 |
| 0.05150735 | -0.1449667 | 0.06144469 | 0.1005446 | 0.22570252 | -0.3763289 |
| -0.0001517 | -0.0521925 | 0.21106339 | -0.4393073 | 0.0053312 | 0.13283829 |
| 0.12470152 | -0.3589714 | -0.0061972 | 0.07370338 | 0.25447422 | -0.3289591 |
| -0.049451 | 0.05717351 | 0.14784867 | -0.3082401 | 0.01207511 | -0.1141143 |
| 0.18880892 | -0.3259364 | 0.04754021 | -0.0576587 | 0.02376083 | -0.2828108 |
| 0.0234996 | -0.1177034 | 0.02549919 | -0.1671077 | 0.00582423 | -0.0715723 |
| 0.16712189 | -0.0122822 | -0.109654 | -0.0327367 | 0.01481733 | -0.0636454 |
| -0.0487184 | 0.01467591 | -0.0759871= | | | |
| | | | | | |
| 0.146753 | -0.0931665 | -0.1475015 | 0.07284982 | -0.0609536 | -0.0945313 |
| -0.0739603 | 0.17018235 | -0.0636651 | 0.04693379 | -0.2586751 | 0.15550844 |
| -0.1548294 | -0.0908961 | -0.0415557 | 0.04915113 | -0.0436857 | -0.031472 |
| -0.1728483 | 0.12621336 | -0.1321529 | -0.1091831 | -0.0989133 | 0.0294641 |
| -0.0950026 | -0.1562225 | -0.0917397 | 0.18711324 | 0.04599057 | -0.2039073 |
| 0.07691807 | 0.13016214 | 0.10801306 | -0.3151104 | 0.0105284 | 0.10938062 |
| -0.035349 | -0.302975 | 0.03706082 | 0.12322487 | 0.07198878 | -0.2535323 |
| 0.04664604 | 0.08887579 | -0.0210248 | -0.1427284 | 0.09078772 | 0.08646259 |
| 0.00194441 | -0.1631221 | 0.11259725 | -0.0984519 | -0.0939511 | -0.218395 |
| 0.13777457 | 0.00339417 | -0.2007502 | -0.0703103 | 0.1548807 | 0.13540466 |
| -0.0514387 | -0.0722146 | 0.07706029 | 0.04593663 | -0.2334163 | -0.0250262 |
| 0.0994828 | -0.035077 | -0.106266 | -0.059766 | 0.13616422 | 0.22308858 |
| -0.1571046 | -0.1713289 | 0.14155054 | 0.00283311 | 0.01067419 | -0.360891 |
| 0.13411179 | -0.0159559 | -0.1296399= | | | |
| | | | | | |
| -0.0304715 | -0.0845574 | 0.17682472 | -0.0552084 | 0.07044557 | -0.1482136 |
| 0.13328855 | -0.1492282 | 0.11350834 | -0.1121938 | 0.02089526 | 0.00104415 |
| 0.0217719 | -0.3102229 | 0.18922243 | -0.0940011 | 0.08787836 | -0.1835242 |
| 0.04117605 | 0.03997391 | 0.06022124 | -0.1808036 | 0.04742034 | -0.0744867 |
| 0.08965616 | -0.1572192 | 0.00942572 | 0.07957069 | 0.12980177 | -0.2440033 |
| 0.08670026 | 0.03785197 | 0.21052985 | -0.3564453 | 0.01492627 | 0.04286519 |
| 0.00865917 | -0.2995701 | -0.0835971 | 0.14536868 | 0.08446889 | -0.1689682 |
| -0.1322389 | 0.21433547 | 0.08046963 | -0.1548838 | -0.021533 | 0.0558197 |
| 0.1623435 | -0.3362183 | -0.1335399 | 0.10284293 | 0.16658102 | -0.3004514 |
| -0.0887844 | 0.07691832 | 0.11459036 | -0.056257 | 0.01970494 | 0.08940192 |
| 0.08622501 | -0.2421202 | 0.00845924 | -0.0151014 | 0.19088623 | -0.1967196 |
| -0.0290916 | -0.0839412 | 0.10590381 | -0.1593935 | -0.0399097 | -0.0861852 |
| 0.17453311 | -0.1529943 | 0.02726452 | 0.06178628 | 0.06624542 | 0.01004315 |
| -0 158326 | -0.0149114 | -0.1479269= | | | |
| | | | | | |
| 0.11429903 | -0.0432327 | 0.14520219 | 0.51860482 | 0.19151463 | -0.1127352 |
| 0.33529782 | 0.24581231 | 0.07311282 | -0.2268714 | 0.31717882 | 0.35736522 |
| 0.09062219 | -0.2974442 | 0.46336258 | 0.17145836 | 0.32802406 | -0.3898261 |
| 0.49959001 | 0.22195752 | 0.32254469 | -0.4994924 | 0.75497276 | 0.35112098 |
| 0.52447188 | -0.5555881 | 0.68481833 | 0.20251468 | 0.39860719 | -0.7198414 |
| 0.78773916 | 0.45518181 | 0.71273196 | -0.7655811 | 0.7155844 | 0.39701831 |
| 0.47296903 | -0.672706 | 0.69020337 | 0.37193877 | 0.47959387 | -0.9032337 |
| 0.80210346 | 0.40167108 | 0.50383294 | -0.6195157 | 0.80366057 | 0.3884458 |
| 0.45408139 | -0.7316507 | 0.48975253 | 0.47984859 | 0.33738744 | -0.5510914 |
| 0.56882453 | 0.29653791 | 0.4472059 | -0.5177853 | 0.36228263 | 0.40129057 |
| 0.4490836 | -0.4754149 | 0.46366793 | 0.31378582 | 0.48470935 | -0.2453159 |
| 0.39600489 | 0.24787127 | 0.20359448 | -0.203447 | 0.25734761 | 0.17168433 |
| 0.35209069 | -0.203685 | 0.25115264 | 0.21313109 | 0.12461348 | 0.10632347 |
| 0.13266218 | 0.20236486 | 1.1078833= | | | |
| | | | | | |
| -0.0112394 | 0.01601524 | 0.11363719 | -0.1440069 | 0.05522444 | -0.0711868 |
| 0.09505147 | -0.0220034 | 0.0714381 | -0.1994763 | 0.12304886 | -0.1611445 |
| 0.16811867 | -0.4498019 | 0.10313182 | -0.0149997 | 0.47659361 | -0.4639786 |
| -0.0380792 | -0.0468904 | 0.37975076 | -0.7120748 | -0.1078557 | 0.10635795 |
| 0.42699403 | -0.6348544 | 0.00025528 | 0.06202703 | 0.57867163 | -0.6733171 |
| -0.0381787 | 0.09532065 | 0.50065184 | -0.7413587 | -0.0193744 | -0.1180785 |
| 0.74187845 | -0.8996705 | 0.03180836 | 0.04010354 | 0.82366729 | -0.6429569 |
| 0.02410492 | -0.0632124 | 0.73732454 | -0.8188882 | 0.04538922 | -0.1471086 |
| 0.7597335 | -0.6287012 | 0.03615654 | -0.1248241 | 0.56647652 | -0.6294683 |
| 0.15992545 | -0.1780757 | 0.3820785 | -0.5642462 | -0.0609947 | -0.0350918 |
| 0.25537059 | -0.4526066 | -0.0761788 | -0.0242514 | 0.35473567 | -0.3512402 |
| -0.1888455 | 0.1974159 | 0.01620384 | -0.1306533 | -0.1468564 | 0.25235301 |
| 0.08058657 | -0.0768841 | -0.316401 | 0.09779498 | 0.08537519 | -0.0738487 |
| -0.2839164 | 0.12684187 | -0.2450078= | | | |
| | | | | | |
| -0.1147067 | -0.0084124 | -0.5239977 | -0.5021591 | 0.02636886 | 0.1470097 |
| -0.5139894 | -0.6221746 | -0.3979228 | 0.30136263 | -0.742976 | -0.4011821 |
| 0.19038832 | 0.55414283 | -1.1652025 | -0.3686967 | -0.4750175 | 0.54713631 |
| -0.9312411 | -0.410718 | -0.1498093 | 0.55332947 | -1.0870041 | -0.4378341 |
| -0.5433689 | 0.92539561 | -0.9013531 | -0.6145319 | -0.5512772 | 1.0310978 |
| -0.9422795 | -0.6914638 | -0.7839714 | 1.4393494 | -0.7092296 | -0.894987 |
| -0.6896155 | 1.1251011 | -0.8161536 | -0.8204682 | -0.8957642 | 1.3315079 |
| -1.0231192 | -0.5556009 | -0.7499282 | 1.281976 | -0.9347371 | -0.6562014 |
| -0.6568274 | 1.1967098 | -1.150661 | -0.5503616 | -0.6640182 | 0.84698498 |
| -0.7811472 | -0.5740913 | -0.4527726 | 0.64911795 | -0.6970047 | -0.5759697 |
| -0.4704399 | 0.51728982 | -0.545236 | -0.8311051 | -0.4240301 | 0.37167478 |
| -0.7735854 | -0.3031097 | -0.4083092 | -0.0152683 | -0.2330878 | -0.5839304 |
| -0 1544528 | 0.2042688 | -0.8989772 | -0.3088974 | -0.2014994 | 0.11505035 |
| -0.4815812 | -0.5319371 | -1.3798244= | | | |
| | | | | | |
| 0.07143499 | -0.1589592 | 0.04816094 | -0.0301291 | 0.15144217 | -0.3037405 |
| 0.1549352 | -0.0608833 | 0.21059546 | -0.4705076 | 0.16360784 | -0.0684895 |
| 0.44703272 | -0.6194252 | 0.19459446 | -0.0523894 | 0.31194624 | -0.8030509 |
| 0.2595928 | -0.119705 | 0.4913742 | -0.8455008 | 0.15694356 | -0.0023983 |
| 0.53066176 | -0.9705743 | 0.1324198 | 0.08982921 | 0.43900672 | -0.8588745 |
| 0.1702383 | 0.02221953 | 0.44412452 | -0.7700244 | 0.10496679 | 0.14137991 |
| 0.5403164 | -0.5077381 | 0.00849557 | 0.1611405 | 0.31764683 | -0.5240273 |
| -0.092208 | 0.21902563 | 0.25788471 | -0.3861519 | -0.2022993 | 0.13711917 |
| 0.22238699 | -0.156256 | -0.2092034 | 0.16458821 | 0.20111787 | -0.1418906 |
| -0.180493 | 0.17164391 | 0.15690604 | -0.0254563 | -0.1990184 | 0.10211211 |
| 0.17421109 | -0.0730809 | -0.3717274 | 0.1436436 | -0.0215865 | -0.2363243 |
| -0.1982318 | 0.06996673 | 0.19735655 | 0.05625506 | -0.241524 | 0.12768924 |
| 0.05979542 | -0.0623277 | -0.2521037 | 0.0944353 | -0.0492548 | 0.05238663 |
| -0.1978694 | 0.05119598 | -0.2067173= | | | |
| | | | | | |
| 0.06230025 | -0.0752745 | 0.32974288 | 0.00985043 | 0.07881941 | -0.0835249 |
| 0.1073643 | -0.090154 | -0.0938452 | 0.00704324 | 0.2569764 | 0.08700065 |
| -0.0272076 | -0.1014201 | 0.19723812 | -0.0935401 | 0.0913924 | -0.0728388 |
| 0.33091745 | -0.0610701 | 0.01335303 | 0.02156818 | 0.21619918 | -0.0909865 |
| 0.01069087 | 0.02569587 | 0.11676744 | -0.0213131 | 0.1322203 | 0.11848255 |
| 0.11231339 | -0.0392407 | 0.06117272 | -0.0234323 | 0.14693312 | 0.13509636 |
| -0.0213237 | -0.0261696 | 0.09474246 | -0.0100756 | 0.10580003 | -0.0147534 |
| 0.12980145 | -0.038394 | 0.08167668 | -0.0105376 | 0.02142166 | -0.0161705 |
| 0.15833771 | 0.01835199 | 0.04420554 | 0.02605363 | 0.27427858 | 0.05774866 |
| -0.0696303 | 0.03802699 | 0.0806741 | 0.03993953 | -0.0121658 | 0.07568218 |
| 0.05538817 | 0.01067943 | 0.04131892 | -0.0267609 | 0.14418064 | 0.0897231 |
| -0.0677462 | -0.0772208 | 0.16641215 | 0.09142463 | 0.02115551 | -0.0876383 |
| 0.14652038 | 0.06084725 | -0.1150111 | -0.0687876 | 0.10878915 | 0.32776353 |
| -0.1929855 | 0.00694158 | 0.26604816= | | | |
| | | | | | |
| -0.0786668 | 0.05454836 | -0.0834711 | 0.07707115 | 0.05659099 | -0.0285798 |
| -0.0029815 | -0.0837616 | 0.02468397 | 0.03531792 | -0.1437671 | 0.10122854 |
| -0.1259448 | -0.0845026 | 0.10171869 | -0.0541042 | 0.05257236 | 0.04065102 |
| -0.1091328 | 0.0090488 | 0.06142418 | -0.167912 | -0.098868 | 0.02574896 |
| 0.00333312 | -0.2812204 | 0.02039073 | -0.052828 | -0.0439769 | -0.0458286 |
| 0.14768517 | 0.02989549 | 0.09454407 | -0.1860176 | -0.0505908 | 0.088718 |
| 0.0611263 | -0.1895157 | 0.08583955 | 0.09382812 | -0.0001466 | -0.4065202 |
| 0.09951859 | 0.14843601 | 0.12351749 | -0.1327625 | 0.10949049 | 0.07129322 |
| 0.05554885 | -0.3743193 | -0.0205463 | 0.12675567 | 0.0775801 | -0.1869074 |
| 0.01806534 | 0.09599103 | -0.0570596 | -0.1523381 | 0.08384241 | 0.00704122 |
| 0.10942505 | -0.0473638 | 0.01151769 | 0.09737793 | 0.07082167 | -0.2184597 |
| -0.0365961 | -0.0962418 | 0.01007566 | -0.0049753 | 0.01404589 | -0.0406134 |
| 0.01934035 | -0.0073082 | -0.0489736 | 0.10457312 | -0.0520154 | -0.0454775 |
| -0.0525739 | 0.06086259 | -0.1788069= | | | |
| | | | | | |
| 0.19904579 | -0.2001437 | 0.04977471 | 0.26628217 | 0.19910193 | 0.15184447 |
| 0.01703933 | 0.06875326 | 0.09066898 | -0.2003548 | 0.26507998 | 0.0629771 |
| 0.39202845 | -0.6033413 | 0.57940209 | -0.0460919 | 0.53419203 | -0.7680888 |
| 0.65535748 | 0.32430753 | 0.64831889 | -1.0950515 | 0.80829531 | 0.05049393 |
| 0.95144385 | -1.2075449 | 0.94851351 | -0.0852669 | 0.94320357 | -1.680338 |
| 0.99852085 | 0.48870567 | 1.7470727 | -1.7586045 | 0.56886804 | 0.66196042 |
| 1.2572207 | -1.5854638 | 0.89351815 | 0.39586932 | 1.586942 | -1.6365775 |
| 0.73526824 | 0.31977594 | 1.2270083 | -1.2818555 | 0.71813524 | 0.37488377 |
| 0.95438999 | -1.2543333 | 0.55854511 | 0.1672449 | 0.56084049 | -0.7980669 |
| 0.45917389 | 0.27823627 | 0.26928344 | -0.9804664 | 0.62299174 | 0.53984308 |
| 0.33946255 | -0.5412283 | 0.1085042 | 0.44658452 | 0.39120093 | -0.5676367 |
| 0.19083619 | 0.37056214 | 0.24114503 | -0.3020035 | 0.39015424 | 0.09788869 |
| 0.30190364 | -0.3655235 | 0.33355939 | 0.44246852 | 0.17172456 | -0.3479928 |
| 0.18584418 | 0.34009755 | 4.5490937= | | | |
| | | | | | |
| 0.13698889 | -0.0798945 | 0.3366704 | 0.17313539 | 0.01228174 | -0.2679709 |
| 0.31540671 | 0.08274947 | 0.11212139 | -0.428847 | 0.57447821 | -0.0305296 |
| 0.00119518 | -0.1978176 | 0.59532708 | -0.0309942 | -0.0107875 | -0.7312108 |
| 0.74023747 | 0.38564634 | 0.03748908 | -0.6475483 | 0.87958473 | 0.05327692 |
| 0.06987014 | -0.5168169 | 1.0081589 | -0.0517421 | 0.08651814 | -0.761238 |
| 0.7840901 | 0.4372991 | 0.13783893 | -0.8574924 | 0.90612286 | 0.06334394 |
| 0.05702339 | -0.5161278 | 0.66693234 | -0.0496743 | 0.07689167 | -0.5775976 |
| 0.70519674 | 0.15731441 | 0.08724558 | -0.7325026 | 0.65517086 | 0.29064488 |
| 0.11747536 | -0.612968 | 0.98160452 | 0.02407174 | 0.02613025 | -0.677594 |
| 0.81293154 | 0.18651071 | 0.03182137 | -0.7051651 | 0.89682412 | 0.181806 |
| 0.24770954 | -0.4320194 | 0.72470272 | 0.12951751 | 0.14626819 | -0.3964331 |
| 0.54755467 | 0.08819038 | 0.22105552 | -0.3489864 | 0.4620938 | 0.06516677 |
| 0.03049339 | -0.1913544 | 0.4782092 | -0.098419 | -0.0160188 | 0.07177288 |
| 0.1008145 | 0.01412579 | 0.42727205= | | | |
| | | | | | |
| -0.0048454 | 0.1204864 | 0.15507312 | 0.25648347 | 0.03982652 | 0.14641231 |
| -0.0273505 | 0.10494121 | 0.1988914 | 0.09454013 | -0.0560908 | 0.07466536 |
| 0.1325469 | 0.15324508 | -0.01398 | 0.08281901 | 0.07909692 | 0.36858437 |
| -0.0007111 | 0.13285491 | -0.1658676 | 0.25348473 | 0.08835109 | 0.16466415 |
| -0.118853 | 0.26435438 | -0.0775707 | 0.09143513 | -0.1019902 | 0.29236633 |
| 0.07947435 | 0.07329605 | -0.0903666 | 0.10754076 | 0.04456592 | 0.18368921 |
| -0.162177 | 0.18712705 | 0.03216886 | 0.04698242 | -0.0385783 | 0.2276271 |
| 0.04106503 | 0.08498254 | -0.0325038 | 0.29328787 | 0.01249749 | 0.10016124 |
| -0.0012895 | 0.2371086 | 0.14713244 | -0.053306 | -0.0808243 | 0.28909287 |
| 0.13412228 | 0.10756335 | -0.0486093 | 0.05799349 | 0.21323961 | -0.0118695 |
| -0.142963 | 0.09792294 | 0.06907349 | 0.05942665 | -0.143813 | 0.21673524 |
| 0.19903891 | 0.02989559 | 0.15750381 | -0.0373194 | 0.12471988 | 0.10462648 |
| -0.0027455 | 0.16604523 | 0.06245366 | -0.0775013 | -0.0160873 | 0.21550164 |
| 0.25000233 | 0.05931267 | 0.22881882= | | | |
| | | | | | |
| 0.04679342 | 0.10158926 | -0.122116 | 0.23491009 | -0.0625733 | 0.19985424 |
| -0.1704439 | 0.302394 | -0.0671487 | 0.33251444 | -0.0581705 | 0.21095584 |
| -0.215752 | 0.32740423 | -0.1597161 | 0.18950906 | -0.1232446 | 0.27883759 |
| -0.0430407 | 0.04886867 | -0.0914212 | 0.28192514 | 0.05275658 | 0.21014904 |
| -0.1322077 | 0.2981362 | 0.1254565 | 0.15627012 | 0.04116358 | 0.08507752 |
| 0.10109599 | 0.23081669 | -0.1617257 | 0.29508773 | -0.0405337 | -0.0497829 |
| -0.0808031 | 0.15750171 | 0.08072432 | 0.12990661 | -0.1935954 | 0.29120663 |
| 0.13912162 | 0.04256131 | -0.1625126 | 0.25232118 | 0.04736055 | -0.0530935 |
| -0.2270383 | 0.22945035 | 0.18167619 | 0.00080986 | -0.1253632 | 0.15695702 |
| 0.01596376 | 0.03504543 | 0.00964208 | 0.11757879 | -0.0230768 | 0.04350457 |
| -0.1284984 | 0.24145114 | 0.20540115 | 0.07580803 | -0.0932236 | 0.14288881 |
| 0.00538179 | 0.05302088 | -0.1001294 | 0.27505419 | 0.22654785 | 0.02395938 |
| -0.0861699 | 0.05814215 | 0.21307872 | 0.01372274 | 0.04515802 | -0.0269269 |
| 0.20031671 | 0.23140682 | 0.16010799= | | | |
| | | | | | |
| 0.37838998 | 0.00934576 | -0.139213 | 0.29823828 | 0.40640026 | -0.067578 |
| -0.038453 | 0.24550894 | 0.30729383 | -0.2807365 | -0.0689575 | 0.26537073 |
| 0.58336282 | -0.2145292 | -0.2378269 | 0.25939462 | 0.64761585 | -0.3581158 |
| 0.07741276 | 0.45081589 | 0.65251595 | -0.4543131 | -0.0671543 | 0.48592216 |
| 0.85640681 | -0.6068144 | -0.1187844 | 0.35959438 | 0.71842372 | -0.7140775 |
| -0.0642752 | 0.37914035 | 0.71409059 | -0.7180941 | 0.21169594 | 0.27888221 |
| 0.79736245 | -0.7102081 | 0.14268413 | 0.41374633 | 0.75569016 | -0.7394939 |
| 0.02592243 | 0.37013471 | 0.82774776 | -0.8136597 | 0.24068722 | 0.45081198 |
| 0.88004726 | -0.6990998 | 0.23456772 | 0.24596012 | 0.67229778 | -0.8148533 |
| 0.30492786 | 0.39735735 | 0.55497372 | -0.6593497 | 0.20656242 | 0.3752968 |
| 0.54989374 | -0.5660355 | 0.1205707 | 0.22377795 | 0.46045718 | -0.519361 |
| 0.17151839 | 0.39539635 | 0.50465524 | -0.3791285 | 0.07184427 | 0.36315975 |
| 0.51068121 | -0.3502096 | -0.2094818 | 0.31471297 | 0.18174268 | -0.1241962 |
| -0.1255455 | 0.35898197 | 0.79502285= | | | |
| | | | | | |
| 0.02952595 | -0.0751979 | -0.2556099 | -0 3040917 | -0.0942183 | -0.0541431 |
| -0.6262965 | -0.1423945 | -0.0537339 | 0.11189342 | -0.3791296 | -0.3382006 |
| 0.02978903 | 0.20563391 | -0.5457558 | -0.3666513 | -0.1922515 | 0.29512301 |
| -0.7473708 | -0.0415357 | 0.18283925 | 0.28153449 | -0.7847292 | -0.2313099 |
| 0.00290797 | 0.6284017 | -0.6397845 | -0.5606785 | -0.1479581 | 0.57049137 |
| -1.0829539 | -0.1822221 | -0.1832336 | 0.49371469 | -0.6362705 | -0.2790937 |
| 0.06966544 | 0.75524592 | -0.9053063 | -0.5826979 | -0.114608 | 0.90401584 |
| -0.8823278 | -0.3404879 | -0.0334436 | 0.50130409 | -0.57275 | -0.3842527 |
| 0.0915129 | 0.44590429 | -0.7808504 | -0.4399623 | -0.1189605 | 0.59226018 |
| -0.499517 | -0.4873153 | -0.2889721 | 0.47303999 | -0.4015501 | -0.2875251 |
| -0.1106236 | 0.27437851 | -0.6061368 | -0.4166524 | -0.0637606 | 0.33875695 |
| -0.6255118 | -0.1046614 | -0.2710638 | 0.26425925 | -0.4123208 | -0.2157291 |
| -0.1468192 | -0.1719856 | -0.4140109 | -0.1058299 | 0.02873472 | -0.1210428 |
| -0.213571 | -0.1335077 | -0.7155944= | | | |
| | | | | | |
| 0.06424081 | -0.0978306 | -0.1169782 | 0.13909493 | -0.0838893 | -0.1300299 |
| -0.1032737 | 0.11563963 | -0.0709175 | -0.028875 | -0.1718288 | -0.026291 |
| 0.05533361 | -0.033985 | -0.049436 | 0.11520655 | -0.0279296 | -0.0170352 |
| 0.05850215 | 0.03830531 | -0.0893732 | -0.0066427 | 0.06969514 | 0.13403182 |
| -0.012636 | -0.1925185 | 0.13028348 | -0.0045112 | 0.05260766 | -0.2759708 |
| -0.0395793 | 0.03069885 | 0.07913893 | -0.1470363 | 0.09080192 | 0.19741131 |
| -0.0917266 | -0.2185763 | 0.04743406 | -0.0364127 | 0.00991712 | -0.2093729 |
| 0.23327024 | -0.0898143 | -0.0578982 | -0.2096201 | 0.09257686 | 0.00566842 |
| 0.10926479 | -0.1167006 | 0.18223672 | 0.09710353 | 0.03838636 | -0.2026017 |
| 0.12219627 | 0.05705986 | -0.0505442 | -0.1334345 | -0.0204458 | 0.01167099 |
| -0.1091286 | -0.075133 | 0.02949276 | -0.0217044 | -0.0782921 | -0.1160332 |
| -0.0210903 | 0.11607172 | -0.0943146 | -0.1014408 | 0.02903902 | 0.02963065 |
| -0.1233738 | -0.0760847 | 0.00098273 | 0.07522969 | 0.05794976 | -0.1959872 |
| 0.06584878 | -0.0323083 | -0.0581293= | | | |

**Table 4.**

| Second neural net weighting matrix (2 x 21) (weights_2) | | | | | |
|---|---|---|---|---|---|
| -0.5675537 | -0.6119734 | 0.20069507 | 0.26132998 | -0.5071653 | 0.2793434 |
| -0.5328685 | 0.31165671 | -0.9999997 | -0.4128213 | -1.0000007 | -0.6456627 |
| -0.209518 | 1.6362301 | -1.9999975 | -0.2563241 | 0.04389827 | 1.7597554 |
| 2.0453076 | 0.08412334 | -0.1645829= | | | |
| | | | | | |
| 0.55343837 | 0.68506879 | -1.1869608 | 0.39551663 | 0.38050765 | 0.40832204 |
| 0.12712023 | -1.7462951 | 0.0818732 | 6.111361 | 0.62210494 | 0.42921746 |
| 0.19891988 | -4.0000067 | -0.5605077 | 1.3601962 | 1.7318885 | -1.0558798 |
| 3.1242371 | 0.22860088 | 1.6726165= | | | |

### E) Code for running the net

Code for running the neural net is provided below in Table 5 (neural_n c) and Table 6 (lin_alg.c).

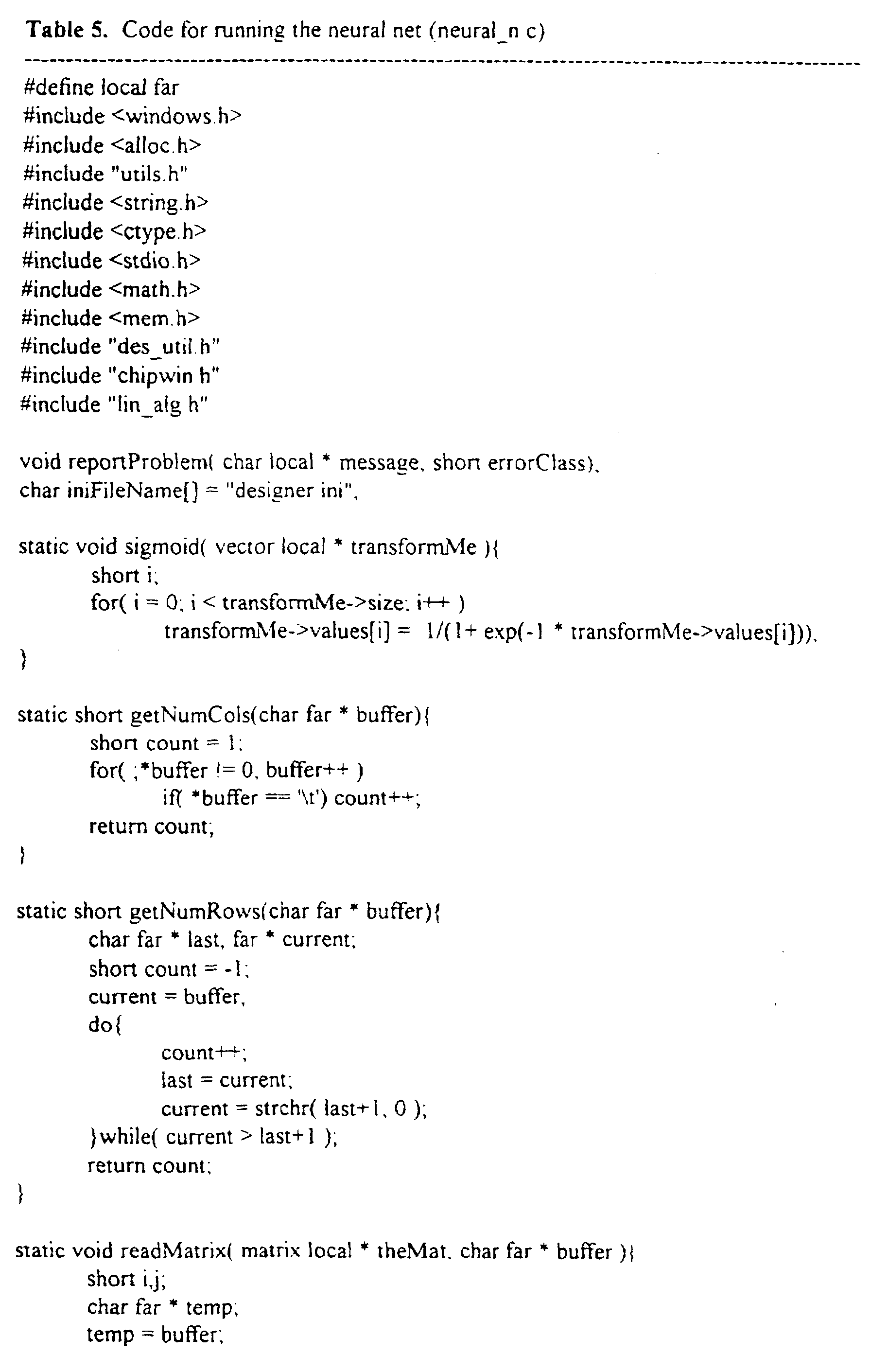

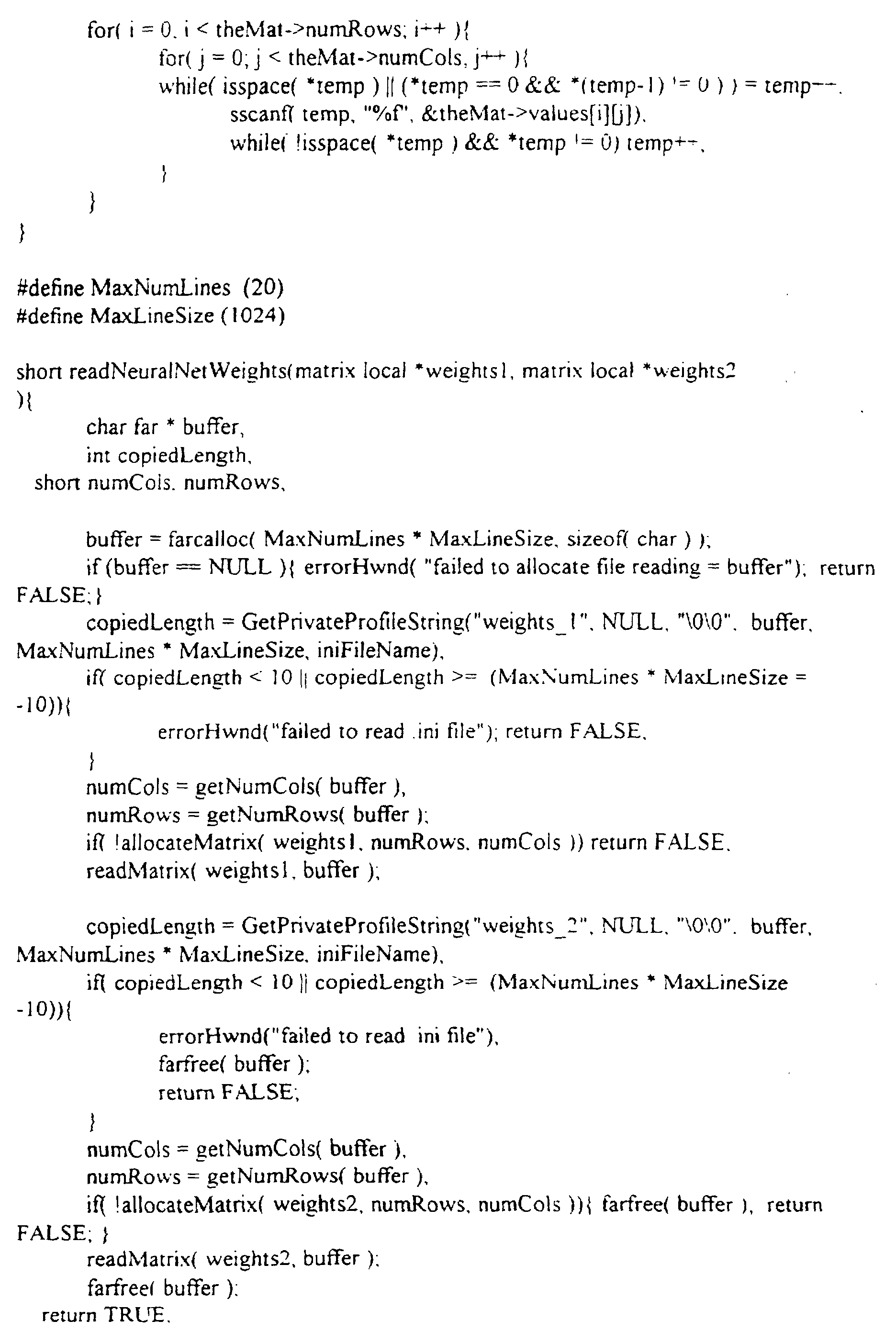

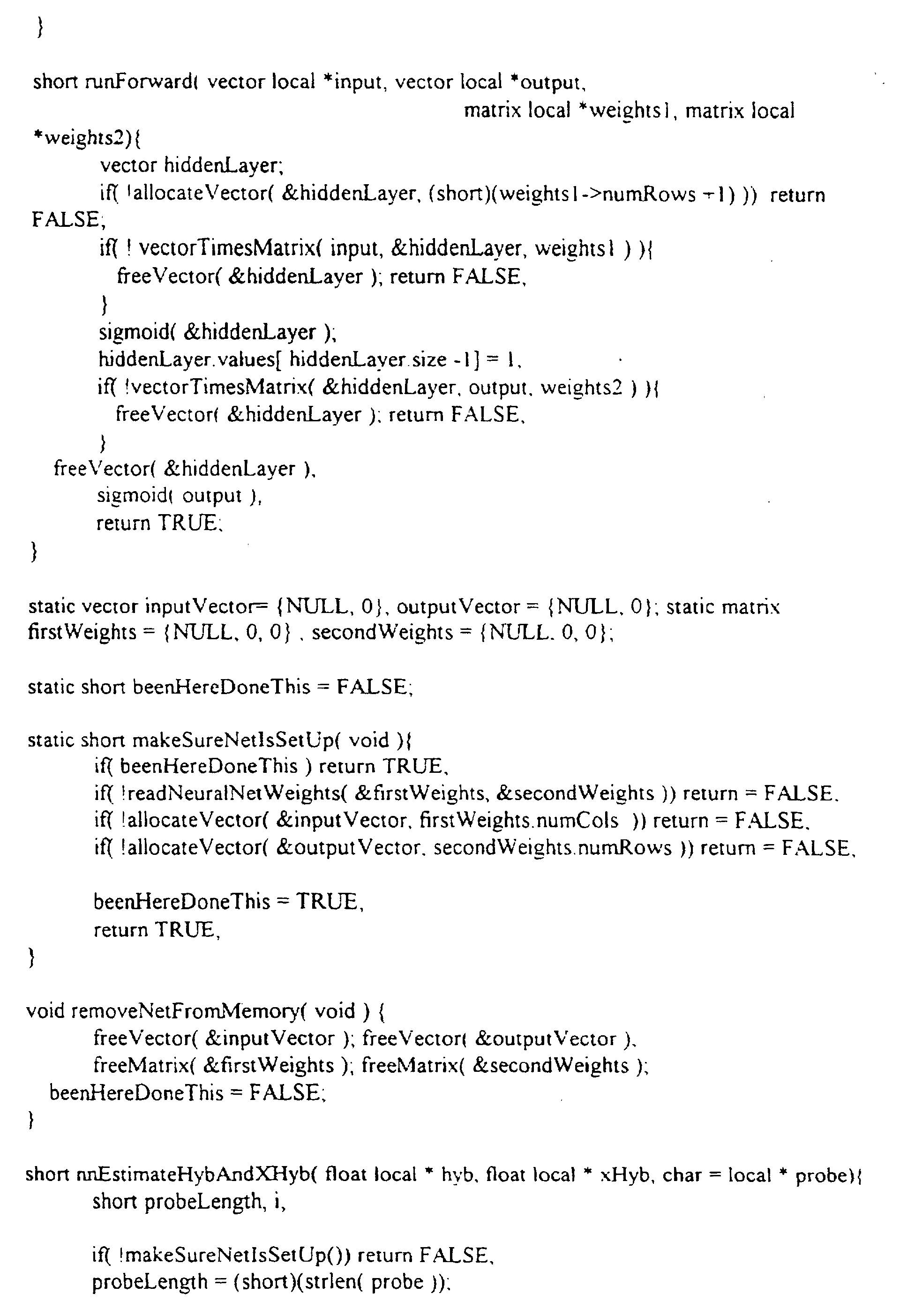

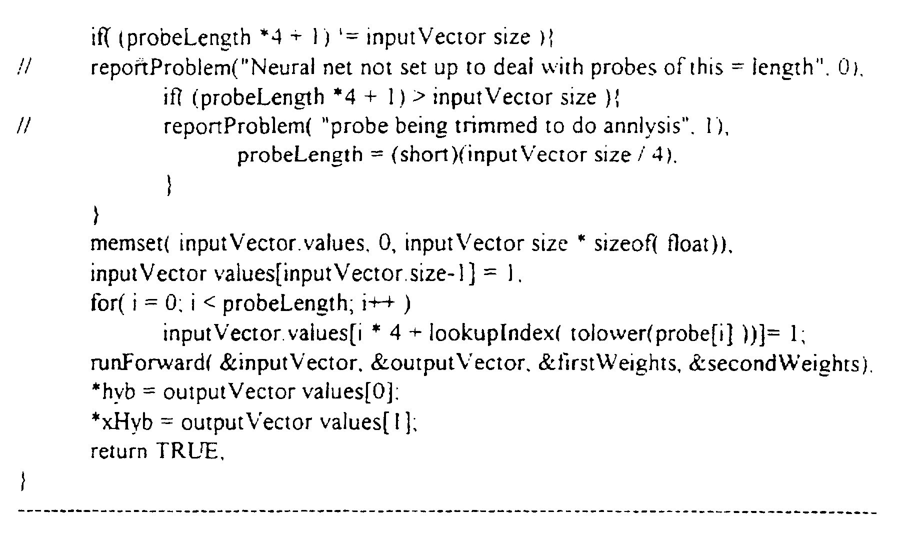

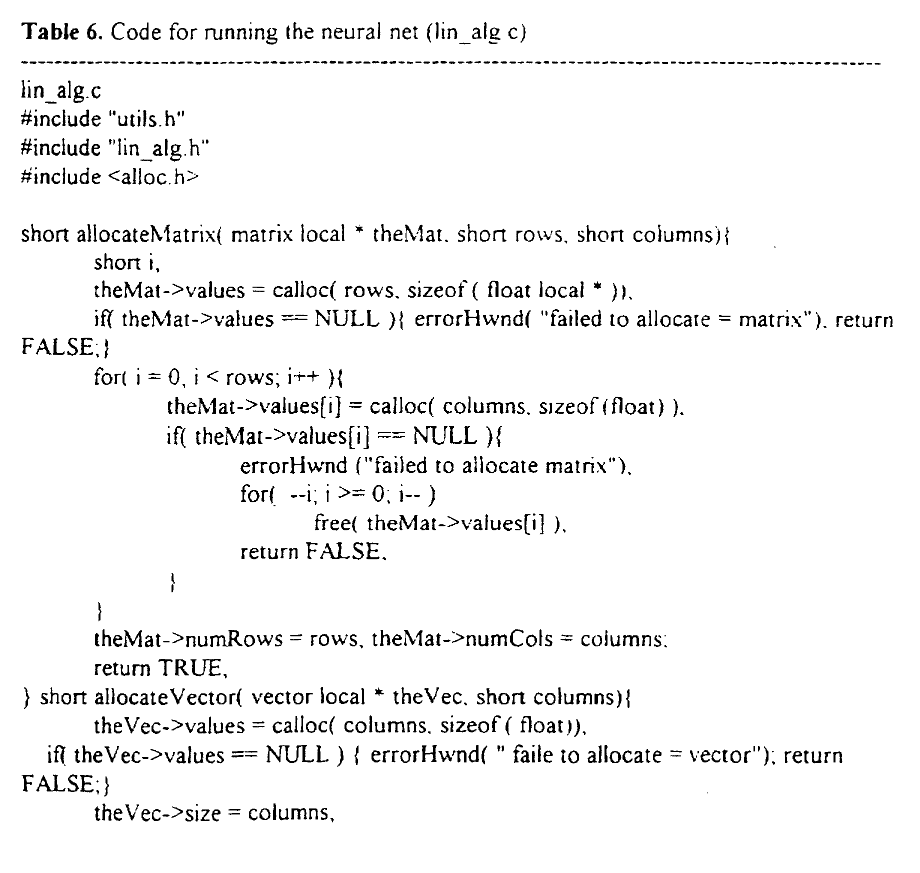

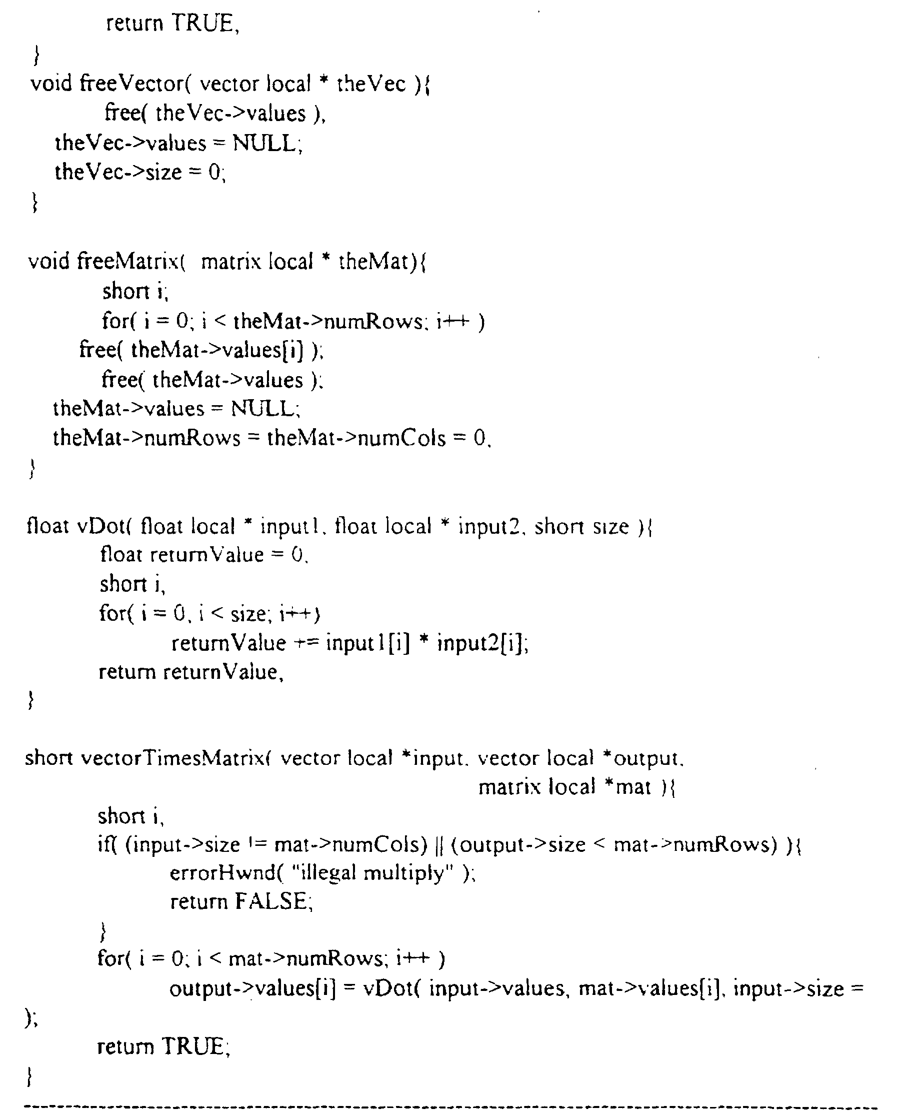

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference for all purposes.

## Claims

1. A method of simultaneously monitoring the expression of a multiplicity of genes, said method comprising:
(a) providing a pool of target nucleic acids comprising RNA transcripts of some of said genes, or nucleic acids derived from said RNA transcripts;
(b) providing a plurality of different probes for analysis of each of the RNA transcripts that are to be monitored; said probes being immobilized as an array on a surface of a substrate in known locations at a density greater than 60 different probes per cm²; said array probes including match and control probes; the array comprising more than 100 different probes, each probe attached to the surface through a single covalent bond;
(c) hybridizing said pool of nucleic acids to the array of nucleic acid probes; and
(d) quantifying hybridization of said target nucleic acids to said array by comparing hybridisation of match and control probes wherein said quantifying provides a measure of the levels of transcription of said genes.

2. A method of claim 1, wherein each of said nucleic acid probes is chemically synthesized or synthesized by light-directed polymer synthesis, or wherein preparation of said nucleic acid probes does not require cloning, a nucleic acid amplification step, or enzymatic synthesis and/or does not require handling of any biological materials.

3. A method of claim 1 or claim 2, wherein for each gene, said array comprises at least 10 different nucleic acid probes complementary to subsequences of that gene, preferably no more than 20 different nucleic acid probes complementary to subsequences of that gene.

4. A method of any one of claims 1 to 3, wherein said nucleic acid probes are from 5 to 45 nucleotides in length, preferably from 20 to 25 nucleotides in length.

5. A method of any one of claims 1 to 4, wherein said array comprises nucleic acid probe sequences from constitutively expressed control genes, optionally said control genes being selected from β-actin, GAPDH, and the transferrin receptor.

6. A method of any one of claims 1 to 5, wherein the variation between different copies of each array is less than 20% wherein said variation is measured as the coefficient of variation in hybridization intensity averaged over at least 5 nucleic acid probes for each gene whose expression the array is to detect.

7. A method of any one of claims 1 to 6, wherein the concentration of nucleic acids in said pool is proportional to the expression levels of said genes.

8. A method of any one of claims 1 to 7, wherein the control nucleic acid probes comprise mismatch control probes such that for each matched probe there exists a mismatch control probe.

9. A method of claim 8, wherein said quantifying comprises either:-
(a) calculating the difference in hybridization signal intensity between each of said nucleic acid probes and its corresponding mismatch control probe; or
(b) calculating the average difference in hybridization signal intensity between each of said nucleic acid probes and its corresponding mismatch control probe for each gene.

10. A method of any of claims 1 to 9, wherein the nucleic acid probes in said array are selected according to any one of claims 26 to 34.

11. A method of any one of claims 1 to 9 wherein the nucleic acid probes in said array are selected according to any one of claims 38 to 42.

12. A method of any one of claims 1 to 11, wherein hybridization and quantification is accomplished in under 48 hours.

13. A method of any one of claims 1 to 12, wherein said hybridization is performed with a fluid volume of 250 µl or less and/or wherein said hybridization comprises a hybridization at low stringency of 30°C to 50°C and 6 X SSPE-T or lower and a wash at higher stringency.

14. A method of any one of claims 1 to 13, wherein said quantifying comprises either:
(a) detecting a hybridization signal that is proportional to the concentration of said RNA in said nucleic acid sample; or
(b) detecting a hybridization signal that is proportional to the concentration of said target nucleic acids for each gene in said pool of target nucleic acids.

15. A method of any one of claims 1 to 14, wherein said pool of nucleic acids is a pool of mRNAs or a pool of RNAs *in vitro* transcribed from a pool of cDNAs.

16. A method of any one of claims 1 to 15, wherein said pool of nucleic acids is amplified from a biological sample.

17. A method of any one of claims 1 to 16, wherein said pool of nucleic acids comprises fluorescently labeled nucleic acids or wherein said pool of target nucleic acids is labeled with a single species of fluorophore.

18. A method of claim 17 which comprises quantifying fluorescence of a label on said hybridized nucleic acids at a spatial resolution of 100 µm or higher, e.g. by means of a scanning confocal fluorescence microscope.

19. A method of any one of claims 1 to 18, wherein said providing a plurality of different probes comprises either:-
(i)
(a) hybridizing a pool of RNAs with a pool of further nucleic acid probes comprising at least some of the match probes to form a pool of hybridized nucleic acids;
(b) treating said pool of hybridized nucleic acids with RNase A, thereby digesting single stranded nucleic acid sequences and leaving intact the hybridized double stranded regions;
(c) denaturing the hybridized double-stranded regions and removing said further nucleic acid probes thereby leaving a pool of RNAs enhanced for those RNAs complementary to the match nucleic acid probes in said array; or
(ii)
(a) hybridizing a pool of RNAs with paired target specific nucleic acid probes where said paired target specific nucleic acid probes are complementary to regions flanking subsequences complementary to said match nucleic acid probes in said array;
(b) treating said pool of nucleic acids with RNase H to digest the hybridized (double stranded) nucleic acid sequences;
(c) isolating the remaining nucleic acid sequences having a length about equivalent to the region flanked by said paired target specific nucleic acid probes; or
(iii)
(a) hybridizing a pool of polyA⁺ mRNAs with nucleic acid probes that hybridize specifically with particular preselected mRNA target messages;
(b) treating said pool of nucleic acids with RNase H to digest the hybridized (double stranded) nucleic acid sequences thereby separating the coding sequence from the polyA⁺ tail;
(c) isolating or amplifying the remaining polyA⁺ RNA in said pool.

20. A method of any one of claims 1 to 19, wherein the probes of the array comprise probes selected to check for splice variant transcripts of a gene.

21. A method of any one of claims 1 to 3 or 5 to 20, wherein the probes are up to 500 bases long.

22. A composition to indicate the expression levels of a multiplicity of genes, said composition comprising an array of a plurality of different probes for each RNA transcript to be analyzed; said probes being immobilized as an array on a surface of a substrate in known locations at a density greater than 60 different probes per cm²; said array probes including match and control probes; the array comprising more than 100 different probes, each probe attached to the surface through a single covalent bond;
and said nucleic acid probes being specifically hybridizable to fluorescently labeled nucleic acids and chosen such that the amount of fluorescence thereby hybridized to said array is indicative of the amount of said RNA transcripts, optionally wherein said fluorescence intensity is proportional to the transcription levels of said multiplicity of preselected genes in a biological sample.

23. A composition of claim 22 and further defined by the specific feature(s) of any one or more of claims 2 to 5, 8, 16, 20 or 21.

24. A kit for the detection of expression levels of a multiplicity of genes, said kit comprising:
a selected plurality of different match and control probes for each RNA transcript that is to be monitored; the selected match and control probes being immobilized as an array on a surface of a substrate in known locations the array comprising more than 100 different probes at a density greater than 60 different probes per cm², each probe attached to the surface through a single covalent bond; and
optionally, instructions describing the use of said array for the quantification of expression levels of said multiplicity of genes;
optionally, wherein said control probes are mismatch probes, there being a corresponding mismatch probe for each match probe.

25. A kit of claim 24, further comprising fluorescent label for labeling RNA or DNA that is to be hybridized to the nucleic acids of said array and/or buffers and reagents for the hybridization of RNA to the nucleic acid probes of said array.

26. A method of selecting a set of probes and immobilizing the probes to a surface of a substrate as an array for monitoring the expression of RNA transcripts or nucleic acids derived therefrom from a plurality of target genes comprising:
(a) providing an array of nucleic acid probes said array comprising a multiplicity of nucleic acid probes, wherein each probe is complementary to a subsequence of said target nucleic acids and for each probe there is a corresponding mismatch control probe, e.g. wherein said mismatch control probes have a 1 base mismatch;
(b) hybridizing said target nucleic acids to said array of nucleic acid probes;
(c) selecting those probes where the difference in hybridization signal intensity between each probe and its mismatch control is detectable, preferably, wherein said difference in hybridization intensity is at least 10% of the background signal; and
(d) immobilizing a plurality of the selected probes for each of the target nucleic acids to be analysed together with control probes to the surface of a substrate to allow quantification of the target nucleic acids, wherein the array is as defined in claim 22.

27. A method of claim 26, further comprising, between steps (c) and (d), hybridizing said array to a pool of nucleic acids comprising nucleic acids other than said target nucleic acids; and selecting probes having the lowest hybridization signal and where both the probe and its mismatch control have a hybridization intensity equal to or less than 10 times background.

28. A method of claim 26 or claim 27, wherein said multiplicity of probes includes all the probes of a single length that are complementary to a subsequence of said target nucleic acid where said probes have a length between about 5 and 50 nucleotides.

29. A method of any of claims 26 to 28, wherein said nucleic acid probes range in length from about 5 to about 45 nucleotides.

30. A method of any one of claims 26 to 29, wherein said nucleic acid probes are all the same length.

31. A method of any one of claims 26 to 30, wherein said array comprises more than 1000 different nucleic acid probes wherein each different nucleic acid probe is localized in a known location of said surface and the density of said different nucleic acid probes is greater than 60 different nucleic acid probes per 1 cm² of said surface.

32. A method of any one of claims 26 to 31, wherein said nucleic acid probes are synthesized by light-directed synthesis.

33. A method of any one of claims 26 to 32, wherein said hybridization comprises hybridization at low stringency of 30°C to 50°C and 6 X SSPE-T or lower followed by one or more washes at progressively increasing stringency until a desired level of hybridization specificity is obtained.

34. A method of any one of claims 26 to 33, wherein said pool of nucleic acids comprising nucleic acids other than said target nucleic acids comprises nucleic acids having a sense opposite that of the target nucleic acids.

35. A method of claim 1, wherein the control nucleic acid probes comprise mismatch probes, and the quantifying step is performed in a computer system by the steps of:
receiving input of hybridization intensities for the plurality of nucleic acid probes including pairs of the match probes and mismatch probes, the hybridization intensities indicating hybridization affinity between the plurality of nucleic acid probes and the pool of nucleic acids, and each pair including a match probe that is perfectly complementary to a portion of the nucleic acids and a mismatch probe that differs from the match probe by at least one nucleotide;
comparing the hybridization intensities of the match and mismatch probes of each pair; and
indicating expression of one or more of the genes in the pool according to results of the comparing step.

36. A method of claim 35, wherein the comparing step includes either:
(a) calculating differences between the hybridization intensities of the match and mismatch probes of each pair, optionally including calculating an average of the differences; or
(b) determining if a difference between the match and mismatch probes or each pair crosses a difference threshold; or
(c) determining if a quotient of the match and mismatch probes of each pair crosses a ratio threshold; or
(d) determining a first number of pairs that have a difference that crosses a difference threshold and a quotient that crosses a ratio threshold; preferably further including determining a second number of pairs that have a difference that does not cross the difference threshold and a quotient that does not cross the ratio threshold.

37. A method of claim 35 or claim 36, wherein the indicating step indicates the gene is expressed if a quotient of the first and the second numbers crosses an expression threshold.

38. A method of selecting probes and immobilizing the probes to a substrate as an array for use in expression monitoring of RNA transcripts or nucleic acids derived therefrom from a plurality of genes, comprising:
(i) in a computer system:
(a) receiving input of a nucleic acid sequence of one of the plurality of genes;
(b) generating a set of probes that are perfectly complementary to the gene; and
(c) identifying a subset of probes, including less than all of the probes in the set, for monitoring the expression of the gene;
(d) repeating (a), (b) and (c) for at least one further gene to identify at least one further subset of probes;
(ii) immobilizing the subsets of probes together with control probes in an array on the surface of a substrate to allow quantification of the transcripts of the plurality of genes, wherein the array is as defined in claim 22.

39. A method of claim 38, wherein the identifying step includes the step of analyzing each probe of the set by criteria that specify characteristics indicative of low hybridization or high cross hybridization; preferably, wherein each of the criteria includes a threshold value such that if a selected probe has a characteristic that crosses the threshold value, low hybridization or high cross hybridization are indicated for the selected probe, and, if desired, further comprising increasing at least one threshold value to increase the probes in the subset.

40. A method of claim 39, further comprising the step of determining the criteria as heuristic rules derived from multiple experiments.

41. A method of claim 39 or claim 40, wherein one of the criteria indicates low hybridization or cross hybridization if either:
(a) occurrences of a specific nucleotide in a probe cross a threshold value; or
(b) the number of a specific nucleotide that repeats sequentially in a probe crosses a threshold value; or
(c) a length of a palindrome in a probe crosses a threshold value; or
(d) a length of a subsequence within a probe that includes only two specific nucleotides crosses a threshold value.

42. A method of any one of claims 38 to 41, wherein the identifying step is performed by a neural network that receives as input the probes of the set and outputs the probes of the subset.

## Patentansprüche

1. Verfahren zum gleichzeitigen Überwachen der Expression einer Vielfalt von Genen, wobei das Verfahren folgendes umfasst:
(a) Bereitstellen eines Pools von Zielnukleinsäuren, umfassend RNA-Transkripte von einigen der Gene oder Nukleinsäuren, die von den RNA-Transkripten hergeleitet sind;
(b) Bereitstellen einer großen Anzahl von verschiedenen Sonden für die Analyse von jedem der RNA-Transkripte, die überwacht werden sollen; wobei die Sonden als eine Anordnung auf einer Oberfläche eines Substrats an bekannten Stellen mit einer Dichte von mehr als 60 verschiedenen Sonden pro cm² immobilisiert sind; wobei die angeordneten Sonden Paarungs- und Kontrollsonden umfassen; wobei die Anordnung mehr als 100 verschiedene Sonden aufweist und jede Sonde über eine einzige kovalente Bindung an die Oberfläche gebunden ist;
(c) Hybridisieren des Pools von Nukleinsäuren mit einer Anordnung von Nukleinsäuresonden; und
(d) quantitatives Bestimmen der Hybridisierung der Zielnukleinsäuren mit der Anordnung durch Vergleichen der Hybridisierung von Paarungs- und Kontrollsonden, wobei durch dieses quantitative Bestimmen ein Maß für die Transkriptionsraten der Gene bereitgestellt wird.

2. Verfahren nach Anspruch 1, wobei jede der Nukleinsäuresonden chemisch synthetisiert wird oder durch lichtgesteuerte Polymersynthese synthetisiert wird, oder wobei für die Herstellung der Nukleinsäuresonden kein Clonieren, kein Nukleinsäure-Amplifikationsschritt oder keine enzymatische Synthese erforderlich ist und/oder keine biologischen Stoffe bearbeitet werden müssen.

3. Verfahren nach Anspruch 1 oder Anspruch 2, wobei die Anordnung für jedes Gen mindestens zehn verschiedene Nukleinsäuresonden, die zu Subsequenzen des Gens komplementär sind, und vorzugsweise nicht mehr als 20 verschiedene Nukleinsäuresonden umfasst, die zu Subsequenzen des Gens komplementär sind.

4. Verfahren nach einem der Ansprüche 1 bis 3, wobei die Nukleinsäuresonden eine Länge von 5 bis 45 Nukleotiden, vorzugsweise eine Länge von 20 bis 25 Nukleotiden aufweisen.

5. Verfahren nach einem der Ansprüche 1 bis 4, wobei die Anordnung Nukleinsäuresonden-Sequenzen aus konstitutiv exprimierten Kontrollgenen umfasst, wobei die Kontrollgene gegebenenfalls ausgewählt sind aus β-Aktin, GAPDH und dem Transferrin-Rezeptor.

6. Verfahren nach einem der Ansprüche 1 bis 5, wobei die Variation zwischen verschiedenen Kopien jeder Anordnung weniger als 20 % beträgt, wobei die Variation als Variationskoeffizient der Hybridisierungsintensität gemessen wird, und zwar als Durchschnitt von mindestens fünf Nukleinsäuresonden für jedes Gen, dessen Expression mit der Anordnung nachgewiesen werden soll.

7. Verfahren nach einem der Ansprüche 1 bis 6, wobei die Konzentration von Nukleinsäuren in dem Pool zu den Expressionsraten der Gene proportional ist.

8. Verfahren nach einem der Ansprüche 1 bis 7, wobei die Kontroll-Nukleinsäuresonden Fehlpaarungs-Kontrollsonden umfassen, so dass für jede gepaarte Sonde eine Fehlpaarungs-Kontrollsonde vorliegt.

9. Verfahren nach Anspruch 8, wobei das quantitative Bestimmen folgendes umfasst: entweder
(a) Berechnen des Unterschieds in der Hybridisierungssignal-Intensität zwischen jeder der Nukleinsäuresonden und ihrer entsprechenden Fehlpaarungs-Kontrollsonde; oder
(b) Berechnen des durchschnittlichen Unterschieds in der Hybridisierungssignal-Intensität zwischen jeder der Nukleinsäuresonden und ihrer entsprechenden Fehlpaarungs-Kontrollsonde für jedes Gen.

10. Verfahren nach einem der Ansprüche 1 bis 9, wobei die Nukleinsäuresonden in der Anordnung nach einem der Ansprüche 26 bis 34 ausgewählt sind.

11. Verfahren nach einem der Ansprüche 1 bis 9, wobei die Nukleinsäuresonden in der Anordnung nach einem der Ansprüche 38 bis 42 ausgewählt sind.

12. Verfahren nach einem der Ansprüche 1 bis 11, wobei die Hybridisierung und quantitative Bestimmung in weniger als 48 Stunden durchgeführt werden.

13. Verfahren nach einem der Ansprüche 1 bis 12, wobei die Hybridisierung mit einem Flüssigkeitsvolumen von 250 µl oder weniger durchgeführt wird und/oder wobei die Hybridisierung eine Hybridisierung bei einer niedrigen Stringenz von 30°C bis 50°C und 6 X SSPE-T oder niedriger und einen Waschgang bei einer höheren Stringenz umfasst.

14. Verfahren nach einem der Ansprüche 1 bis 13, wobei das quantitative Bestimmen folgendes umfasst: entweder
(a) Nachweisen eines Hybridisierungssignals, das zur Konzentration der RNA in der Nukteinsäureprobe proportional ist; oder
(b) Nachweisen eines Hybridisierungssignals, das zur Konzentration der Zielnukleinsäuren für jedes Gen in dem Pool von Zielnukleinsäuren proportional ist.

15. Verfahren nach einem der Ansprüche 1 bis 14, wobei der Pool von Nukleinsäuren ein Pool von mRNAs oder ein Pool von RNAs, *in vitro* transkribiert von einem Pool von cDNAs, ist.

16. Verfahren nach einem der Ansprüche 1 bis 15, wobei der Pool von Nukleinsäuren aus einer biologischen Probe amplifiziert ist.

17. Verfahren nach einem der Ansprüche 1 bis 16, wobei der Pool von Nukleinsäuren fluoreszierend markierte Nukleinsäuren umfasst, oder wobei der Pool von Zielnukleinsäuren mit einer einzigen Fluorophorenart markiert ist.

18. Verfahren nach Anspruch 17, umfassend ein quantitatives Bestimmen der Fluoreszenz einer Markierung auf den hybridisierten Nukleinsäuren bei einem räumlichen Auflösungsvermögen von 100 µm oder höher, z.B. anhand eines konfokalen Fluoreszenz-Rastermikroskops.

19. Verfahren nach einem der Ansprüche 1 bis 18, wobei das Bereitstellen einer großen Anzahl von verschiedenen Sonden folgendes umfasst: entweder
(i)
(a) Hybridisieren eines Pools von RNAs mit einem Pool von weiteren Nukleinsäuresonden, umfassend mindestens einige der Paarungssonden, wodurch ein Pool von hybridisierten Nukleinsäuren gebildet wird;
(b) Behandeln des Pools von hybridisierten Nukleinsäuren mit RNase A, wodurch einzelsträngige Nukleinsäuresequenzen gespalten werden und die hybridisierten doppelsträngigen Regionen intakt bleiben;
(c) Denaturieren der hybridisierten doppelsträngigen Regionen und Entfernen der weiteren Nukleinsäuresonden, wodurch ein Pool von RNAs zurückbleibt, die mit denjenigen RNAs angereichert sind, die zu den Paarungs-Nukleinsäuresonden in der Anordnung komplementär sind; oder
(ii)
(a) Hybridisieren eines Pools von RNAs mit gepaarten Ziel-spezifischen Nukleinsäuresonden, wobei die gepaarten Ziel-spezifischen Nukleinsäuresonden zu Regionen komplementär sind, die Subsequenzen flankieren, welche zu den Paarungs-Nukleinsäuresonden in der Anordnung komplementär sind;
(b) Behandeln des Pools von Nukleinsäuren mit RNase H, wodurch die hybridisierten (doppelsträngigen) Nukleinsäuresequenzen gespalten werden;
(c) Isolieren der restlichen Nukleinsäuresequenzen, die eine Länge aufweisen, die etwa gleich ist wie die der Region, die durch die gepaarten Ziel-spezifischen Nukleinsäuresonden flankiert ist; oder
(iii)
(a) Hybridisieren eines Pools von PolyA⁺-mRNAs mit Nukleinsäuresonden, die mit bestimmten vorgewählten mRNA-Ziel-Informationen spezifisch hybridisieren;
(b) Behandeln des Pools von Nukleinsäuren mit RNase H, so dass die hybridisierten (doppelsträngigen) Nukleinsäuresequenzen gespalten werden, wodurch die codierende Sequenz vom PolyA⁺-Schwanz abgetrennt wird;
(c) Isolieren oder Amplifizieren der restlichen PolyA⁺-RNA-in dem Pool.

20. Verfahren nach einem der Ansprüche 1 bis 19, wobei die Sonden der Anordnung Sonden umfassen, die so ausgewählt sind, dass damit nach Spleißvarianten-Transkripten eines Gens gesucht werden kann.

21. Verfahren nach einem der Ansprüche 1 bis 3 oder 5 bis 20, wobei die Sonden bis zu 500 Basen lang sind.

22. Zusammensetzung zum Anzeigen der Expressionsraten einer Vielfalt von Genen, wobei die Zusammensetzung für jedes zu analysierende RNA-Transkript eine Anordnung von einer großen Anzahl verschiedener Sonden umfasst; wobei die Sonden als eine Anordnung auf einer Oberfläche eines Substrats an bekannten Stellen mit einer Dichte von mehr als 60 verschiedenen Sonden pro cm² immobilisiert sind; wobei die angeordneten Sonden Paarungs- und Kontrollsonden umfassen; wobei die Anordnung mehr als 100 verschiedene Sonden aufweist und jede Sonde über eine einzige kovalente Bindung an die Oberfläche gebunden ist;
und wobei die Nukleinsäuresonden mit fluoreszierend markierten Nukleinsäuren spezifisch hybridisierbar sind und so ausgewählt sind, dass die Stärke der Fluoreszenz, die auf diese Weise mit der Anordnung hybridisiert ist, die Menge der RNA-Transkripte anzeigt, wobei gegebenenfalls die Fluoreszenzintensität proportional ist zu den Transkriptionsraten einer Vielfalt von vorgewählten Genen in einer biologischen Probe.

23. Zusammensetzung nach Anspruch 22 und weiterhin definiert durch das (die) spezifische(n) Merkmal(e) nach einem oder mehreren der Ansprüche 2 bis 5, 8, 16, 20 oder 21.

24. Kit zum Nachweisen von Expressionsraten einer Vielfalt von Genen, wobei das Kit folgendes umfasst:
eine gewählte große Anzahl von verschiedenen Paarungs- und Kontrollsonden für jedes RNA-Transkript, das überwacht werden soll; wobei die gewählten Paarungs- und Kontrollsonden als eine Anordnung auf einer Oberfläche eines Substrats an bekannten Stellen immobilisiert sind, wobei die Anordnung mehr als 100 verschiedene Sonden mit einer Dichte von mehr als 60 verschiedenen Sonden pro cm² umfasst, wobei jede Sonde über eine einzige kovalente Bindung an die Oberfläche gebunden ist; und
gegebenenfalls Anleitungen; in denen die Verwendung der Anordnung zum quantitativen Bestimmen von Expressionsraten der Vielfalt von Genen beschrieben wird;
wobei gegebenenfalls die Kontrollsonden Fehlpaarungssonden sind, so dass für jede Paarungssonde eine entsprechende Fehlpaarungssonde vorliegt.

25. Kit nach Anspruch 24, umfassend weiterhin eine fluoreszierende Markierung zur Markierung einer RNA oder DNA, die mit den Nukleinsäuren der Anordnung hybridisiert werden soll, und/oder Puffer und Reagenzien für die Hybridisierung von RNA mit den Nukleinsäuresonden der Anordnung.

26. Verfahren zum Selektieren eines Satzes von Sonden und Immobilisieren der Sonden auf einer Oberfläche eines Substrats als eine Anordnung zum Überwachen der Expression von RNA-Transkripten oder daraus hergeleiteten Nukleinsäuren aus einer großen Anzahl von Zielgenen, umfassend:
(a) Bereitstellen einer Anordnung von Nukleinsäuresonden, wobei die Anordnung eine Vielfalt von Nukleinsäuresonden umfasst, wobei jede Sonde zu einer Subsequenz der Zielnukleinsäuren komplementär ist und für jede Sonde eine entsprechende Fehlpaarungs-Kontrollsonde vorliegt, wobei z.B. die Fehlpaarungs-Kontrollsonden eine Ein-Basen-Fehlpaarung aufweisen;
(b) Hybridisieren der Zielnukleinsäuren mit der Anordnung von Nukleinsäuresonden;
(c) Selektieren derjenigen Sonden, bei denen der Unterschied in der Hybridisierungssignal-Intensität zwischen jeder Sonde und ihrer Fehlpaarungs-Kontrolle nachweisbar ist, wobei vorzugsweise der Unterschied in der Hybridisierungsintensität mindestens 10 % des Hintergrundsignals beträgt; und
(d) Immobilisieren einer großen Anzahl von ausgewählten Sonden für jede der zu analysierenden Zielnukleinsäuren zusammen mit Kontrollsonden auf der Oberfläche eines Substrats, wodurch die quantitative Bestimmung der Zielnukleinsäuren ermöglicht wird, wobei die Anordnung wie in Anspruch 22 definiert ist.

27. Verfahren nach Anspruch 26, umfassend weiterhin zwischen den Schritten (c) und (d) ein Hybridisieren der Anordnung mit einem Pool von Nukleinsäuren, umfassend Nukleinsäuren, die von den Zielnukleinsäuren verschieden sind; und Selektieren von Sonden, die das niedrigste Hybridisierungssignal aufweisen, und wobei sowohl die Sonde als auch ihre Fehlpaarungskontrolle eine Hybridisierungsintensität zeigen, die gleich oder niedriger ist als der 10-fache Wert des Hintergrunds.

28. Verfahren nach Anspruch 26 oder Anspruch 27, wobei die Vielfalt von Sonden alle Sonden mit einer einzigen Länge umfasst, die zu einer Subsequenz der Zielnukleinsäuren komplementär sind, wobei die Sonden eine Länge zwischen etwa 5 und 50 Nukleotiden aufweisen.

29. Verfahren nach einem der Ansprüche 26 bis 28, wobei die Länge der Nukleinsäuresonden in einem Bereich von etwa 5 bis etwa 45 Nukleotiden liegt.

30. Verfahren nach einem der Ansprüche 26 bis 29, wobei die Nukleinsäuresonden alle die gleiche Länge haben.

31. Verfahren nach einem der Ansprüche 26 bis 30, wobei die Anordnung mehr als 1000 verschiedene Nukleinsäuresonden umfasst, wobei jede unterschiedliche Nukleinsäuresonde an einer bekannten Stelle der Oberfläche liegt und die Dichte der verschiedenen Nukleinsäuresonden größer ist als 60 verschiedene Nukleinsäuresonden pro 1 cm² der Oberfläche.

32. Verfahren nach einem der Ansprüche 26 bis 31, wobei die Nukleinsäuresonden durch lichtgesteuerte Synthese synthetisiert werden.

33. Verfahren nach einem der Ansprüche 26 bis 32, wobei die Hybridisierung eine Hybridisierung bei einer niedrigen Stringenz von 30°C bis 50°C und 6 X SSPE-T oder niedriger umfasst, gefolgt von einem oder mehreren Waschgängen bei stetig zunehmender Stringenz, bis ein gewünschtes Niveau der Hybridisierungsspezifität erreicht ist.

34. Verfahren nach einem der Ansprüche 26 bis 33, wobei der Pool von Nukleinsäuren, der Nukleinsäuren umfasst, die von den Zielnukleinsäuren verschieden sind, Nukleinsäuren enthält, die einen "Sense" aufweisen, der zu dem der Zielnukleinsäuren entgegengesetzt ist.

35. Verfahren nach Anspruch 1, wobei die Kontroll-Nukleinsäuresonden Fehlpaarungssonden umfassen und der Schritt zum quantitativen Bestimmen in einem Computersystem durch die folgenden Schritte erfolgt:
Erfassen der Eingabe von Hybridisierungsintensitäten für die große Anzahl von Nukleinsäuresonden, umfassend Paare der Paarungssonden und Fehlpaarungssonden, wobei die Hybridisierungsintensitäten die Hybridisierungsaffinität zwischen der großen Anzahl von Nukleinsäuresonden und dem Pool von Nukleinsäuren anzeigen, und wobei jedes Paar eine Paarungssonde, die zu einem Teil der Nukleinsäuren exakt komplementär ist, und eine Fehlpaarungssonde enthält, die sich von der Paarungssonde durch mindestens ein Nukleotid unterscheidet;
Vergleichen der Hybridisierungsintensitäten der Paarungs- und Fehlpaarungssonden für jedes Paar; und
Anzeigen der Expression von einem oder mehreren der Gene in dem Pool gemäß den Ergebnissen des Vergleichsschritts.

36. Verfahren nach Anspruch 35, wobei der Vergleichsschritt folgendes umfasst: entweder
(a) Berechnen von Unterschieden zwischen den Hybridisierungsintensitäten der Paarungs- und Fehlpaarungssonden für jedes Paar, gegebenenfalls umfassend das Berechnen eines Durchschnitts der Unterschiede; oder
(b) Bestimmen, ob ein Unterschied zwischen den Paarungs- und Fehlpaarungssonden von jedem Paar einen Unterschieds-Schwellenwert überschreitet; oder
(c) Bestimmen, ob ein Quotient der Paarungs- und Fehlpaarungssonden von jedem Paar einen Verhältnis-Schwellenwert überschreitet; oder
(d) Bestimmen einer ersten Anzahl von Paaren, die einen Unterschied, der einen Unterschieds-Schwellenwert überschreitet, und einen Quotienten aufweisen, der einen Verhältnis-Schwellenwert überschreitet; vorzugsweise weiterhin umfassend Bestimmen einer zweiten Anzahl von Paaren, die einen Unterschied, der den Unterschieds-Schwellenwert nicht überschreitet, und einen Quotienten aufweisen, der den Verhältnis-Schwellenwert nicht überschreitet.

37. Verfahren nach Anspruch 35 oder Anspruch 36, wobei der Anzeigeschritt anzeigt, dass das Gen exprimiert ist, wenn ein Quotient aus der ersten und der zweiten Anzahl einen Expressions-Schwellenwert überschreitet.

38. Verfahren zum Selektieren von Sonden und Immobilisieren der Sonden an ein Substrat als eine Anordnung zur Verwendung zum Überwachen der Expression von RNA-Transkripten oder davon hergeleiteten Nukleinsäuren aus einer großen Anzahl von Genen, umfassend:
(i) in einem Computersystem:
(a) Erfassen der Eingabe einer Nukleinsäuresequenz eines Gens aus der großen Anzahl von Genen;
(b) Erzeugen eines Satzes von Sonden, die zu dem Gen exakt komplementär sind; und
(c) Identifizieren eines Subsatzes von Sonden, der weniger als sämtliche Sonden im Satz umfasst, zum Überwachen der Expression des Gens;
(d) Wiederholen von (a), (b) und (c) für mindestens ein weiteres Gen, wodurch mindestens ein weiterer Subsatz von Sonden identifiziert wird;
(ii) Immobilisieren der Subsätze von Sonden zusammen mit Kontrollsonden in einer Anordnung auf der Oberfläche eines Substrats, wodurch die quantitative Bestimmung der Transkripte der großen Anzahl von Genen ermöglicht wird, wobei die Anordnung wie in Anspruch 22 definiert ist.

39. Verfahren nach Anspruch 38, wobei der Identifizierungsschritt den Schritt umfasst, in dem jede Sonde des Satzes durch Kriterien analysiert wird, die Merkmale spezifizieren, die eine niedrige Hybridisierung oder eine hohe Kreuzhybridisierung anzeigen; wobei vorzugsweise jedes der Kriterien einen Schwellenwert umfasst, so dass, wenn eine ausgewählte Sonde ein Merkmal aufweist, das den Schwellenwert überschreitet, für die ausgewählte Sonde eine niedrige Hybridisierung oder eine hohe Kreuzhybridisierung angezeigt wird, und wobei gewünschtenfalls mindestens ein Schwellenwert erhöht werden kann, um die Sonden in dem Subsatz zu vermehren.

40. Verfahren nach Anspruch 39, das weiterhin den Schritt zum Bestimmen der Kriterien nach heuristischen Regeln umfasst, die aus verschiedenen Experimenten gefunden wurden.

41. Verfahren nach Anspruch 39 oder Anspruch 40, wobei eines der Kriterien eine niedrige Hybridisierung oder Kreuzhybridisierung anzeigt, wenn entweder:
(a) die Häufigkeit eines spezifischen Nukleotids in einer Sonde einen bestimmten Schwellenwert überschreitet; oder
(b) die Anzahl eines spezifischen Nukleotids, das sich in einer Sonde aufeinanderfolgend wiederholt, einen Schwellenwert überschreitet; oder
(c) eine Länge eines Palindroms in einer Sonde einen Schwellenwert überschreitet; oder
(d) eine Länge einer Subsequenz innerhalb einer Sonde, die lediglich zwei spezifische Nukleotide umfasst, einen Schwellenwert überschreitet.

42. Verfahren nach einem der Ansprüche 38 bis 41, wobei der Identifizierungsschritt durch ein neuronales Netz durchgeführt wird, das die Sonden des Satzes als Eingabe erfasst und aus dem die Sonden des Subsatzes als Ausgabe erhalten werden.

## Revendications

1. Procédé de contrôle simultané de l'expression d'une multiplicité de gènes, ledit procédé comprenant :
(a) l'apport d'un pool d'acides nucléiques cibles comprenant des transcrits d'ARN de certains desdits gènes, ou des acides nucléiques dérivés desdits transcrits d'ARN ;
(b) l'apport d'une pluralité de sondes différentes pour l'analyse de chacun des transcrits d'ARN à contrôler ; lesdites sondes étant immobilisées en une matrice sur une surface d'un substrat dans des sites connus à une densité supérieure à 60 sondes différentes par cm² ; lesdites sondes de matrice comprennent des sondes d'appariement et témoins ; la matrice comprenant plus de 100 sondes différentes, chaque sonde attachée à la surface par une liaison simple covalente ;
(c) l'hybridation dudit pool d'acides nucléiques à la matrice de sondes d'acide nucléique ; et
(d) la quantification de l'hybridation desdits acides nucléiques cibles à ladite matrice en comparant l'hybridation des sondes d'appariement et témoins, où ladite quantification fournit une mesure des niveaux de transcription desdits gènes.

2. Procédé selon la revendication 1, dans lequel chacune desdites sondes d'acide nucléique est synthétisée par voie chimique ou synthétisée par synthèse de polymère dirigée par la lumière, ou dans lequel la préparation desdites sondes d'acide nucléique ne nécessite pas de clonage, une étape d'amplification d'acide nucléique, ou une synthèse enzymatique et/ou ne nécessite pas de manipulation de substances biologiques.

3. Procédé selon la revendication 1 ou 2, dans lequel, pour chaque gène, ladite matrice comprend au moins 10 sondes d'acide nucléique différentes complémentaires de sous-séquences de ce gène, de préférence pas plus de 20 sondes d'acide nucléique différentes complémentaires de sous-séquences de ce gène.

4. Procédé selon l'une quelconque des revendications 1 à 3, dans lequel lesdites sondes d'acide nucléique ont une longueur de 5 à 45 nucléotides, de préférence une longueur de 20 à 25 nucléotides.

5. Procédé selon l'une quelconque des revendications 1 à 4, dans lequel ladite matrice comprend des séquences de sonde d'acide nucléique de gènes de contrôle exprimés de manière constitutive, lesdits gènes de contrôle étant éventuellement choisis parmi la β-actine, la GAPDH, et le récepteur de transferrine.

6. Procédé selon l'une quelconque des revendications 1 à 5, dans lequel la variation entre différentes copies de chaque matrice est inférieure à 20%, dans lequel ladite variation est mesurée comme le coefficient de variation de l'intensité d'hybridation moyenné sur au moins 5 sondes d'acide nucléique pour chaque gène dont l'expression doit être détectée par la matrice.

7. Procédé selon l'une quelconque des revendications 1 à 6, dans lequel la concentration d'acides nucléiques dans ledit pool est proportionnelle aux niveaux d'expression desdits gènes.

8. Procédé selon l'une quelconque des revendications 1 à 7, dans lequel lesdites sondes d'acide nucléique témoins comprennent des sondes témoins de mésappariement, telles que pour chaque sonde appariée il existe une sonde témoin de mésappariement.

9. Procédé selon la revendication 8, dans lequel ladite quantification comprend soit :
(a) le calcul de la différence d'intensité du signal d'hybridation entre chacune desdites sondes d'acide nucléique et sa sonde témoin de mésappariement correspondante ; ou
(b) le calcul de la différence moyenne de l'intensité du signal d'hybridation entre chacune desdites sondes d'acide nucléique et sa sonde témoin de mésappariement correspondante pour chaque gène.

10. Procédé selon l'une quelconque des revendications 1 à 9, dans lequel les sondes d'acide nucléique dans ladite matrice sont choisies selon l'une quelconque des revendications 26 à 34.

11. Procédé selon l'une quelconque des revendications 1 à 9, dans lequel les sondes d'acide nucléique dans ladite matrice sont choisies selon l'une quelconque des revendications 38 à 42.

12. Procédé selon l'une quelconque des revendications 1 à 11, dans lequel l'hybridation et la quantification sont accomplies dans les 48 heures.

13. Procédé selon l'une quelconque des revendications 1 à 12, dans lequel ladite hybridation est effectuée avec un volume de liquide de 250 µl ou inférieur, et/ou dans lequel ladite hybridation comprend une hybridation à faible stringence de 30°C à 50°C et avec 6 X SSPE-T ou inférieur, et un lavage à stringence plus élevée.

14. Procédé selon l'une quelconque des revendications 1 à 13, dans lequel ladite quantification comprend soit :
(a) la détection d'un signal d'hybridation qui est proportionnel à la concentration dudit ARN dans ledit échantillon d'acide nucléique ; ou
(b) la détection d'un signal d'hybridation qui est proportionnel à la concentration desdits acides nucléiques cibles pour chaque gène dans ledit pool d'acides nucléiques cibles.

15. Procédé selon l'une quelconque des revendications 1 à 14, dans lequel ledit pool d'acides nucléiques est un pool d'ARNm transcrits *in vitro* à partir d'un pool d'ADNc.

16. Procédé selon l'une quelconque des revendications 1 à 15, dans lequel ledit pool d'acides nucléiques est amplifié à partir d 'un échantillon biologique.

17. Procédé selon l'une quelconque des revendications 1 à 16, dans lequel ledit pool d'acides nucléiques comprend des acides nucléiques à marquage fluorescent ou dans lequel ledit pool d'acides nucléiques est marqué avec une espèce unique de fluorophore.

18. Procédé selon la revendication 17, qui comprend la quantification de la fluorescence d'un marqueur sur lesdits acides nucléiques hybridés, à une résolution spatiale de 100 µm ou supérieur, par exemple au moyen d'un microscope de fluorescence confocal à balayage.

19. Procédé selon l'une quelconque des revendications 1 à 18, dans lequel ledit apport d'une pluralité de sondes différentes comprend soit :
(i)
(a) l'hybridation d'un pool d'ARN avec un pool d'autres sondes d'acide nucléique comprenant au moins une partie des sondes d'appariement pour former un pool d'acides nucléiques hybridés ;
(b) le traitement dudit pool d'acides nucléiques hybridés avec la RNase A, digérant ainsi les séquences d'acide nucléique simple brin et laissant intactes les régions double brin hybridées ;
(c) la dénaturation des régions double brin hybridées et l'élimination desdites autres sondes d'acide nucléique, laissant ainsi un pool d'ARN amplifiés pour les ARN complémentaires aux sondes d'acide nucléique d'appariement dans ladite matrice ; ou
(ii)
(a) l'hybridation d'un pool d'ARN avec des sondes d'acide nucléique spécifiques de cible appariée où lesdites sondes d'acide nucléique spécifiques de cible appariée sont complémentaires des régions flanquant les sous-séquences complémentaires desdites sondes d'acide nucléique d'appariement dans ladite matrice ;
(b) le traitement dudit pool d'acides nucléiques avec la RNase H pour digérer les séquences d'acide nucléique hybridées (double brin) ;
(c) l'isolation des séquences d'acide nucléique restantes ayant une longueur à peu près équivalente à la région flanquée par lesdites sondes d'acide nucléique spécifiques de cible appariée ; ou
(iii)
(a) l'hybridation d'un pool d'ARNm polyA⁺ avec des sondes d'acide nucléique qui s'hybrident spécifiquement avec les messages cibles d'ARNm particuliers présélectionnées ;
(b) le traitement dudit pool d'acides nucléiques avec la RNase H pour digérer les séquences d'acide nucléique hybridées (double brin), séparant ainsi la séquence codante de la queue polyA⁺ ;
(c) l'isolation ou l'amplification des ARN polyA⁺ restants dans ledit pool.

20. Procédé selon l'une quelconque des revendications 1 à 19, dans lequel les sondes de la matrice comprennent des sondes choisies pour vérifier les transcrits variants par épissage d'un gène.

21. Procédé selon l'une quelconque des revendications 1 à 3 ou 5 à 20, dans lequel les sondes ont jusqu'à 500 bases de long.

22. Composition pour indiquer les niveaux d'expression d'une multiplicité de gènes, ladite composition comprenant une matrice d'une pluralité de sondes différentes pour chaque transcrit d'ARN à analyser ; lesdites sondes étant immobilisées en une matrice sur une surface d'un substrat dans des sites connus à une densité supérieure à 60 sondes différentes par cm²; lesdites sondes de matrice comprenant les sonde d'appariement et témoins ; la matrice comprenant plus de 100 sondes différentes, chaque sonde attachée sur la surface par une liaison simple covalente ;
et lesdites sondes d'acide nucléique pouvant être hybridées spécifiquement à des acides nucléiques à marquage fluorescent, et choisies de telle manière que la quantité de fluorescence ainsi hybridée à ladite matrice est indicatrice de la quantité desdits transcrits d'ARN, éventuellement dans lequel ladite intensité de fluorescence est proportionnelle aux niveaux de transcription de ladite multiplicité de gènes présélectionnés dans un échantillon biologique.

23. Composition selon la revendication 22 et définie en outre par la(les) caractéristique(s) specifique (s) de l'une ou plusieurs des revendications 2 à 5, 8, 16, 20 ou 21.

24. Kit de détection des niveaux d'expression d'une multiplicité de gènes, ledit kit comprenant :
une pluralité choisie de sondes d'appariement et témoins différentes pour chaque transcrit d'ARN qui doit être contrôlé ; les sondes d'appariement et témoins choisies étant immobilisées en une matrice d'une surface d'un substrat dans des sites connus, la matrice comprenant plus de 100 sondes différentes à une densité supérieure à 60 sondes différentes par cm², chaque sonde attachée à la surface par une liaison simple covalente ; et
éventuellement, les instructions décrivant l'utilisation de ladite matrice pour la quantification des niveaux d'expression de ladite multiplicité de gènes ;
éventuellement, dans lequel lesdites sondes témoins sont des sondes de mésappariement, avec une sonde de mésappariement qui correspond à chaque sonde d'appariement.

25. Kit selon la revendication 24, comprenant en outre un marqueur fluorescent pour marquer l'ARN ou l'ADN qui doit être hybridé aux acides nucléiques de ladite matrice et/ou les tampons et réactifs pour l'hybridation de l'ARN aux sondes d'acide nucléique de ladite matrice.

26. Procédé de sélection d'un ensemble de sondes et immobilisation des sondes sur une surface d'un substrat en une matrice pour contrôler l'expression des transcrits d'ARN ou des acides nucléiques qui en sont dérivés à partir d'une pluralité de gènes cibles comprenant :
(a) l'apport d'une matrice de sondes d'acide nucléique, ladite matrice comprenant une multiplicité de sondes d'acide nucléique, dans laquelle chaque sonde est complémentaire d'une sous-séquence desdits acides nucléiques cibles, et, pour chaque sonde, il y a une sonde témoin de mésappariement correspondante, par exemple où lesdites sondes témoins de mésappariement ont un mésappariement d'1 base ;
(b) hybridation desdits acides nucléiques cibles à ladite matrice de sondes d'acide nucléique ;
(c) la sélection de ces sondes où la différence d'intensité du signal d'hybridation entre chaque sonde et son témoin de mésappariement est détectable, de préférence dans laquelle ladite différence d'intensité d'hybridation est d'au moins 10% du signal de bruit de fond ; et
(d) l'immobilisation d'une pluralité des sondes choisies pour chacun des acides nucléiques cibles à analyser ensemble avec les sondes témoins sur la surface d'un substrat pour permettre la quantification des acides nucléiques cibles, où la matrice est telle que définie dans la revendication 22.

27. Procédé selon la revendication 26, comprenant en outre, entre les étapes (c) et (d), l'hybridation de ladite matrice à un pool d'acides nucléiques comprenant des acides nucléiques différents desdits acides nucléiques cibles ; et la sélection des sondes ayant le signal d'hybridation le plus faible et où à la fois la sonde et son témoin de mésappariement ont une intensité d'hybridation inférieure ou égale à 10 fois le bruit de fond.

28. Procédé selon la revendication 26 ou la revendication 27, dans lequel ladite multiplicité de sondes comprend toutes les sondes de longueur unique qui sont complémentaires d'une sous-séquence dudit acide nucléique cible, où lesdites sondes ont une longueur de 5 à 50 nucléotides.

29. Procédé selon l'une quelconque des revendications 26 à 28, dans lequel lesdites sondes d'acide nucléique ont une longueur d'environ 5 à environ 45 nucléotides.

30. Procédé selon l'une quelconque des revendications 26 à 29, dans lequel lesdites sondes d'acide nucléique ont la même longueur.

31. Procédé selon l'une quelconque des revendications 26 à 30, dans lequel ladite matrice comprend au moins 1000 sondes d'acide nucléique différentes dans lesquelles chaque sonde d'acide nucléique différente est localisée dans un site connu de ladite surface et la densité desdites sondes d'acide nucléique différentes est supérieure à 60 sondes d'acide nucléique différentes par cm² de ladite surface.

32. Procédé selon l'une quelconque des revendications 26 à 31, dans lequel lesdites sondes d'acide nucléique sont synthétisées par synthèse dirigée par la lumière.

33. Procédé selon l'une quelconque des revendications 26 à 32, dans lequel ladite hybridation comprend une hybridation à faible stringence de 30°C à 50°C et avec 6 X SSPE-T ou inférieur, suivi par un ou plusieurs lavages à stringence croissant progressivement jusqu'à ce qu'un niveau souhaité de spécificité d'hybridation soit obtenu.

34. Procédé selon l'une quelconque des revendications 26 à 33, dans lequel ledit pool d'acides nucléiques comprenant des acides nucléiques différents desdits acides nucléiques cibles comprend les acides nucléiques ayant un sens opposé à celui des acides nucléiques cibles.

35. Procédé selon la revendication 1, dans lequel les sondes d'acide nucléique témoins comprennent les sondes de mésappariement, et l'étape de quantification est effectuée dans un système informatique par les étapes de :
réception de l'entrée des intensités d'hybridation pour la pluralité de sondes d'acide nucléique comprenant les paires des sondes d'appariement et des sondes de mésappariement, les intensités d'hybridation indiquant l'affinité d'hybridation entre la pluralité de sondes d'acide nucléique et le pool d'acides nucléiques, et chaque paire comprenant une sonde d'appariement qui est parfaitement complémentaire d'une partie des acides nucléiques, et une sonde de mésappariement qui diffère de la sonde d'appariement par au moins un nucléotide ;
comparaison des intensités d'hybridation des sondes d'appariement et de mésappariement de chaque paire ; et
indication de l'expression d'un ou de plusieurs gènes dans le pool selon les résultats de l'étape de comparaison.

36. Procédé selon la revendication 35, dans lequel l'étape de comparaison comprend soit :
(a) le calcul des différences entre les intensités d'hybridation des sondes d'appariement et de mésappariement de chaque paire, comprenant éventuellement le calcul d'une moyenne de la différence ; ou
(b) de déterminer si une différence entre les sondes d'appariement et de mésappariement de chaque paire dépasse un seuil de différence ; ou
(c) de déterminer si un quotient des sondes d'appariement et de mésappariement de chaque paire dépasse un rapport seuil ; ou
(d) de déterminer un premier nombre de paires qui ont une différence qui dépasse un seuil de différence et un quotient qui dépasse un rapport seuil ; de préférence, comprenant en outre la détermination d'un second nombre de paires qui ont une différence qui ne dépasse pas le seuil de différence et un quotient qui ne dépasse pas un rapport seuil.

37. Procédé selon la revendication 35 ou la revendication 36, dans lequel l'étape indicatrice indique que le gène est exprimé si un quotient des premier et second nombres dépasse un seuil d'expression.

38. Procédé de sélection de sondes et d'immobilisation des sondes sur un substrat en une matrice pour une utilisation dans le contrôle de l'expression de transcrits d'ARN ou d'acides nucléiques dérivés de ceux-ci à partir d'une pluralité de gènes, comprenant :
(i) dans un système informatique :
(a) la réception de l'entrée d'une séquence d'acide nucléique d'une des pluralités de gènes ;
(b) la production d'un ensemble de sondes qui sont parfaitement complémentaires au gène ; et
(c) l'identification d'un sous-ensemble de sondes, comprenant moins de la totalité des sondes dans l'ensemble, pour contrôler l'expression du gène ;
(d) la répétition de la), de (b) et de (c) pour au moins un autre gène pour identifier au moins un autre sous-ensemble de sondes ;
(ii) l'immobilisation des sous-ensembles de sonde ensemble avec les sondes témoins dans une matrice sur la surface d'un substrat pour permettre la quantification des transcrits de la pluralité de gènes, où la matrice est telle que définie dans la revendication 22.

39. Procédé selon la revendication 38, dans lequel l'étape d'identification comprend l'étape d'analyse de chaque sonde de l'ensemble par des critères qui spécifient les caractéristiques indicatrices d'une faible hybridation ou d'une hybridation croisée élevée ; de préférence, dans lequel chacun des critères comprend une valeur seuil, telle que si une sonde choisie a une caractéristique qui dépasse la valeur seuil, une faible hybridation ou une hybridation croisée élevée sont indiquées pour la sonde choisie, et, si on le souhaite, comprenant en outre l'augmentation d'au moins une valeur seuil pour augmenter les sondes dans le sous-groupe.

40. Procédé selon la revendication 39, comprenant en outre l'étape de détermination des critères comme règles heuristiques dérivées d'expériences multiples.

41. Procédé selon la revendication 39 ou la revendication 40, dans lequel un des critères indique une faible hybridation ou une hybridation croisée si, soit :
(a) les occurrences d'un nucléotide spécifique dans une sonde dépassent une valeur seuil ; ou
(b) le nombre d'un nucléotide spécifique se répétant de manière séquentielle dans une sonde dépasse une valeur seuil ; ou
(c) une longueur de palindrome dans une sonde dépasse une valeur seuil ; ou
(d) une longueur d'une sous-séquence dans une sonde qui comprend seulement deux nucléotides spécifiques dépasse une valeur seuil.

42. Procédé selon l'une quelconque des revendications 38 à 41, dans lequel l'étape d'identification est effectuée par un réseau neuronal qui reçoit en entrée les sondes de l'ensemble et resort les sondes du sous-ensemble.
